# EUROPEAN PATENT SPECIFICATION

(11) **EP 2 759 605 B1**
(45) Date of publication and mention of the grant of the patent: **14.11.2018**
(21) Application number: 14152566.7
(22) Date of filing: 24.01.2014
(51) Int. Cl.: C12Q 1/6886, G06F 19/18, G06F 19/24, G06F 19/00

(54) **A method for predicting a manifestation of an outcome measure of a cancer patient**
Verfahren zur Vorhersage einer Manifestation einer Ergebnismessung eines Krebspatienten
Procédé permettant de prédire une manifestation de mesure d'un résultat d'un patient atteint d'un cancer

(30) Priority: 25.01.2013 US 201361756801 P; 25.01.2013 EP 13152610; 25.01.2013 EP 13152797
(43) Date of publication of application: 30.07.2014
(73) Proprietor: Signature Diagnostics AG, 14473 Potsdam (DE)
(72) Inventor: Rosenthal, André, 14194 Ludwigsfelde (DE); Adams, Hans-Peter, 14478 Potsdam (DE)
(74) Representative: Hoppe, Georg Johannes

(56) References cited:
- EP-A1- 2 508 619
- WO-A1-2005/108621
- WO-A2-2013/172779
- US-A1- 2011 301 863
- LI LANG ET AL: "A mixture model approach in gene-gene and gene-environmental interactions for binary phenotypes", JOURNAL OF BIOPHARMACEUTICAL STATISTICS,, vol. 18, no. 6, 1 January 2008 (2008-01-01), pages 1150-1177, XP009133263, ISSN: 1520-5711
- F. MOLINARI ET AL: "Increased Detection Sensitivity for KRAS Mutations Enhances the Prediction of Anti-EGFR Monoclonal Antibody Resistance in Metastatic Colorectal Cancer", CLINICAL CANCER RESEARCH, vol. 17, no. 14, 15 July 2011 (2011-07-15) , pages 4901-4914, XP055113596, ISSN: 1078-0432, DOI: 10.1158/1078-0432.CCR-10-3137
- JAESEONG JO ET AL: "Prediction of Colorectal Cancer Risk Using a Genetic Risk Score: The Korean Cancer Prevention Study-II (KCPS-II)", GENOMICS & INFORMATICS, vol. 10, no. 3, 1 January 2012 (2012-01-01), page 175, XP055113706, ISSN: 1598-866X, DOI: 10.5808/GI.2012.10.3.175
- MOHAMMAD SHAHROKH ESFAHANI ET AL: "Probabilistic reconstruction of the tumor progression process in gene regulatory networks in the presence of uncertainty", BMC BIOINFORMATICS, BIOMED CENTRAL, LONDON, GB, vol. 12, no. Suppl 10, 18 October 2011 (2011-10-18), page S9, XP021109910, ISSN: 1471-2105, DOI: 10.1186/1471-2105-12-S10-S9
- M. GERSTUNG ET AL: "Quantifying cancer progression with conjunctive Bayesian networks", BIOINFORMATICS, vol. 25, no. 21, 1 November 2009 (2009-11-01), pages 2809-2815, XP055113439, ISSN: 1367-4803, DOI: 10.1093/bioinformatics/btp505
- DATABASE BIOSIS [Online] BIOSCIENCES INFORMATION SERVICE, PHILADELPHIA, PA, US; September 2013 (2013-09), ROSENTHAL A ET AL: "ONCO-Predict-II: A deep sequencing test predicting outcome in patients with colorectal cancer of stage II based on mutations signatures in driver genes", XP9177588, Database accession no. PREV201400046953 & EUROPEAN JOURNAL OF CANCER, vol. 49, no. Suppl. 2, September 2013 (2013-09), page S491, 17TH ECCO / 38TH ESMO / 32ND ESTRO EUROPEAN CANCER CONGRESS ON REINFORCING MULTIDISCIPLINARITY; AMSTERDAM, NETHERLANDS; SEPTEMBER 27 -OCTOBER 01, 2013 ISSN: 0959-8049(print)
- HERMAN F. FUMIÃ ET AL: "Boolean Network Model for Cancer Pathways: Predicting Carcinogenesis and Targeted Therapy Outcomes", PLOS ONE, vol. 8, no. 7, 26 July 2013 (2013-07-26), page e69008, XP055113409, ISSN: 1932-6203, DOI: 10.1371/journal.pone.0069008
- NOAH BERLOW ET AL: "A new approach for prediction of tumor sensitivity to targeted drugs based on functional data", BMC BIOINFORMATICS, BIOMED CENTRAL, LONDON, GB, vol. 14, no. 1, 29 July 2013 (2013-07-29), page 239, XP021158312, ISSN: 1471-2105, DOI: 10.1186/1471-2105-14-239
- Anonymous: "Boolean algebra", Wikipedia, 22 June 2011 (2011-06-22), pages 1-19, XP055404407, Retrieved from the Internet: URL:https://en.wikipedia.org/w/index.php?t itle=Boolean_algebra&oldid=435591730 [retrieved on 2017-09-06]

## Description

The invention pertains to a method for predicting a manifestation of an outcome measure of a cancer patient based on a tumor DNA containing tissue sample from the cancer patient as well as to a method for determining a function that allows for the prediction of the manifestation of an outcome measure (such as the development of a metastasis vs. no development of a metastasis or response to therapy vs. no response to therapy) of a cancer patient.

### Introduction

Cancer, in particular solid tumor cancer, is a group of diseases that can occur in every organ of the human body and affects a great number of people.

Colorectal cancer, for example, affects 73.000 patients in Germany and approximately 145.000 patients in the United States. It is the second most frequent solid tumor after breast and prostate cancer. Treatment of patients with colorectal cancer differs dependent on the location of the tumor, the stage of the disease, various additional risk factors and routine practice in various countries. Standard treatment for patients with colon cancer that is locally defined (stage I and stage II or has spread only to lymph nodes (stage III) always involves surgery to remove the primary tumor. Standard treatment for patients with rectum cancer may differ from country to country and from hospital to hospital as a significant part of these patients will receive neo-adjuvant radio/chemotherapy followed by surgery to remove the tumor tissue.

The five-year survival rates of patients with colorectal cancer depend on the clinical stage of the individual patient, the histopathological diagnosis, stage-specific treatment options as well as on routine medical practice that differs from country to country, and often also from hospital to hospital. There are also significant differences in the routine treatment of patient with colorectal cancer in the western world.

In most countries, patients with UICC stage I disease will not receive any additional chemotherapy after surgery as their five-year survival is approximately 95 %.

Treatment options for patients with UICC stage II colon cancer differ in many Western countries. The five-year survival of patients with UICC II disease is approximately 80 % to 82 %, meaning that 18 % to 20 % will experience a progression of disease - often liver or lung metastasis. Once the disease will have spread to distant organs the outcome of the patients is much worse, and the majority of these patient will die relatively quickly despite heavy treatment. Therefore, guidelines in some Western countries recommend offering adjuvant chemotherapies to patients with UICC stage II disease including 5-flourouracil and leucovorine or in combination with oxaliplatin. In other European countries including Germany, the guidelines do not recommend to offer patients with UICC stage II disease adjuvant chemotherapy. There is a controversy if adjuvant chemotherapy should be offered to UICC II patients or not. Randomized clinical data that show a benefit of adjuvant chemotherapy is still missing for these patient cohorts.

Patients with locally advanced colorectal cancer - loco-regional lymph nodes are infiltrated with cancer cells - have a five-year survival rate of 49 %. The treatment guidelines therefore recommend that after surgery all patients should receive adjuvant chemotherapy, either a triple combination of 5-FU, leucovorin and oxaliplatin (FOLFOX4 or FOLFOX6 regimes) or dual combination of capecitabine - an orally available 5-FU derivative - and oxaliplatin (CAPOX). For elderly patients with low ECOG performance scores or know toxicities, the dual 5-FU/leucovorin scheme should be used. In the routine practice only 60 to 80 % of patients with UICC stage III disease will however receive adjuvant chemotherapy. In Germany, only 60 % of UICC stage III patients will be treated with FOLFOX or 5-FU/leucovorin. There is also a difference in treatment between non-city environment and city populations. In general, approximately 50 % of patients with UICC stage III disease will experience progression of disease within 1 to 2 years after surgery. Once distant metatastasis is diagnosed, these patients will be offered additional therapies including treatment with targeted antibodies drugs that inhibit the EGFR receptor including cetuximab or panitumumab or antibodies directed against the VGFA ligand (bevacizimab). Several lines of therapies are offered, but most of these patients with disease progression will die within a five-year interval.

The five-year survival rate for patients with advanced, metastatic disease is dramatically low. Only 8 % will survive the first five years after surgery. It is these patients for which most of the treatment options with targeted therapies were developed over the last ten years, however, with limited success. The first targeted antibody therapy involved an anti-EGFR antibody cetuximab that was approved in 2004 by the FDA as monotherapy or in combination with Irinotecan, for patients with metastatic CRC (mCRC) that failed prior chemotherapy with irinotecan. In the original BOND study the response rate of the patients for the cetuximab was approximately 11 %. In 2007, a second anti-EGFR antibody, panitumumab, was approved for the treatment of mCRC patients. However, the FDA approved panitumumab only in combination with a KRAS wildtype (wt), as it was shown in 2007 that only patients with wt KRAS gene would benefit from panitumumab. However, the data also showed that many patients with mCRC and wt KRAS did not benefit from panitumumab. Also, there were some mCRC patients with mutations in the KRAS gene that showed response to panitumumab. Similar data was also published in 2008 to 2009 for cetuximab that led to a label change for the approval of cetuximab. At the moment, both cetuximab and panitumumab are only approved for patients with mCRC and wildtype KRAS status.

### Companion Diagnostics (CDx)

Accurate prediction of response/nonresponse to therapy is a prerequisite for individualized approaches to treatment. Current clinical practice in the treatment of patients with solid tumors does not offer effective and accurate prediction of response/nonresponse to chemotherapy and hormone therapy.

In prostate cancer no predictive biomarkers are known or established that predict response to radiation, hormone therapy or chemotherapy with taxanes. The same is true for advanced non-small cell lung cancer (NSCLC). Approximately 70 % to 80 % of all NSCLC patients have stage IIIB or stage IV disease at the time of first diagnosis. For the majority of these patients no predictive markers exist that allow prediction of response to small molecule drugs like erlotinib or iressa that inhibit the kinase function of the EGF receptor. Only in a small cohort of NSCLC patients with EGFR mutations in the kinase domain response to erlotinib was observed. Still 90 % of the NSCLC patients of stage IIIB and IV have a five-year survival of less than 8 % despite treatment.

The situation in breast cancer is more complex. For example, most patients with early breast cancer (lymphnode negative, estrogene (ER+) and/or progesterone receptor positive (PR+)) will receive radiation, chemotherapy and hormone therapy with tamoxifen after surgical removal of the tumor. The five-year survival of these patient cohorts is between 90 to 95 %. However, only 4 % of the patients will benefit from the addition of chemotherapy. Current treatment guidelines still recommend the overtreatment of 100 % of these patients with chemotherapy in order to reach the 4 % patients that may benefit. Similarly, a significant portion of the patients do not benefit from tamoxifen although there are ER positive. Effective methods to predict response to the chemotherapy or hormone therapy are still missing.

There is one FDA approved CDx in breast cancer. Determination of the HERII status is predictive of response to trastuzumab, an anti HERII antibody. Thus, patients with HERII positive breast cancer will receive trastuzumab at some point of their treatment. However, only 25 % of all breast cancer patients are HERII positive and of those only 20-25 % of the patients benefit from trastuzumab, meaning that 75-80 % of HERII positive breast cancer patients are over treated and have no benefit from this expensive treatment.

In colorectal cancer, no predictive biomarkers are established in the adjuvant treatment of UICC II or UICC III patients.

At time of first diagnosis, 70 % of the CRC patients are in UICC stage II and UICC stage III. 20 % of the UICC stage II and 49 % of the UICC stage III patients will suffer from progression of disease within 2 to 3 years after surgery. The majority of the patients are diagnosed with metastasis in the liver; about 20% are diagnosed with metastatic disease in the lung. Hence, anti-EGFR antibody drugs like cetuximab and panitumumab would be ideal drugs to treat these patients before metastasis will occur if responders to these drugs could be identified and separated from non-responders. Recently, two randomized phase III trials, one in the US and one in Europe, evaluating cetuximab vs. cetuximab plus FOLFOX in UICC stage III patients did not meet their endpoints. Secondary endpoint analysis showed that patients with wild type KRAS did not benefit in the Cetuximab/FOLFOX arm in comparison to patients in the FOLFOX arm (ASCO, 2010).

WO-A-2005/108621 discloses a method for determining the risk of relapse of cancer in which the quantity of mutated alleles to non-mutated alleles is determined. In particular gene segments of KRAS and TP53 are analysed.

EP-A-2 508 619 discloses a method of analysing SNPs in PIK3CA, KRAS and BRAF. The finding of one mutation allows the conclusion that the patient will not respond to anti-EGFR therapy.

Therefore, there is a large clinical need in the art to predict whether a patient with cancer of a certain type and/or of a certain stage will respond to a particular treatment. In addition, there is a large clinical need in the art to predict how the cancer of a certain type and/or of a certain stage of a patient will develop over time.

### Short Description of the Invention

The present invention provides methods, as defined in the claims, for predicting a manifestation of an outcome measure of a cancer patient based on a tumor DNA containing tissue sample from the cancer patient as well as methods for determining a function that allows for the prediction of the manifestation of an outcome measure, for example development of a metastasis vs. no development of a metastasis or response to therapy vs. no response to therapy, of a cancer patient based on a tumor DNA containing tissue sample from the patient.

In one aspect, the invention provides a method for determining a function that allows for the prediction of the manifestation of an outcome measure (for example the development of a metastasis vs. no development of a metastasis, or response to therapy vs. no response to therapy) of a cancer patient.

The method is based on a tumor DNA containing tissue sample obtained from the patient. In certain embodiments of the method, the tumor DNA containing tissue sample is tumor tissue, sputum, stool, urine, bronchial lavage, cerebro-spinal fluid, blood, plasma, or serum.

The tumor DNA containing tissue sample can, in particular, be a fresh-frozen sample, or a formalin-fixed paraffin-embedded sample.

The cancer is preferably a solid-tumor cancer, such as a cancer of the colon, breast, prostate, lung, pancreas, stomach, or melanoma. The cancer can be of any clinical stage.

The method comprises determining the DNA sequence of at least two gene segments, e.g. determining the DNA sequence of segments of at least two genes, in a group of cancer patients, which is comprised of patients with at least two disjunctive manifestations of the outcome measure. For this purpose, the at least two gene segments or the at least two genes are each divided in segments of a size that allows for the reliable determination of the DNA sequence. Segments can be, for example, between 20 and 500 base pairs. Segments of 100 to 250 base pairs are preferred.

The determination of the DNA sequence can be performed using any appropriate method known in the state of the art. Preferred is DNA sequencing of the segments (amplicons) of at least two cancer genes using oligonucleotides. Preferred is the use of next-generation sequencing methods (NGS).

The method further comprises the step of determining the sequence variation of the at least two gene segments or the at least two genes of the tumor DNA as either "present" (i.e. containing a sequence variation), if at least one significant sequence variation can be determined, or as "absent" (i.e. not containing a sequence variation), if no significant sequence variation can be determined, wherein the sequence variation comprises a silent or synonymous sequence variation.

The method further comprises the step of combining the sequence variation statuses of the at least two gene segments or of the at least two genes using a logical operator, thereby generating a prediction function, such that patients with one specific manifestation of the outcome measure are distinguishable from patients with another disjunctive manifestation of the same outcome measure, wherein the prediction function comprises sequence variations or its negation as variables and a logical operator combining the variables, wherein the logical operator is chosen from the group consisting of: negation of conjunction (Nand), negation of disjunction (Nor), equivalence (Eqv), negation of equivalence (exclusive disjunction, Xor), material implication (Imp), and negation of material implication (Nimp). By combining sequence variation statuses using at least one logical operator, the biological information contained in each sequence variation status is aggregated and thereby maximized. In other words, using logical operators, the biological information contained in each sequence variation status is aggregated and thereby the overall information is maximized. Thus, the prediction function is a maximization function. For example, in one embodiment of the invention, the existence of a sequence variation within segments of a first gene of the tumor DNA and of a second gene of the tumor DNA is determined as present or absent, respectively. Subsequently, the existence of sequence variations of the first and the second gene are combined using a logical operation (prediction function). It is then possible to determine the existence of a sequence variation within segments of a third gene of the tumor DNA as present or absent and combine the existence of sequence variations of the third gene using a logical operation with the sequence variation of the first and of the second gene such that the prediction function is maximized, i.e. that the prediction value is maximized (e.g. AROC or combined Jaccard ratio).

In certain embodiments of the method, predicting the outcome measure of the cancer patient comprises the prediction of progression of disease of a cancer of the patient, such as the local recurrence of the cancer, the occurrence of secondary malignancy, or the occurrence of metastasis (vs. no progression of disease). In other embodiments of the method, predicting the outcome measure of the cancer patient comprises the prediction of the response vs. nonresponse of the patient to a cancer treatment with a drug, such as adjuvant chemotherapy, neo-adjuvant chemotherapy, palliative chemotherapy or the use of targeted drugs in combination with a chemotherapy or radio-chemotherapy.

In certain embodiments, the drug is, e.g., Bevacizumab, Cetuximab, Panitumumab, IMC-11F8 and/or Oxaliplatin.

Bevacizumab (Avastin®) is a drug that blocks angiogenesis. It is used to treat various cancers, including colorectal cancer. Bevacizumab is a humanized monoclonal antibody that binds to vascular endothelial growth factor A (VEGF-A), which stimulates angiogenesis. Oxaliplatin (Eloxatin®, Oxaliplatin Medac®), is [(1R,2R)-cyclohexane-1,2-diamine](ethanedioato-O,O')platinum(II) and is known in the art as a cancer chemotherapy drug.

Cetuximab (IMC-C225, Erbitux®) is a chimeric (mouse/human) monoclonal antibody, an epidermal growth factor receptor (EGFR) inhibitor, usually given by intravenous infusion. Cetuximab is administered for the treatment of cancer, in particular for treatment of metastatic colorectal cancer and head and neck cancer.

Cetuximab binds specifically to the extracellular domain of the human epidermal growth factor receptor. It is composed of the Fv regions of a murine anti-EGFR antibody with human IgG1 heavy and kappa light chain constant regions and has an approximate molecular weight of 152 kDa. Cetuximab is produced in mammalian (murine myeloma) cell culture.

Panitumumab, also known as ABX-EGF, is a fully human monoclonal antibody specific to the epidermal growth factor receptor (EGFR). Panitumumab is manufactured by Amgen and sold as Vectibix®.

IMC-11F8 is a potent, fully human monoclonal antibody that targets the epidermal growth factor receptor (EGFR). It is currently in Phase II studies for metastatic colorectal cancer with one or more Phase III trials planned in 2009. IMC-11F8 is in development by Eli Lilly.

In the invention, the step of the prediction of the sequence variation comprises analyzing sequence variations that do not altering the protein sequence (silent or synonymous variations) of the encoded protein. In particular embodiments of the invention, the step of the prediction of the sequence variation further comprises analyzing sequence variations that alter the protein sequence.

The sequence variation that alters the protein sequence comprises missense variations, nonsense variations (sequence variations introducing a premature STOP codon), splicing variations, deletion variations, insertion variations, or frame shift variations. The sequence variations that do not alter the protein sequence comprise silent sequence variations (silent amino acid replacements) and synonymous variations.

The logical operation is part of a prediction function. The prediction function comprises the existence of sequence variations or its negation as variables and at least one logical operator. The logical operator is negation of conjunction (Nand), negation of disjunction (Nor), equivalence (Eqv), negation of equivalence (exclusive disjunction, Xor) material implication (Imp), or negation of material implication (Nimp) combining the variables. Within a prediction function, the same or different logical operators may be used, if the prediction function comprises more than one logical operator.

In one embodiment of the invention, the prediction function comprises at least three logical operators, for example, three, four, five, six, seven or more logical operators.

With respect to the logical operators, all standard logic rules of Boolean algebra apply, namely the law of the excluded middle, double negative elimination, law of noncontradiction, principle of explosion, monotonicity of entailment, idempotency of entailment, commutativity of conjunction, and De Morgan duality. Therefore, it is often possible to replace a given prediction function comprising the existence of sequence variations or its negation as variables and at least one logical operator with another prediction function comprising the existence of sequence variations or its negation as variables and at least one logical operator without obtaining a different result.

The prediction function is preferably optimized (i.e. maximized or minimized) for at least one of the following: sensitivity, specificity, positive predictive value, negative predictive value, correct classification rate, miss-classification rate, area under the receiver operating characteristic curve (AROC), odds-ratio, kappa, negative Jaccard ratio, positive Jaccard ratio, combined Jaccard ratio or cost, wherein area under the receiver operating characteristic curve (AROC) and the combined Jaccard Ratio are preferred.

In preferred embodiments of the invention, the step of constructing a prediction function combining the sequence variation statuses comprises the construction of a prediction function on a subset of patient data (sequence variation status and manifestation of the outcome measure) and prospective evaluation of the performance on patient data not used for construction of the prediction function.

In certain embodiments of the invention, the relative frequency of sequence variations within segments of the at least two gene segments or of the at least two genes is at least 3 % in a given patient population, preferably 5 %.

The at least two gene segments or at least two genes used in the method are so-called cancer genes, i.e. they are associated with the outcome measure of the patient. In one embodiment, the gene or genes are chosen from genes listed in Tables 1 to 8.

In another aspect, the invention provides a method for predicting a manifestation of an outcome measure of a cancer patient based on a tumor DNA containing tissue sample from the cancer patient. Use is made in this method of a function that allows for the prediction of the manifestation of an outcome measure, of a cancer patient based on a tumor DNA containing tissue sample from the patient as described above and herein.

Specifically, the method for predicting a manifestation of an outcome measure of a cancer patient based on a tumor DNA containing tissue sample from a cancer patient comprises determining an existence of a significant sequence variation within segments of at least two gene segments or of the at least two genes of the tumor DNA. The existence of a significant sequence variation is determined to be "present" (containing a sequence variation) if at least one significant sequence variation can be determined, or as "absent" (not containing a sequence variation) if no significant sequence variation can be determined, wherein the sequence variation comprises a silent or synonymous sequence variation.

As stated above, the at least two gene segments or the at least two genes of the tumor DNA are associated with the outcome measure of the patient. In other words, the at least two gene segments or the at least two genes used in the method are so-called cancer genes, i.e. they are associated with the outcome measure of the patient. In one embodiment, the gene(s) are chosen from genes listed in Tables 1 to 8.

The method further comprises the step of combining the existence of significant sequence variations of the at least two gene segments or of the at least two genes using a logical operation (i.e., a prediction function, as described above and herein), and predicting based on the results of the logical operation the manifestation of an outcome measure of the patient.

Preferred prediction functions are listed together with clinical performance for different outcome measures in Tables 9 to 35.

The method is based on a tumor DNA containing tissue sample obtained from the patient. In certain embodiments of the method, the tumor DNA containing tissue sample is tumor tissue, sputum, stool, urine, bronchial lavage, cerebro-spinal fluid, blood, plasma, or serum.

The tumor DNA containing tissue sample can, in particular, be a fresh-frozen sample, or a formalin-fixed paraffin-embedded sample.

The cancer is preferably a solid-tumor cancer, such as a cancer of the colon, breast, prostate, lung, pancreas, stomach, or melanoma. The cancer can be of different clinical stages.

In certain embodiments of the method, predicting the manifestation of an outcome measure of the cancer patient comprises the prediction of progression of disease of a cancer of the patient, such as the local recurrence of the cancer, the occurrence of secondary malignancy, or the occurrence of metastasis (vs. no progression of disease). In other embodiments of the method, predicting the manifestation of an outcome measure of the cancer patient comprises the prediction of the response vs. nonresponse of the patient to a cancer treatment with a drug, such as adjuvant chemotherapy, neo-adjuvant chemotherapy, palliative chemotherapy or the use of targeted drugs in combination with a chemotherapy or radio-chemotherapy.

In the invention, the step of the prediction of the sequence variation comprises analyzing sequence variations that do not altering the protein sequence (silent or synonymous variations) of the encoded protein. In particular embodiments of the invention, the step of the prediction of the sequence variation comprises analyzing sequence variations that alter the protein sequence.

The sequence variation that alters the protein sequence comprises missense variations, nonsense variations (sequence variations introducing a premature STOP codon), splicing variations, deletion variations, insertion variations, or frame shift variations. The sequence variations that do not alter the protein sequence comprise silent sequence variations (silent amino acid replacements) and synonymous variations.

The logical operator is part of a prediction function. The prediction function comprises the existence of sequence variations or its negation as variables and at least one logical operator. The logical operator is negation of conjunction (Nand), negation of disjunction (Nor), equivalence (Eqv), negation of equivalence (exclusive disjunction, Xor) material implication (Imp), or negation of material implication (Nimp) combining the variables. Within a prediction function, the same or different logical operators may be used, if the prediction function comprises more than one logical operator.

With respect to the logical operators, all standard logic rules of Boolean algebra apply, namely the law of the excluded middle, double negative elimination, law of noncontradiction, principle of explosion, monotonicity of entailment, idempotency of entailment, commutativity of conjunction, and De Morgan duality. Therefore, it is often possible to replace a given prediction function comprising the existence of sequence variations or its negation as variables and at least one logical operator with another prediction function comprising the existence of sequence variations or its negation as variables and at least one logical operator without obtaining a different result.

The prediction function is preferably optimized (i.e. maximized or minimized) for at least one of the following: sensitivity, specificity, positive predictive value, negative predictive value, correct classification rate, miss-classification rate, area under the receiver operating characteristic curve (AROC), odds-ratio, kappa, negative Jaccard ratio, positive Jaccard ratio, combined Jaccard ratio or cost.

The sequence variations are in certain embodiments of the method filtered by the type of variation, preferably by missense, nonsense, silent, synonymous, frame shift, deletion, insertion, splicing, noncoding, or combinations thereof.

In another aspect, the invention provides a computer program that is adapted to perform the methods described above and herein.

The computer program that is adapted to perform the steps of determining an existence of a significant sequence variation within segments of the gene or of the at least two genes of the tumor DNA as "present" (containing a sequence variation), if at least one significant sequence variation can be determined, or as "absent" (not containing a sequence variation), if no significant sequence variation can be determined, wherein the at least two genes of the tumor DNA are associated with the outcome measure of the patient; and wherein the sequence variation comprises a silent or synonymous sequence variation; and/or combining the existence of significant sequence variations of the at least two gene segments of genes using a logical operation (prediction function) thereby aggregating the biological information contained in each sequence variation status, and/or predicting based on the results of the logical operation the manifestation of the outcome measure of the patient.

In another aspect, the description provides a storage device comprising the computer program as described above and herein.

In another aspect, the description provides a kit, comprising oligonucleotides for sequencing the segments (amplicons) of at least two cancer associated genes, and the computer program described above and herein.

The invention provides methods, as defined in the claims, for predicting a manifestation of an outcome measure of a cancer patient based on a tumor DNA containing tissue sample from the cancer patient as well as methods for determining a function that allows for the prediction of the manifestation of an outcome measure, for example development of a metastasis vs. no development of a metastasis or response to therapy vs. no response to therapy, of a cancer patient based on a tumor DNA containing tissue sample from the patient.

Three steps are required for the invention
- Filtering of significant sequence variations
- Functional filtering
- Construction of a prediction function to link sequence variations to the manifestation of an outcome measure

First, the filtering of significant sequence variations is described.

### Filtering of significant sequence variations

The invention necessitates deep sequencing, which is sequencing with high coverage, of the DNA of at least two segments of at least two gene segments or of at least two genes. Several technologies exist that perform this task. The inventors have used the Illumina technology platform for deep sequencing (Illumina, Inc., San Diego, CA 92122 USA). Common to all deep sequencing methods are the results, namely sequence alignment maps (SAM/BAM-files) of the sequenced bases, which makes up the DNA and an analysis of sequence variation data (VCF-files). The sequence alignment uses the human reference genome provided by the Genome Reference Consortium. It is publicly available from the National Institute of Biotechnology Information of The National Institute of Health of the United States of America.

While the inventors use the information of the type of variation that is provided by the manufacturer of the sequencing technology, the inventors use a method of our own invention to determine whether a sequence variation is significant or not.

Table A displays a small part of deep sequencing results of an analysis of a gene segment, namely KRAS. For each unique chromosome position it needs to be decided whether a significant variation is present or not. This invention exploits the fact that the inventors are always dealing with a mixture of normal and tumor DNA. Given a solid tumor sample, the fraction of tumor cells is always significantly lower than 100 percent, because there is always some fraction of normal tissue, muscle cells, and stromal cells present. The preparation of the tumor tissue always can ensure that the tumor fraction is at least 10%. In cell-free DNA extracted from blood plasma the vast majority stems from normal tissue, however it cannot be ascertained how big the fraction of tumor DNA is. Thus, the decision whether a significant variation is present can be made without the knowledge of the human reference genome.

The overall hypothesis, whether a significant variation is present or not, can be split into four null hypotheses:
1.) The fraction of the overall most frequent nucleotide is not significantly smaller than 99% of the overall coverage.
2.) The fraction of the most frequent nucleotide on allele I is not significantly smaller than 99% of the coverage of allele I.
3.) The fraction of the most frequent nucleotide on allele II is not significantly smaller than 99% of the coverage of allele II.
4.) The fraction of the overall second most frequent nucleotide is not significantly higher than 1% of the overall coverage.

If hypothesis 1 and either hypothesis 2 or hypothesis 3 and hypothesis 4 is rejected by an appropriate statistical test, then there is a statistically significant variation present. Appropriate statistical tests are among others the Poisson test or the binomial exact test. Depending on the number of unique chromosome positions sampled it is good statistical practice to adjust the overall error of first kind, which is called alpha, to account for multiple testing. In the presented examples the number of unique chromosome positions is 26711. Hence the statistical tests are made at the alpha = 0.05/26711 level, and the upper and lower confidence limits are computed accordingly. In case that another panel with a different number of unique chromosome positions is used, the correction for multiple testing must be adjusted accordingly.

In biological terms, hypothesis 1 and hypothesis 4 ensure that the observed sequence variation is not measurement noise, whereas hypothesis 2 and hypothesis 3 ensure that the variation is not a heterozygous sequence variation.

The manufacturer of the panel ensures that the average measurement noise at each unique position is 1%, which has been confirmed by scientific publications. However, using 315 own samples the inventors used the observed noise levels for each position across all samples to ascertain valid variations above the noise level.

**Table A: Example of the Analysis of a Segment of the Gene KRAS**

| Chromosome | Position | Reference Nucleotide | Forward Strand Allele I | | | | Reverse Strand Allele II | | | | Overall Coverage |
|---|---|---|---|---|---|---|---|---|---|---|---|
| | | | A | C | G | T | A | C | G | T | |
| 12 | 25380286 | T | 1 | 1 | 0 | 173 | 1 | 1 | 0 | 182 | 359 |
| 12 | 25380287 | G | 0 | 0 | 176 | 0 | 0 | 0 | 185 | 0 | 361 |
| 12 | 25380288 | T | 0 | 0 | 0 | 176 | 0 | 0 | 0 | 185 | 361 |
| 12 | 25380289 | C | 0 | 176 | 0 | 0 | 0 | 185 | 0 | 0 | 361 |
| 12 | 25380290 | G | 4 | 0 | 172 | 0 | 5 | 0 | 180 | 0 | 361 |
| 12 | 25380291 | A | 176 | 0 | 0 | 0 | 184 | 0 | 1 | 0 | 361 |
| 12 | 25380292 | G | 1 | 175 | 0 | 0 | 2 | 182 | 0 | 1 | 361 |
| 12 | 25380293 | A | 136 | 0 | 0 | 40 | 145 | 0 | 0 | 40 | 361 |

As shown in Table A, the analysis of DNA segments results in counts of the four bases, namely Arginine (A), Cytosine (C), Guanine (G), and Tyrosine (T), which make up the genetic code. To demonstrate the statistical tests, the code for the publicly available R- statistical software package is given for chromosome 12 position 25380290:
Hypothesis 1: poisson.test(x = (361-9), T = 361, r = 0.99, alternative = "less", conf.level = 1-0.05/26711) results in a p-value of 0.4011
Hypothesis 2: poisson.test(x = (172-4), T = 172, r = 0.99, alternative = "less", conf.level = 1-0.05/26711) results in a p-value of 0.4507
Hypothesis 3: poisson.test(x = (180-5), T = 180, r = 0.99, alternative = "less", conf.level = 1-0.05/26711) results in a p-value of 0.4246
Hypothesis 4: poisson.test(x = 9, T = 361, r = 0.01, alternative = "greater", conf.level = 1-0.05/26711) results in a p-value of 0.01186

Since all p-values are greater than 0.05/26711 = 0.0000181 none of the null-hypotheses can be rejected, thus is no statistically significant variation.

This is a little different for chromosome 12 position 25380293, again the R-code is given so that any knowledgeable person can repeat the following hypothesis tests:
Hypothesis 1: poisson.test(x = (136+145), T = 361, r = 0.99, alternative = "less", conf.level = 1-0.05/26711) results in a p-value of 1.580681e-05
Hypothesis 2: poisson.test(x = (176-40), T = 176, r = 0.99, alternative = "less", conf.level = 1-0.05/26711) results in a p-value 0.001539
Hypothesis 3: poisson.test(x = (185-40), T = 185, r = 0.99, alternative = "less", conf.level = 1-0.05/26711) results in a p-value of 0.002028
Hypothesis 4: poisson.test(x = 80, T = 361, r = 0.01, alternative = "greater", conf.level = 1-0.05/26711) results in a p-value of 2.2e-16

In this instance, hypothesis 4 needs to be rejected, but not hypotheses 1, 2, and 3. Thus, even if a variation of 80 out of 361 appears to be significant, this does not hold if strict bio-statistical principles are employed. This also exemplifies that a high overall coverage is required to detect statistically significant variations.

This filtering of significant variation does not require knowledge about a reference.

Next, the functional filtering is described.

### Functional filtering

Some genetic variations lead to a change in the sequence of the coded proteins, while others do not. Table B lists some properties of the most frequent types of functions of variations. Unfortunately the functional changes are not clearly disjunctive.

**Table B: Functions of Genetic Variations**

| Variation Type | Description | Impact on the protein |
|---|---|---|
| Point Variation | | |
| Missense | single nucleotide substitution changing the amino acid | Yes |
| Nonsense | single nucleotide substitution resulting in a premature stop codon | Yes |
| Silent | substitution outside the exon without an impact on a protein | No |
| Synonymous | silent mutation within an exon, not changing any amino acid | No |

| Indels | | |
|---|---|---|
| Frame shift | Indels changing the open reading frame | Yes |
| Deletion | deletes of 3 or multiples of 3 nucleotides; do not change the open reading frame | Yes |
| Insertion | inserts of 3 or multiples of 3 nucleotides; do not change the open reading frame | Yes |
| Splicing | inserts or deletes of a number of nucleotides in the site at which splicing of an intron takes place | Yes |

| Other | | |
|---|---|---|
| Noncoding | Substitutions/Indels outside the gene | No |
| Unknown | Unknown | Unknown |

Of course, it is most convincing for biologist and oncologists if a genetic variation changes some protein to explain the linkage of genetic variations to clinical outcome measures.

Albeit is has become already apparent from scientific publications that just the frequency of genetic variations of a tumor is clearly related to outcome measures. Cancer patients with many, in fact hundreds of genetic variations of their tumor have a significantly better outcome than patients with few genetic variations in their tumor DNA.

### Construction of a prediction function to link sequence variations to the manifestation of an outcome measure

### Logical Operation with one or two Operands

First, it is determined whether a predefined segment of a gene, here indicated with A, contains a particular type of genetic variation or not. A = TRUE is assigned if and only if at least one particular genetic variation (or a combination of types of genetic variations) is present on segment A, otherwise A = FALSE is assigned. In mathematical terms, the inventors conjoin the presence of a particular genetic variation (or combinations of types of genetic variations) over all positions of a gene segment and assign the results of this conjunction to a variable, here A. If advantageous for the prediction, the inventors can use the negation of the result of such a conjunction, here denoted with an exclamation mark in front of the symbol assigned to this segment, here A. Table C shows the truth table of the negation.

**Table C: Truth Table Negation**

| A | Negation !A |
|---|---|
| FALSE | TRUE |
| TRUE | FALSE |

Such variables, denoting the existence of a particular type of genetic variation on disjunctive gene segments, here denoted with A and B, can be combined using one of the logical operators given in tables D and E. It is known to skilled persons that such functions are ambiguous and are easily transformed using the rules of Boolean algebra. For example, A And B is the same as B And A, the law of commutability applies to all operators but the material implication and their negation. In digital electronics the Nand gate is used to represent other logical operations, as one can show using the truth tables that !A is equivalent to A Nand A, A And B is equivalent to (A Nand B) Nand (A Nand B), and A Or B is equivalent to (A Nand B) Nand (B Nand B).

**Table D: Truth Tables of Conjunction and Disjunction**

| A | B | Conjunction | Negation of Conjunction | Disjunction | Negation of Disjunction |
|---|---|---|---|---|---|
| | | A And B | A Nand B | A Or B | A Nor B |
| FALSE | FALSE | FALSE | TRUE | FALSE | TRUE |
| FALSE | TRUE | FALSE | TRUE | TRUE | FALSE |
| TRUE | FALSE | FALSE | TRUE | TRUE | FALSE |
| TRUE | TRUE | TRUE | FALSE | TRUE | FALSE |

Such transformations would defeat one of the purposes of the intervention, namely to produce prediction functions that are interpretable by biologists and/or oncologists. Likewise, the inventors could transform all logical operations in conjunctive or disjunctive normal form to make them unambiguous again with the loss of biological interpretability.

The reason for using logical operators to combine information on sequence variations is as follows. Typically, sequence variations in tumors are sparse. There are a few so-called hot-spots, which harbor up to 16% of all known variations in a tumor entity. Most importantly, the vast majority of sequence variations in tumors occur in a random fashion. Therefore, the information needs to be aggregated to be useful for predictive purposes. That is exactly what these logical operators do.

**Table E: Truth-Tables of Equivalence and Implication**

| A | B | Equivalence | Exclusive Disjunction | Material Implication | Negation of Material Implication |
|---|---|---|---|---|---|
| | | A Eqv B | A Xor B | A Imp B | A Nimp B |
| FALSE | FALSE | TRUE | FALSE | TRUE | FALSE |
| FALSE | TRUE | FALSE | TRUE | TRUE | FALSE |
| TRUE | FALSE | FALSE | TRUE | FALSE | TRUE |
| TRUE | TRUE | TRUE | FALSE | TRUE | FALSE |

Next, the results of the aggregates, or better results of logical functions needs to be related to a particular manifestation of an outcome measure. This is facilitated by the cross classification of the result of one or more logical operations on two or more results of sequence variation analysis, see table F.

### Performance Measures

**Table F: Cross Classification of Results of Logical Operations and Manifestation of a Clinical Outcome Measure**

| Genetic Variation Present | Manifestation of a Clinical Outcome Measure | |
|---|---|---|
| | FALSE | TRUE |
| FALSE | True Negative TN | False Negative FN |
| TRUE | False Positive FP | True Positive TP |

When aggregated over some observations that are patients with analyzed DNA, typical performance measures can be derived as shown in Table G. These measures can be used to evaluate and optimize the relation between the aggregation of sequence variations using logical operations and manifestations of clinical outcome measures. Optimization means minimization of miss-classification rate or costs, or maximization of one of the other measures. Keep in mind that any function with an area under the receiver operating characteristic curve (AROC) of 0.5 or higher has potential clinical utility.

**Table G: Measures Derived from Two-valued Cross-Classification Tables**

| Name | Computation |
|---|---|
| Sensitivity | TP / (TP + FN) |
| Specificity | TN / (TN + FP) |
| Positive Predictive Value | TP / (TP + FP) |
| Negative Predictive Value | TN / (TN + FN) |
| Correct Classification Rate | (TN + TP) / (TN + FN + FP + TP) |
| Miss-Classification Rate | (FN + FP) / (TN + FN + FP + TP) |
| Area under the Receiver Operating Characteristic Curve (AROC) | ½ TP / (TP + FN) + ½ TN / (TN + FP) |
| Odds-Ratio | (FP * FN) / (TN * TP) |
| Negative Jaccard Ratio | TN / (FP + TN + FN) |
| Positive Jaccard Ratio | TP / (FP + TP + FN) |
| Combined Jaccard Ratio | ½ TN / (FP + TN + FN) + ½ TP / (FP + TP + FN) |
| Cost | Cost(TP) * TP + Cost(FN) * FN + Cost(FP) * FP + Cost(TP) * TP |

### Construction of Predictive Functions

The inventors implanted two strategies to construct predictive functions, a retrospective approach and a prospective approach. While the retrospective approach uses all available data, the prospective approach uses a double nested bootstrap procedure.

Briefly, in the double nested bootstrap procedures data of all available cases/observations are split in three groups:
- The outer loop: A discovery set comprised of ∼63% of all data, and a prospective validation set comprised of the rest.
- The inner loop: The discovery set is split again in two groups, again ∼63% are used to construct a prediction function, this is called the learning set. The rest is called the internal validation set.

The inner loop procedure: After construction of the prediction function, and assessments of its performance, the prediction function is applied to the internal validation set. If the performance within the internal validation set is within the 95% confidence limits of the performance of the learning set, the prediction function is a candidate for prospective validation. The discovery set is randomly re-split in a set for construction of a prediction function, and an internal validation set. Again, the performance is evaluated on both sets. The inner loop is repeated many times, typically 100 times or more. The means of the measures of the performance of the repetitions is used to decide which prediction function shall be evaluated in a strict prospective fashion on the prospective validation set.

The outer loop procedure: In the outer loop the "best" prediction functions of the inner loops are assessed. Then the total set is again split randomly into the two sets of a prospective validation set and learning/internal validation set.

The outer loop procedure is also repeated many times, typically 100 or more times. Thus, the final result is a representation of 10000 or more repetitions.

The advantage of this approach is two-fold. First, the outer loop generates second order unbiased estimates for a future clinical validation. Second, the results are not prone to over fitting. The results are generalizable.

The disadvantage of this approach is also clear, only about 40% of the data are utilized for construction of prediction function and assessment of the performance. The function may perform better if more data are used. Hence the retrospective approach might perform better, in particular in small datasets. Of course, using all data is prone to over fitting the prediction function to the actual data and loss of generalizability.

In some sense one could argue that the bootstrap gives a pessimistic estimate of the performance while the retrospective approach results in optimistic estimates.

The construction of the prediction function can be likened to regression trees. The nodes are the values of the distinct segments of the genes, TRUE if a particular sequence variation is detected, false otherwise. Additionally, the negations are used as nodes. However, those and only those gene segments can be used which are two-valued with respect to the filtered function(s) in the dataset.

For example, the inventors observed 3 segments of 3 genes, namely KRAS, BRAF, and APC. The nodes would be KRAS, !KRAS, BRAF, !BRAF, APC, and !APC. Then, the inventors note the performance of each node using the measure of the outcome, either using the bootstrap or the retrospective approach.

Next, the inventors used the logical functions given in tables D and E, to generate logical combinations, or prediction functions. Just to give the first using the node KRAS from the KRAS-BRAF-APC example, the next layer of nodes within the tree would represent: KRAS And BRAF, KRAS And !BRAF, KRAS And APC, KRAS And !APC, KRAS Nand BRAF, KRAS Nand !BRAF, KRAS Nand APC, KRAS Nand !APC, KRAS Or BRAF, KRAS Or !BRAF, KRAS Or APC, KRAS Or !APC, KRAS Nor BRAF, KRAS Nor !BRAF, KRAS Nor APC, KRAS Nor !APC, KRAS Eqv BRAF, KRAS Eqv !BRAF, KRAS Eqv APC, KRAS Eqv !APC, KRAS Xor BRAF, KRAS Xor !BRAF, KRAS Xor APC, KRAS Xor !APC, KRAS Imp BRAF, KRAS Imp !BRAF, KRAS Imp APC, KRAS Imp !APC, KRAS Nimp BRAF, KRAS Nimp !BRAF, KRAS Nimp APC, KRAS Nimp !APC
Once the information on one gene segment is part of the prediction function, is not used again; this restricts the number of layers in the tree to the number of different segments plus 1. However, the number of nodes within each layer is enormous. The foremost reason not to reuse a segment again is biological interpretability. [Recursive partitioning in contrast may reuse the same variable over and over again.]

First tries to just add segment information that increase the performance measure showed that it is always possible to find a local optimum in the solution space, but that is not necessarily the overall optimum. Hence, the inventors decided to compute the permutations of all possible combinations.

Taken together, the inventors have devised a method to identify and aggregate genetic variation information in functions that have clinical use for prediction of manifestations of clinical outcome measures, which allow for biological interpretation.

### Description of the invention with regard to solid tumors

In the following, the invention is be described in relation to several types and stages of solid tumors, namely breast cancer, lung cancer, skin cancer (melanoma), ovarian cancer, pancreas cancer, prostate cancer, stomach cancer, and colorectal cancer. It will be understood by a person skilled in the art that the invention can also be practiced in relation to other types of solid tumor cancer based on the general knowledge of the skilled person together with the description provided herein.

In the method predicting a manifestation of an outcome measure of a cancer patient, at least two genes are analyzed for sequence variations. For this purpose, the genes are partitioned into segments of appropriate length. The length of the segments may vary from 20 base pairs to 500 base pairs, preferably from 50 base pairs to 250 base pairs. Such segments allow for a convenient and accurate determination of the sequence in order to find sequence variations in the DNA sample form the cancer patient.

The at least two genes that are analyzed are associated with the outcome measure of the patient, i.e. they are associated with the solid tumor cancer disease of the patient. In a preferred embodiment of the invention, the at least two genes that are analyzed are chosen from a list of genes of Tables 1 to 8. Specifically, the genes associated with breast cancer are listed in Table 1; the genes associated with lung cancer are listed in Table 2; the genes associated with skin cancer (melanoma) are listed in Table 3; the genes associated with ovarian cancer are listed in Table 4; the genes associated with pancreas cancer are listed in Table 5; the genes associated with prostate cancer are listed in Table 6; the genes associated with stomach cancer are listed in Table 7; and the genes associated with colorectal cancer are listed in Table 8. For each gene listed with regard to a certain type of cancer, the number of sequence variations ("mutations") is given together with the number of samples that were analyzed and the mutation frequency resulting therefrom.

### Examples

In the following, the invention will be described in relation to several types and stages of solid tumors, namely in respect to colorectal cancer of stage II (predicting outcome), colorectal cancer of stages III and IV (predicting response to treatment), and in patient derived xenografts (PDXs) of colorectal tumors.

### Example 1: Prediction of Progression of Disease in Stage II Colorectal Cancer (retrospective analysis)

### Tables 9-17

173 patients with colorectal cancer of UICC stage II for which follow-up data of 3 years was available were selected from the prospective MSKK study. Macro-dissection of formalin-fixed, paraffin-embedded (FFPE) samples of 173 Patients with UICCC stage II colorectal cancer were used, for which a 3 year follow-up was available. 40 /173 patients were diagnosed with metastases in liver, lung, or peritoneum. 27/173 patients were diagnosed with secondary malignancies. 12/173 patients were diagnosed with local recurrences. 94/173 patients had no progression of disease event. Tumor tissues of all 173 patients were deep sequenced using a cancer panel of known cancer genes. 96 tumor tissues were also subjected to exome sequencing using the Illumina HiSeq. Raw sequence data was collected and analyzed.

Following DNA isolation, deep sequencing of selected cancer genes (oncogenes and tumor suppressor genes) with approx. 200 amplicons (^{∼} 27 kb). Two giga bases raw sequence per run was performed. Multiplexing was between 12fold, 24fold, 48fold and 96fold. At 96 plex, coverage within the 200 amplicons is 200 to 2.000 fold. At 24 plex, coverage is 1.000 to 8.000fold.

The number of screened patients from prospective multicenter MSKK study was 1481; 173 patients were selected from this group.

Progression of disease events are defined as: No progression of disease event within 3 years after resection of primary tumors, diagnosis of metastasis (liver, lung, peritoneal), diagnosis of local recurrence, and diagnosis of secondary malignancy.

The following selection criteria were applied:
- Histo-pathological diagnosis of CRC UICC stage II,
- At least 11 regional lymph nodes were examined and all lymph nodes were free of tumor cells,
- No clinical sign of metastatic disease at time of surgery,
- R0-Resection,
- No neo-adjuvant therapy,
- Tumor tissue available, macro dissected for enrichment of tumor fraction, and passed quality control by the studies reference pathologists (KK and KHK),
- Extracted DNA of sufficient amount and quality as shown by agarose gel electrophoresis and ultraviolet absorbance measurement,
- Patients alive at least 3 years after date of surgery without any sign of any progression of disease event, namely diagnosis of loco-regional recurrence, metastatic disease, or secondary malignancy - OR - patients diagnosed with one of the aforementioned progression of disease events within 3 years after date of surgery,
- No death within 30 days after surgery.

Below, examples of predictions functions that were found in retrospective analyses are described with respect to the tables. The prediction functions are based on missense sequence variations only (A) or on missense and nonsense sequence variations only (B) or on missense and nonsense and silent and synonymous mutations only (C).

### Example 1. A. Missense sequence variations only

Table 9 shows prediction functions and their performance based on sequence variations of one gene only. !TP53 is the highest performing single marker followed by KRAS and !APC, if AROC is optimized. If optimization uses the combined Jaccard ratio, then !TP53 is the best performing single marker followed by KRAS and PIK3CA. The preferred function for prediction of metastasis using missense sequence variations is !TP53.

Table 10 shows the performance of prediction functions for 1 up to 6 genes, based on missense variations only. !TP53 has the largest single impact. The second best marker is XOR BRAF or its logic equivalence XOR !BRAF. The third best marker is OR SMO or its logical equivalent. The fourth, fifth and sixth marker !APC AND !PTEN AND !RET contribute only to the specificity of the function and increases specificity by 6 % or 32 false positives versus 37 false positives in the function of 3.

If !TP53 is omitted completely in a function, the sensitivity decreases. Example: BRAF OR SMO AND !APC AND !PTEN AND !RET S+ 0.15, S- 0.936, PPV 0.500, NPV 0.721, AROC 0.540, CJR 0.409.
With a function length of six, the maximum of performance is reached. Longer functions do not perform any better. Prediction functions containing 7 or more markers have a worse performance.

Functions optimized for AROC have a better performance with respect to sensitivity than functions optimized for combined Jaccard ratio. The position of a given marker in the function is not critical. !TP53 can be at the first, second or third position in a function of 3 or even at the sixth position in a function of 6 markers.

The position of XOR BRAF or of OR SMO as well as the position of !APC or !PTEN or !RET can be changed without change of performance.

Table 11 shows further preferred prediction functions.

### Example 1. B. Missense and Nonsense sequence variations only

### Mutations N1=354; N2=465

Table 12 shows preferred prediction functions based on missense and nonsense sequence variations only and their clinical performance (sequence variations N1=354; N2=465), Performance of Best One to Six Genes
As can be seen in Table 12, adding further genes up to 8 does not change performance of a function. Adding more than 8 sequence variation statuses leads to a decrease of performance.

Table 13 shows further preferred prediction functions for determining progression of disease in Stage II Colorectal Cancer as an outcome measure. The addition of nonsense sequence variations does not change the structure of the function, as there are only 42 additional sequence variations and preferentially only in TP53 and APC.

### Example 1. C. Missense and Nonsense and Silent and Synonymous Mutations Only

### Mutations N1=1044; N2=800

Table 14 shows preferred prediction functions based on missense and nonsense and silent and synonymous sequence variations only (sequence variations N1=1044; N2=800) and their performance.

Table 15 shows further preferred prediction functions based on missense and nonsense and silent and synonymous sequence variations only (sequence variations N1=1044; N2=800) and their performance.

As can be seen, the use of missense sequence variations for predicting progression of disease is preferred in this example. Nonsense mutations add a little in performance, especially regarding specificity. A function length of between 1 and 6 sequence variation statuses is preferred.

Table 16 shows best performing functions with missense and nonsense sequence variations and with a sensitivity > 70%.

Table 17 shows best performing functions with missense mutations only and with a sensitivity > 70 %.

### Example 2: Prediction of Progression of Disease in Stage II Colorectal Cancer (prospective analysis)

Table 18: Results of prediction functions were compiled based on missense and nonsense sequence variations in a prospective study. Data not adjusted.

### Example 3: Prediction of Response to Treatment to Bevacizumab plus Chemotherapy in Patients with Advanced, Metastatic Colorectal Cancer of UICC Stage IV (retrospective analysis)

### Tables 19-26

33 Patients with Stage IV Colorectal Cancer for which Follow-up according to RECIST criteria was available. Patients treated with Bevacizumab in combination with chemotherapy. 11 of 33 patients experienced response to treatment according to RECIST (total remission, partial remission). 22 of 33 patients experienced no response to treatment according to RECIST (stable disease, progression of disease).

Primary tumor tissue samples (FFPE, frozen samples) were macro-dissected, followed by DNA isolation. Deep sequencing of 212 amplicons in a panel of 40 selected cancer genes were performed in each of the 33 patients allowing high coverage for each base pair (ca. 34 kilobases of sequence for each patient). The coverage per base was 300-4.000 fold. This high coverage allows identifying mutations with great confidence.

### Example 3. A. Missense and Nonsense Mutations Only

Table 19 shows prediction functions and performance data for the Prediction of Response to Treatment to Bevacizumab plus Chemotherapy in Patients with Advanced, Metastatic Colorectal Cancer of UICC Stage IV (Mutations N1=256, N2=96; Minimum of 1 Patient mutated in any given cancer gene; N=33: 11 Patients with Response; 33 Patients with no Response); the Performance of Single Genes is shown.

!TP53 is the strongest single marker followed by KRAS and !APC if AROC (area under the curve) is optimized for. !TP53 is the strongest single marker followed by KRAS and PIK3CA if AROC (Combined Jaccard Ratio) is optimized for. For this application, a function of two genes is preferred comprising at least!TP53 or its equivalent TP53.

### Example 3. A1. Mutations Count 1: Gene must be mutated at least in 1/33 Patients

Table 20 shows the performance of 1 to 6 Genes wherein a gene must be mutated at least in 1/33 patients.

### Example 3. A2. Mutations Count 2: Gene must be mutated at least in 2/33 Patients (>5 % frequency)

Table 21 shows the performance of 2 to 6 Genes wherein a gene must be mutated at least in 2/33 patients.

### Example 3. A3. Mutations Count 5: Gene must be mutated at least in 5/33 Patients (5% to 30% frequency)

Table 22 shows the performance of 2 to 6 Genes wherein a gene must be mutated at least in 2/33 patients.

The data presented above shows that TP53, PIK3CA, !SMAD4 and !CTNNB1 have the largest single impact on performance of the prediction function. The second best marker after !TP53 is OR Kit or AND PIK3CA. The second best marker after PIK3CA is AND KRAS. The second best marker after !SMAD is OR ATM, and the second best marker after !CTNNB1 is AND ITP53.

With a function length of four genes, the maximum performance for AROC and CJR is reached for !CTNNB! AND !TP53 OR KIT AND MET and its equivalent string !TP53 OR KIT AND !CTNNB1 AND MET.

All gene markers can be moved freely from position 1 to 4 within the function without loosing performance.

With string length of five genes, the maximum performance for AROC is !TP53 OR KIT AND CTNNB1 AND !MET OR SMAD4, and for the combined Jaccard ration (CJR) the maximum performance is !CTNNB1 AND !TP53 AND !KDR AND !MET OR PIK3CA.

The difference between the performance of the seven best performance signatures is marginal and within the 95 % confidence limits. Most signature reach maximum performance with a function length of 5 genes, only one signature with a function length at 4 or 6 genes. Longer functions with more than 5 or 6 genes do not have an increased performance. Functions optimized by AROC have a better performance with respect to sensitivity than functions optimized by combined Jaccard ratio. The position of a given marker in the string is not critical.

### Example 3. B. Missense sequence variations only, N1=210; N2=72

Table 23 shows the performance of functions containing 3, 4 and 5 sequence variation statuses, based on missense sequence variations only.

The table shows that a function obtained with missense mutations alone has a slightly lower performance than function with missense and nonsense mutations. This might be due to the slightly increased number of mutations.

### Example 3. C. Missense AND Synonymous Mutations, N1=352 N2=134

Table 24 shows the performance of functions containing 5, 6 and 7 sequence variation statuses, based on missense and synonymous sequence variations only.

### Example 3. D. Missense AND Nonsense AND Synonymous AND Silent N1=565; N2=205

Table 25 shows the performance of functions containing 4, 5, and 3 (the latter with mutation count 5) sequence variation statuses, based on missense and nonsense and synonymous sequence variations only.

### Example 4 Prediction of Response to Treatment to Bevacizumab plus Chemotherapy in Patients with Advanced, Metastatic Colorectal Cancer of UICC Stage IV (prospective analysis)

Table 25B shows the performance of exemplary functions.

### Example 5 Prediction of Response to Treatment to Bevacizumab Monotherapy in Patient derived Xenograft Models (data on 67 PDX models)

Transplantation of 239 human, primary colorectal tumors of patients with colorectal cancer of all four UICC stages was performed onto nude mice. 149 xenograft models were successfully engrafted. 133 xenograft models were quality checked versus matched primary human tumors. 75 tumors/xenograft models were selected for large therapy treatment experiments with three approved drugs in mCRC patients: Oxaliplatin, Cetuximab, and Bevacizumab. For each drug and each of the 67 xenograft models, five mice were treated in addition to five control animals (335 animals plus 335 controls per drug). At the end of the therapy experiment, the median diameter of the tumors (C) of the 5 control animals is divided by the median diameter of the five treated animals (T).

Table 26 shows the performance of functions containing 1, 2, 3, 4, 5, 6, 7, and 8 sequence variation statuses, based on missense and nonsense and synonymous sequence variations only. N1=131, N2=131.

Table 27 shows the performance of a preferred function (T/C <25. Mutation Count 5; R=11; NR=56; Tumor growth of PDXs must be inhibited by at least 75%).

Table 28 shows the performance of preferred functions (T/C <35. Mutation Count 5; R=19; NR=48).

### Example. Response to Bevacizumab plus Chemotherapy in Patients with metastatic colorectal cancer

Table 29 shows the best performing signatures with missense and nonsense, a mutation count of 2 (5% frequency) and with a sensitivity > 70%.

Table 30 shows the best performing signatures with missense and nonsense, a mutations count of 5 (5-30% frequency) and with a sensitivity >70%.

### Example. Response to Bevacizumab monotherapy in patient derived xenografts (PDXs)

Table 31 shows preferred functions (T/C </=30,13 Responder PDXs, 54 Nonresponder PDXs)
Table 32 shows preferred functions T/C</=35, 19 Responder PDXs, 48 Nonresponder PDXs)
Table 33 shows preferred functions (T/C </=25, 11 Responder PDXs, 56 Nonresponder PDxs)

### Example 5 Prediction of Progression of Disease in Stage III Colorectal Cancer (retrospective analysis)

### Tables 34-35

350 patients with colorectal cancer of UICC stage III for which follow-up data of at least two years was available were selected from the prospective MSKK study. The following selection criteria were applied:
- Pathological confirmed colorectal carcinoma in UICC stage III
- At least one positive lymph node
- No neo-adjuvant therapy
- R0 resection
- No clinical evidence of metastases
- No other clinical exclusion criteria
- Pass pathological QC tumor tissue
- Pass QC tumor DNA
- At least two years progression free survival time or diagnosis of a progression of disease event.

Patients had received standard adjuvant chemotherapy including 5-fluorouracil, leucovorin, and oxaliplatin (FOLFOX scheme), or 5-fluorouracil and leucovrin. Some patients received oral capecitabine instead of infusional 5-fluorouracil. Progression of disease events were defined as: (i) no progression within 3 years, four years or five years after resection of primary tumors, (ii) diagnosis of metastasis (liver, lung, peritoneal), (iii) diagnosis of local recurrence, and diagnosis of secondary malignancy.

Of the 350 patients with a two year follow up, 24 patients had distant metastasis (mainly liver metastasis), 4 patients had a local recurrence or a secondary malignancy, and 13 patients had death as progression event. 309/350 patients had no progression of disease event. Of the 289 patients with a three year follow up, 42 patients distant metastasis (mainly liver metastasis), 6 had a local recurrence or a secondary malignancy, and 14 patients had death as progression event. 227/289 patients had no progression of disease event. Of the 242 patients with a four year follow up, 57 patients had distant metastasis (mainly liver metastasis), 8 had a local recurrence or a secondary malignancy, and 16 patients had death as progression event. 161/242 patients had no progression of disease event. Of the 186 patients with a five year follow up, 66 patients had distant metastasis (mainly liver metastasis), 6 patients had a local recurrence or a secondary malignancy, and 20 patients had death as progression event. 94/186 patients had no progression of disease event.

Macro-dissection of cryo tumor and FFPE tumor samples of 350 Patients with stage III colorectal cancer were used. Tumor DNA was isolated using an automated method on the Qiacube robot (Qiagen, Germany). Tumor DNA was quantified, and at least 250ng of tumor DNA of all 350 patients were deep sequenced using the MiSeq sequencer (Illumina, Inc.) and a cancer panel of 37 known cancer genes organized in 120 distinct amplicons. Up to 96 sequenced samples were multiplexed per MiSeq run. Raw sequence data was collected and analyzed.

Below, examples of predictions functions that were found in retrospective analyses are described with respect to the tables. The prediction functions are based on missense and nonsense sequence variations only, which alter the function of the encoded protein.

### Example 6.1

Table 34 shows various prediction functions of the best performing genes for predicting metastasis in distant organs as progression of disease in patients with colorectal cancer of stage III who underwent R0 resection and were treated using adjuvant chemotherapy. Overall survival is the event time.

In the group of patients with a three year follow up (N=233), 42 patients had a metastasis event while 191 patients remained without any progression of disease event. SMAD4mi (nonsense mutations in the SMAD4 gene) was the strongest single marker of 11 cancer genes which showed missense and nonsense mutations in at least five patients. SMAD4mi showed a sensitivity S+ of 0,262 and a specificity (S-) of 0,937, and an area under the receiver operating characteristic curve (AROC) of 0,600. Adding the next marker OR KITmi improved S+ to 0.500, reduced S- to 0.817 and improved AROC to 0.658. The prediction function of the two markers SMAD4mi OR KITmi reads as follows: missense mutations in the SMAD4 gene, or missense mutations in the KIT gene, or missense mutations in both the SMAD4 gene and the KIT gene predict patients with colorectal cancer of stage III with higher risk of metastasis as progression of diseases who have a three year follow up time. Adding a third marker OR FBXW7mi improves the AROC to 0.684. The prediction function of three markers SMAD4mi OR KITmi OR FBXW7 reads as follows: missense mutations in the SMAD4 gene, or missense mutations in the KIT gene, or missense mutations in the FBXW7 gene, or missense mutations in any two of the three genes, or missense mutations in all three genes predict patients with colorectal cancer of stage III with higher risk of metastasis as progression of disease who have a three year follow up time. The prediction function can be further improved by adding two more markers XOR ATMmi and XOR METmi. The prediction function with these five markers, SMAD4mi OR KITmi OR FBXW7mi XOR ATMmi XOR METmi, has an AROC of 0.716. Any further marker does not increase the accuracy of the prediction function.

In the group of patients with a four year follow up (N=192), or a five year follow up (N=142), we observed the same prediction function of three markers: !APCns OR SMAD4mi OR FBXW7mi. !APCns (no nonsense mutations in the APC genes) turned out to be the strongest single marker of the 11 cancer genes which showed missense and nonsense mutations in at least five patients. !APCns showed a sensitivity (S+) of 0.509, a specificity (S-) of 0.696, and an area under the operating receiver characteristics curve AROC of 0.603 (four year follow up). In the patient group with five year follow up, !APCns had a S+ of 0.485, S- of 0.763, and an AROC of 0.624. The next strongest marker was OR SMAD4mi improving the AROC to 0.642 and 0.658 in the patients with four or five year follow up, respectively. Finally, the maximum of the prediction function was reached by adding as third marker: OR FBXW7mi. The resulting prediction function !APCns OR SMAD4mi OR FBXW7mi showed an AROC of 0.660 and 0.678 in the patients with four years or five years observation time, respectively.

Table 35 shows various prediction functions in the same patient groups with colorectal cancer of stage III if progression free survival (PFS) is the event time and not overall survival and using distant metastasis as the event. Prediction functions are very similar to those shown in Table 34. The best performing signature for patients with a follow up time of 5 years is !APCns OR FBXW7 OR SMAD4mi with a S+ of 0.629, a S- of 0.678 and an AROC of 0.653. This prediction function differs only from Table 34 in that OR FBXW7 is at the second position and OR SMAD4mi is at the third position.

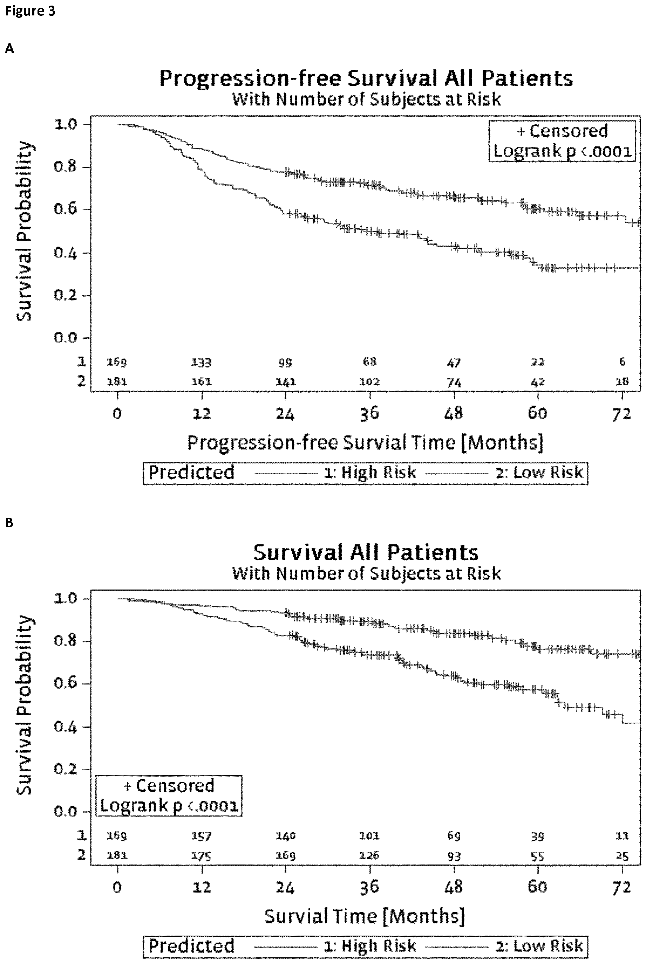

Figure 3 shows the survival curves of the best performing prediction function !APCns OR SMAD4mi OR FBXW7mi with progression free survival (PFS) and overall survival (OS) as the event time. In the survival curve with PFS as the event time a difference of 40 months between the high-risk group and the low risk group was observed. This difference is statistical significant (Logrank p< 0.001. The Hazard ratio is 2.043. In the survival curve with OS the hazard ratio is 2.551 and thus even higher.

### Tables

Table 1: Genes associated with breast cancer.
Table 2: Genes associated with lung cancer.
Table 3: Genes associated with skin cancer (melanoma).
Table 4: Genes associated with ovarian cancer.
Table 5: Genes associated with pancreas cancer.
Table 6: Genes associated with prostate cancer.
Table 7: Genes associated with stomach cancer.
Table 8: Genes associated with colorectal cancer.
Table 9: Prediction of Progression of Disease in Stage II Colorectal Cancer, Missense Mutations Only (Sequence variations: N1=396, N2=296, Minimum 2 Patients mutated in any given cancer gene; N=134, 40 Patients with Metastases, 94 Patients with no Recurrence)
Table 10: Prediction of progression of disease in Stage II Colorectal Cancer, Missense Mutations Only, Performance of One to Six Genes
Table 11: Prediction of Progression of Disease in Stage II Colorectal Cancer, Missense sequence variations Only, Other preferred prediction functions
Table 12: Prediction of Progression of Disease in Stage II Colorectal Cancer, Missense and Nonsense sequence variations Only (sequence variations N1=354; N2=465), Performance of Best One to Six Genes
Table 13: Prediction of Progression of Disease in Stage II Colorectal Cancer, Preferred prediction functions
Table 14: Prediction of Progression of Disease in Stage II Colorectal Cancer, Missense and Nonsense and Silent and Synonymous sequence variations Only (sequence variations N1=1044; N2=800); Performance of Best One to Six Genes
Table 15: Prediction of Progression of Disease in Stage II Colorectal Cancer, Missense and Nonsense and Silent and Synonymous Mutations Only (sequence variations N1=1044; N2=800); preferred prediction functions
Table 16: Prediction of Progression of Disease in Stage II Colorectal Cancer, Best performing prediction function with missense and nonsense mutations and with a sensitivity > 70 %
Table 17: Prediction of Progression of Disease in Stage II Colorectal Cancer, best performing prediction function with missense mutations only and with a sensitivity > 70%
Table 18: Results of prediction functions were compiled based on missense and nonsense sequence variations in a prospective study. Data not adjusted.
Tables 19 to 33: Prediction of Response to Treatment to Bevacizumab plus Chemotherapy in Patients with Advanced, Metastatic Colorectal Cancer of UICC Stage IV
Table 19: shows prediction functions and performance data for the Prediction of Response to Treatment to Bevacizumab plus Chemotherapy in Patients with Advanced, Metastatic Colorectal Cancer of UICC Stage IV (Sequence variations N1=256, N2=96; Minimum of 1 Patient mutated in any given cancer gene; N=33: 11 Patients with Response; 33 Patients with no Response); the Performance of Single Genes is shown.
Table 20: performance of 1 to 6 Genes wherein a gene must be mutated at least in 1/33 patients.
Table 21 shows the performance of 2 to 6 Genes wherein a gene must be mutated at least in 2/33 patients.
Table 22 shows the performance of 2 to 6 Genes wherein a gene must be mutated at least in 5/33 patients.
Table 23 shows the performance of functions containing 3, 4 and 5 sequence variation statuses, based on missense sequence variations only.
Table 24 shows the performance of functions containing 5, 6 and 7 sequence variation statuses, based on missense and synonymous sequence variations only.
Table 25 shows the performance of functions containing 4, 5, and 3 (the latter with mutation count 5) sequence variation statuses, based on missense and nonsense and synonymous sequence variations only.
Table 26 shows the performance of functions containing 1, 2, 3, 4, 5, 6, 7, and 8 sequence variation statuses, based on missense and nonsense and synonymous sequence variations only. N1=131, N2=131.
Table 27 shows preferred functions (T/C <25. Mutation Count 5; R=11; NR=56; Tumor growth of PDXs must be inhibited by at least 75%).
Table 28: shows preferred functions (T/C <35. Mutation Count 5; R=19; NR=48).
Table 29 shows a preferred signature with missense and nonsense, a mutation count of 2 (5% frequency) and with a sensitivity > 70%.
Table 30 shows preferred signatures with missense and nonsense, a mutations count of 5 (5-30% frequency) and with a sensitivity >70%.
Tables 31 to 33: Response to bevacizumab monotherapy in patient-derived xenografts (PDXs).
Table 31 shows preferred signatures (T/C </=30, 13 Responder PDXs, 54 Nonresponder PDXs).
Table 32 shows a preferred signature (T/C</=35, 19 Responder PDXs, 48 Nonresponder PDXs).
Table 33 shows a preferred signature (T/C </=25, 11 Responder PDXs, 56 Nonresponder PDxs).
Table 34 shows prediction functions and performance data for the prediction of progression of disease in patients with colorectal cancer of stage III who underwent surgical R0 resection followed by standard adjuvant chemotherapy. Prediction functions were based on deep sequencing data of 37 key cancer genes organized in 120 amplicons and analysis of missense and nonsense mutations if they occurred in at least five patients using Boolean operators. Patients had different follow up times: 365 days (1 year), 731 days (2 years), 1.096 days (3 years), 1.461 days (4 years), and 1.826 days (5 years). Metastasis to distant organs was the measured event compared to patients who did not show any event (metastasis, local recurrence, secondary malignancy, death) in the same follow up period. Event time is overall survival (OS).
Table 35 shows prediction functions and performance data for the prediction of progression of disease in patients with colorectal cancer of stage III who underwent surgical R0 resection followed by standard adjuvant chemotherapy. Prediction functions were based on deep sequencing data of 37 key cancer genes organized in 120 amplicons and analysis of missense and nonsense mutations if they occurred in at least five patients using Boolean operators. Patients had different follow up times: 365 days (1 year), 731 days (2 years), 1.096 days (3 years), 1.461 days (4 years), and 1.826 days (5 years). Metastasis to distant organs was the measured event compared to patients who did not show any event (metastasis, local recurrence, secondary malignancy, death) in the same follow up period. Event time is progression-free survival (PFS).

### Figures

**Figure 1****:** Discovery Optimization: AROC = Area under the Receiver Operating Characteristic Curve The signature with 10 genes reads: !TP53 Eqv !BRAF Or SMAD4 Or ATM Or KRAS And !FLT3 And !FBXW7 Or PIK3CA Or KIT Or MET
**Figure 2****:** The signature with 6 genes reads: !TP53 XOR BRAF AND !FLT3 OR ATM OR PIK3CA AND !FBXW7

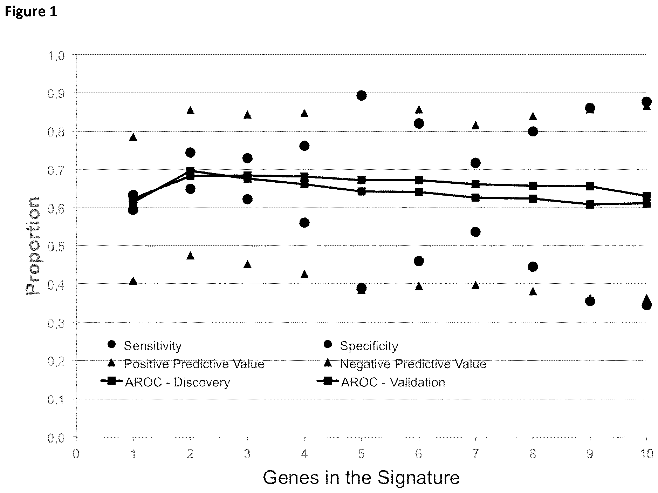

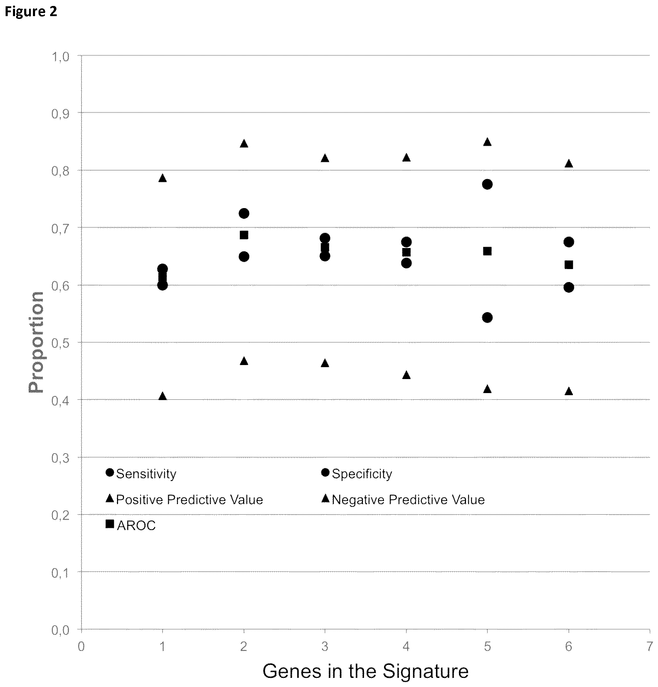

Figures 1 and 2 relate to the stratification of patients with colorectal cancer of UICC Stage II using prognostic mutation signatures obtained by deep amplicon sequencing of cancer genes.

Figure 1 shows results of a bootstrap "signature" (prediction function) finding algorithm for prediction of metastasis. In words, the signature expresses: Those patients who have neither missense nor nonsense variations or have missense or nonsense variations in both genes, TP53 and BRAF, have the highest likelihood of developing metastatic disease. The addition of SMAD4 missense or nonsense variation shows no improvement, thus holds not up in the prospective validation.

From the 13 genes displaying statistically significant missense or nonsense mutations (also found in the COSMIC database), TP53 has the largest single gene impact on performance of the signature with respect to predicting metastasis. The element !TP53 which reads "No missense and nonsense mutations in TP53" has a sensitivity (S+) of 0.59, a specificity (S-) of 0.63, a positive predictive value (PPV) of 0.41 and negative predictive value (NPV) of 0.78.

The first element !TP53 is now connected with the second element !BRAF using the Boolean operator Eqv. The meaning of the first two elements of the signature !TP53 Eqv !BRAF is as follows: "Patients who have neither missense nor nonsense mutations in TP53 and BRAF, or patients who have missense or nonsense mutations in both genes, have the highest likelihood of developing metastatic disease". !TP53 Eqv !BRAF has the following performance: S+ 0.74, S- 0,65, PPV 0.48, NPV 0,86, AROC 0.69.

The addition of Eqv !BRAF increases S+ by 0.15 and S- by 0,02. The addition of OR SMAD4 missense or nonsense mutations shows no improvement. This holds not up in the prospective validation.

Further extension of the signature by OR ATM OR KRAS does not improve overall performance as measured by the AROC. However, a signature with five elements !TP53 Eqv !BRAF Or SMAD4 OR ATM or KRAS leads to increased sensitivity of 0.89, however on the expense of a lower specificity of 0.39. Such a signature with high sensitivity might be of use for selection of patients at high risk of metastasis for a chemotherapy study. The signature would predict 36 True Positives of the 40 patients with the risk of metastasis correctly. Only 4 patients with high risk of metastasis would not be identified and would be False Negatives. However, of the 94 patients with no risk of progression the signature would only identify 37 correctly as True Negatives, thus leading to 57 False Positive patients.

The results of the prospective discovery can be complemented by the retrospective analysis shown in figure 2. Almost all genes discovered prospectively are also found in a retrospective fashion. Naturally, the logical operators and the sign of the status may change. The best retrospective signature is !TP53 XOR BRAF which is almost identical to !TP53 Eqv !BRAF.

In the retrospective analysis the addition of OR PIK3CA to the function of four elements !TP53 XOR BRAF AND !FLT3 OR ATM leads to an increased sensitivity of 0.775 and a decreased specificity of 0.543. Thus 32 of the 40 high risk patients and 51 of the 94 patients with no risk of progression of disease were identified correctly. Addition of OR KRAS instead of OR PIK3CA leads to a further increase of sensitivity similar to the prospective analysis.

The signature !TP53 XOR BRAF AND !PIK3CA has a sensitivity of 55% and a specificity of 71%. By exchanging AND ! PIK3CA through OR PIK3CA one achieves a sensitivity of 77.5% and a specificity of 54.3%, hence one has swapped sensitivity for specificity without change to positive, or negative predictive value, or AROC.

**Figure 3****:** Survival curves for the best performing prediction function !APCns OR SMAD4mi OR FBXW7mi in patients with colorectal cancer of stage III. A: Progression-free survival of all patients; B: Survival of all patients. (For A and B: upper curve: low risk, lower curve: high risk).
PFS: High Risk Median Survival Time 37.2 months (95%-CI: 26.283 -51.450);
Low Risk Median Survival Time 77.4 (95%-CI: 65.347 - ?) months;
Hazard Ratio: 2.043 (95% CI: 1.496 - 2.7892).
Survival: Hazard Ratio: 2.551 (95% CI: 1.669 - 3.756)

### Materials and Methods

### Extraction of nucleic acids

Extraction of nucleic acids from the tissue samples was performed using the AllPrep DNA/RNA Mini Kit (Qiagen, Hilden). The preparation was done on a Qiacube robot from Qiagen. Starting material was approximately 10-20 mg of cryo preserved tumor tissue cut in 4µm slices on a cryotom.

Before starting the protocol the following things need to be prepared:
- Add 10 µl β-mercaptoethanol per 1 ml Buffer RLT Plus. Dispense in a fume hood and (Buffer RLT Plus is stable at room temperature (15-25ºC) for 1 month after addition of β-ME.)
- Buffer RPE, Buffer AW1, and Buffer AW2 are each supplied as a concentrate. Before using for the first time, add the appropriate volume of ethanol (96-100%), as indicated on the bottle, to obtain a working solution.
- Buffer RLT Plus may form a precipitate upon storage. If necessary, redissolve by warming, and then place at room temperature.

### DNA Isolation

Add 350 µl of Buffer RLT Plus and vortex well until tissue gets dissolved. Centrifuge 3 minutes at maximum speed (14000g). Transfer the supernatant directly into a 2 ml Safe-Lock tube.

Prepare the Qiacube robot:
- Put filter-tips in racks (1000µL)
- Set 2mL safe lock tubes (Eppendorf) containg the sample in the shaker (positions 1-12)
- Prepare DNAse incubation mix by dissolving the lyophilised DNase I (1500 Kunitz units) in 550 µl RNase-free water
- Fill the reagent-Rack bottles:
   1. *Position 1*: Buffer RLT
   2. *Position 2*: 96-100% EtOH
   3. *Position 3*: empty
   4. *Position 4*: Buffer FRN
   5. *Position 5*: Buffer RPE
   6. *Position 6*: RNase-free water
- Load the rotor adapter for DNA isolation
   1. Position 1 : empty
   2. Position 2 : DNA column (white, lid cut off)
   3. Position 3 : elution tube for DNA
- Start program RNA / Allprep DNA RNA FFPE / part A DNA
- After finishing the program remove the column (discard) and store the elution tube on ice.

### RNA Isolation

Prepare the Qiacube robot:
1. Position 1 : RNeasy Minelute spin column (rosa, lid cut off)
2. Position 2 : empty
3. Position 3 : Elution-tube für RNA
   - Start programm RNA / Allprep DNA RNA FFPE /part B Total RNA (including small RNA)

After finishing the program the RNA tubes are stored at -80°C. The used rotor adapter are discarded and the robot is cleaned up.

### Preparation of the MiSeq Library - Sequencing

**TruSeq Amplicon - Cancer Panel Acronyms**

| Acronym | Definition |
|---|---|
| ACD1 | Amplicon Control DNA 1 |
| ACP1 | Amplicon Control Oligo Pool 1 |
| AFP1 | Amplicon Fixed Panel 1 |
| CLP | CLean-up Plate |
| DAL | Diluted Amplicon Library |
| EBT | Elution Buffer with Tris |
| ELM3 | Extension Ligation Mix 3 |
| FPU | Filter Plate Unit |
| HT1 | Hybridization Buffer |
| HYP | HYbridization Plate |
| IAP | Indexed Amplification Plate |
| LNA1 | Library Normalization Additives 1 |
| LNB1 | Library Normalization Beads 1 |
| LNS1 | Library Normalization Storage Buffer 1 |
| LNW1 | Library Normalization Wash 1 |
| LNP | Library Normalization Plate |
| OHS1 | Oligo Hybridization for Sequencing Reagent 1 |
| PAL | Pooled Amplicon Library |
| PMM2 | PCR Master Mix 2 |
| SGP | StoraGe Plate |
| SW1 | Stringent Wash 1 |
| TDP1 | TruSeq DNA Polymerase 1 |
| UB1 | Universal Buffer 1 |

### Hybridization of Oligo Pool

During this step, a custom pool containing upstream and downstream oligos specific to the targeted regions of interest is hybridized to your genomic DNA samples.
- Remove the AFP1, OHS1, ACD1, ACP1, and genomic DNA from -15° to -25°C storage and thaw at room temperature.
- Set a 96-well heat block to 95°C.
- Pre-heat an incubator to 37°C to prepare for the extension-ligation step.
- Create your sample plate layout using the Illumina Experiment Manager or the LabTracking Form. Record the plate positions of each sample DNA/AFP1, ACD1/ACP1(TSCA_Control), and index primers.
- Apply the HYP (HYbridization Plate) barcode plate sticker to a new 96-well PCR plate.
- Add 5 µl of control DNA ACD1 to 1 well in the HYP plate for the assay control.
- Add 5 µl of genomic DNA to each remaining well of the HYP plate to be used in the assay.
- Using a multi-channel pipette, add 5 µl of AFP1 to the wells containing genomic DNA.(Change tips after each column to avoid cross-contamination.)
- If samples are not sitting at the bottom of the well seal the HYP plate with adhesive aluminum foil and centrifuge at 1,000 x g at 20°C for 1 minute.
- Using a multi-channel pipette, add 40 µl of OHS1 to each sample in the HYP plate. Gently pipette up and down 3-5 times to mix. Change tips after each column to avoid cross-contamination.
- Seal the HYP plate with adhesive aluminum foil and centrifuge at 1,000 x g at 20°C for 1 minute.
- Place the HYP plate in the pre-heated block at 95°C and incubate for 1 minute.
- Set the temperature of the pre-heated block to 40°C and continue incubating for 80 minutes.

### Removal of Unbound Oligos

This process removes unbound oligos from genomic DNA using a filter capable of size selection.
- Remove ELM3 from -15° to -25°C storage and thaw at room temperature.
- Remove SW1 and UB1 from 2° to 8°C storage and set aside at room temperature.
- Assemble the filter plate assembly unit in the order from top to bottom: Lid, Filter Plate, Adapter Collar, and MIDI plate. Apply the FPU (Filter Plate Unit) barcode plate sticker.
- Pre-wash the FPU plate membrane as follows:
   1. Using a multi-channel pipette, add 45 µl of SW1 to each well.
   2. Cover the FPU plate with the filter plate lid and keep it covered during each centrifugation step.
   3. Centrifuge the FPU at 2,400 xg at 20°C for 2 minutes.
- After the 80-minute incubation, confirm the heat block has cooled to 40°C. While the HYP plate is still in the heat block, reinforce the seal using a rubber roller or sealing wedge.
- Remove the HYP plate from the heat block and centrifuge at 1,000 xg at 20°C for 1 minute to collect condensation.
- Using a multi-channel pipette set to 60 µl, transfer the entire volume of each sample onto the center of the corresponding pre-washed wells of the FPU plate. Change tips after each column to avoid cross-contamination.
- Cover the FPU plate with the filter plate lid and centrifuge the FPU at 2,400 xg at 20°C for 2 minutes.
- Wash the FPU plate as follows:
   1. Using a multi-channel pipette, add 45 µl of SW1 to each sample well.
   2. Cover the FPU plate with the filter plate lid and centrifuge the FPU at 2,400 xg for 2 minutes.
- Repeat the wash as described in the previous step.
- If the wash buffer does not drain completely, centrifuge again at 2,400 xg for 2 minutes. Discard all the flow-through (containing formamide) collected up to this point in an appropriate hazardous waste container, then reassemble the FPU. The same MIDI plate can be re-used for the rest of the pre-amplification process.
- Using a multi-channel pipette add 45 µl of UB1 to each sample well.
- Cover the FPU plate with the filter plate lid and centrifuge the FPU at 2,400 xg for 2 minutes.

### Extension-Ligation of Bound Oligos

This process connects the hybridized upstream and downstream oligos. A DNA polymerase extends from the upstream oligo through the targeted region, followed by ligation to the 5' end of the downstream oligo using a DNA ligase. This results in the formation of products containing your targeted regions of interest flanked by sequences required for amplification.
- Using a multi-channel pipette, add 45 µl of ELM3 to each sample well of the FPU plate.
- Seal the FPU plate with adhesive aluminum foil, and then cover with the lid to secure the foil during incubation.
- Incubate the entire FPU assembly in the pre-heated 37°C incubator for 45 minutes.
- While the FPU plate is incubating, prepare the IAP (Indexed Amplification Plate) as described in the following section

### PCR Amplification

In this step, your extension-ligation products are amplified using primers that add index sequences for sample multiplexing (i5 and i7) as well as common adapters required for cluster generation (P5 and P7).
- Prepare fresh 50 mM NaOH.
- Determine the index primers to be used in the assay using the Illumina Experiment Manager. Record index primer positions on the Lab Tracking Form.
- Remove PMM2 and the index primers (i5 and i7) from -15° to -25°C storage and thaw on a bench at room temperature. Vortex each tube to mix and briefly centrifuge the tubes in a microcentrifuge.
- Arrange i5 primer tubes (white caps, clear solution) vertically in a rack, aligned with rows A through H.
- Arrange i7 primer tubes (orange caps, yellow solution) horizontally in a rack, aligned with columns 1 through 12.
- Apply the IAP (Indexed Amplification Plate) barcode plate sticker to a new 96-well PCR plate.
- Using a multi-channel pipette, add 4 µl of i5 primers (clear solution) to each column of the IAP plate.
- To avoid index cross-contamination, discard the original *white* caps and apply new *white* caps provided in the TruSeq Custom Amplicon Index Kit.
- Using a multi-channel pipette, add 4µl of i7 primers (yellow solution) to each row of the IAP plate. Tips must be changed after each row to avoid index cross-contamination.
- To avoid index cross-contamination, discard the original *orange* caps and apply new *orange* caps provided in the TruSeq Custom Amplicon Index Kit.
- For 96 samples, add 56 µl of TDP1 to 2.8 ml of PMM2 (1 full tube). Invert the PMM2/TDP1 PCR master mix 20 times to mix well. You will add this mix to the IAP plate in the next section
-
- When the 45-minute extension-ligation reaction is complete, remove the FPU from the incubator. Remove the aluminum foil seal and replace with the filter plate lid.
- Centrifuge the FPU at 2,400 xg for 2 minutes.
- Using a multi-channel pipette, add 25 µl of 50 mM NaOH to each sample well on the FPU plate. Ensuring that pipette tips come in contact with the membrane, pipette the NaOH up and down 5-6 times. Tips must be changed after each column.
- Incubate the FPU plate at room temperature for 5 minutes.
- While the FPU plate is incubating, use a multi-channel pipette to transfer 22 µl of the PMM2/TDP1 PCR master mix to each well of the IAP plate containing index primers. Change tips between samples.
- Transfer samples eluted from the FPU plate to the IAP plate as follows:
   1. Set a multi-channel P20 pipette to 20 µl.
   2. Using fine tips, pipette the NaOH in the first column of the FPU plate up and down 5-6 times, then transfer 20 µl from the FPU plate to the corresponding column of the IAP plate. Gently pipette up and down 5-6 times to thoroughly combine the DNA with the PCR master mix. (Slightly tilt the FPU plate to ensure complete aspiration and to avoid air bubbles.)
   3. Transfer the remaining columns from the FPU plate to the IAP plate in a similar manner. Tips must be changed after each column to avoid index and sample crosscontamination.
   4. After all the samples have been transferred, the waste collection MIDI plate of the
- FPU can be discarded. The metal adapter collar should be put away for future use. If only a partial FPU plate is used, clearly mark which wells have been used, and store the FPU plate and lid in a sealed plastic bag to avoid contamination of the filter membrane.
- Cover the IAP plate with Microseal 'A' and seal with a rubber roller.
- Centrifuge at 1,000 xg at 20°C for 1 minute.
- Transfer the IAP plate to the post-amplification area.
- Perform PCR using the following program on a thermal cycler:
   - 95°C for 3 minutes
   - 27 cycles of:
      - 95°C for 30 seconds
      - 62°C for 30 seconds
      - 72°C for 60 seconds
   - 72°C for 5 minutes
   - Hold at 10°C

### PCR Clean-Up

- Bring the AMPure XP beads to room temperature.
- Prepare fresh 80% ethanol from absolute ethanol.
- Centrifuge the IAP plate at 1,000 xg for 1 min (20°C) to collect condensation.
- To confirm that the library has been successfully amplified, run an aliquot of the control and selected test samples on a a Bioanalyzer (1 µl). Expect the PCR product sizes to be around 350bp (Control ACP1) or 310bp (Cancer Panel AFP1).
- Apply the CLP (CLean-up Plate) barcode plate sticker to a new MIDI plate.
- Using a multi-channel pipette, add 45 µl of AMPure XP beads to each well of the CLP plate.
- Using a multi-channel pipette set to 60 µl, transfer the entire PCR product from the IAP plate to the CLP plate. Change tips between samples.
- Seal the CLP plate with Microseal 'B' and shake on a microplate shaker at 1,800 rpm for 2 minutes.
- Incubate at room temperature without shaking for 10 minutes.
- Place the plate on a magnetic stand for 2 minutes or until the supernatant has cleared.
- Using a multi-channel pipette set to 100 µl and with the CLP plate on the magnetic stand, carefully remove and discard the supernatant. Change tips between samples.
- With the CLP plate on the magnetic stand, wash the beads with freshly prepared 80% ethanol as follows:
   1. Using a multi-channel pipette, add 200 µl of freshly prepared 80% ethanol to each sample well. Changing tips is not required if you use care to avoid crosscontamination. You do not need to resuspend the beads at this time.
   2. Incubate the plate on the magnetic stand for 30 seconds or until the supernatant appears clear.
   3. Carefully remove and discard the supernatant.
- Repeat the 80% ethanol wash described in the previous step. Use a P20 multi-channel pipette to remove excess ethanol.
- Remove the CLP plate from the magnetic stand and allow the beads to air-dry for 10 minutes.
- Using a multi-channel pipette, add 30 µl of EBT to each well of the CLP plate. Seal the CLP plate with Microseal 'B' and shake on a microplate shaker at 1,800 rpm for 2 minutes. After shaking, if any samples are not resuspended, gently pipette up and down or lightly tap the plate on the bench to mix, then repeat this step.
- Incubate at room temperature without shaking for 2 minutes.
- Place the plate on the magnetic stand for 2 minutes or until the supernatant has cleared.
- Apply the LNP (Library Normalization Plate) barcode plate sticker to a new MIDI plate.
- Carefully transfer 20 µl of the supernatant from the CLP plate to the LNP plate. Change tips between samples.
- 19 Seal the LNP plate with Microseal 'B' and then centrifuge at 1,000 xg for 1 minute.

### Library Normalization

- Prepare fresh 0.1N NaOH.
- Remove LNA1 from -15° to -25°C storage and bring to room temperature. Use a 20° to 25°C water bath as needed. Once at room temperature, vortex vigorously and ensure that all precipitates have completely dissolved.
- Remove LNB1 and LNW1 from 2° to 8°C storage and bring to room temperature.
- Vigorously vortex LNB1 for at least 1 minute with intermittent inversion until the beads are well-resuspended and no pellet is found at the bottom of the tube when the tube is inverted.
- For 96 samples, add 4.4 ml of LNA1 to a fresh 15 ml conical tube.
- Use a P1000 pipette set to 1000 µl to resuspend LNB1 thoroughly by pipetting up and down 15-20 times, until the bead pellet at the bottom is completely resuspended.
- Immediately after LNB1 is thoroughly resuspended, use a P1000 pipette to transfer 800 µl of LNB1 to the 15 ml conical tube containing LNA1. Mix well by inverting the tube 15-20 times. The resulting LNA1/LNB1 bead mix is enough for 96 samples. Pour the bead mix into a trough and use it immediately in the next step.
- Using a multi-channel pipette, add 45 µl of the combined LNA1/LNB1 to each well of the LNP plate containing libraries.
- Seal the LNP plate with Microseal 'B' and shake on a microplate shaker at 1,800 rpm for 30 minutes.
- Place the plate on a magnetic stand for 2 minutes and confirm that the supernatant has cleared.
- With the LNP plate on the magnetic stand, using a multi-channel pipette set to 80 µl carefully remove and discard the supernatant in an appropriate hazardous waste container.
- Remove the LNP plate from the magnetic stand and wash the beads with LNW1 as follows:
   1. Using a multi-channel pipette, add 45 µl of LNW1 to each sample well.
   2. Seal the LNP plate with Microseal 'B'.
   3. Shake the LNP plate on a microplate shaker at 1,800 rpm for 5 minutes.
   4. Place the plate on the magnetic stand for 2 minutes or until the supernatant has cleared.
   5. Carefully remove and discard the supernatant in an appropriate hazardous waste container.
- Repeat the LNW1 wash described in the previous step.
- Remove the LNP plate from the magnetic stand and add 30 µl of 0.1 N NaOH (less than a week old) to each well to elute the sample.
- Seal the LNP plate with Microseal 'B' and shake on a microplate shaker at 1,800 rpm for 5 minutes.
- During the 5 minute elution, apply the SGP (StoraGe Plate) barcode plate sticker to a new 96-well PCR plate.
- Add 30 µl LNS1 to each well to be used in the SGP plate.
- After the 5 minute elution, ensure all samples in the LNP plate are completely resuspended. If the samples are not completely resuspended, gently pipette those samples up and down or lightly tap the plate on the bench to resuspend the beads, then shake for another 5 minutes.
- Place the LNP plate on the magnetic stand for 2 minutes or until the supernatant appears clear.
- Using a multi-channel pipette set to 30 µl, transfer the supernatant from the LNP plate to the SGP plate. Change tips between samples to avoid cross-contamination.
- Seal the SGP plate with Microseal 'B' and then centrifuge at 1,000 xg for 1 minute.

### Library Pooling and MiSeq Sample Loading

- Set a heat block suitable for 1.5 ml centrifuge tubes to 96°C.
- Remove a MiSeq reagent cartridge from -15 to -25°C storage and thaw at room temperature.
- In an ice bucket, prepare an ice-water bath by combining 3 parts ice and 1 part water.
- If the SGP plate was stored frozen, thaw the SGP plate at room temperature.
- Centrifuge the SGP plate at 1,000 xg for 1 minute at 20°C to collect condensation.
- Apply the PAL (Pooled Amplicon Library) barcode sticker to a fresh Eppendorf tube.
- Determine the samples to be pooled for sequencing. Calculate your supported sample multiplexing level based on the desired mean coverage using the following table.
- If the SGP plate was stored frozen, using a P200 multi-channel pipette, mix each library to be sequenced by pipetting up and down 3-5 times. Change tips between samples.
- Using a P20 multi-channel pipette, transfer 5 µl of each library to be sequenced from the SGP plate, column by column, to a PCR eight-tube strip. Change tips after each column to avoid sample cross-contamination. Seal SGP with Microseal 'B' and set aside.
- Combine and transfer the contents of the PCR eight-tube strip into the PAL tube. Mix PAL well.
- Apply the DAL (Diluted Amplicon Library) barcode sticker to a fresh Eppendorf tube.
- Add 594µl of HT1 to the DAL tube.
- Transfer 6 µl of PAL to the DAL tube containing HT1. Using the same tip, pipette up and down 3-5 times to rinse the tip and ensure complete transfer.
- Mix DAL by vortexing the tube at top speed. (If you would like to save the remaining PAL for future use, store the PAL tube at -15° to -25°C. The diluted library DAL should be freshly prepared and used immediately for MiSeq loading. Storing DAL may result in a significant reduction of cluster density.)
- Using a heat block, incubate the DAL tube at 96°C for 2 minutes.
- After the incubation, invert DAL 1-2 times to mix and immediately place in the ice-water bath.
- Keep DAL in the ice-water bath for 5 minutes.
- Load DAL into a thawed MiSeq reagent cartridge into the Load Samples reservoir.
- Sequence your library as indicated in the MiSeq System User Guide.

### Xenografts

Xenograft models provide sufficient tissue material for molecular studies of biomarkers that are predictive for response/nonresponse to therapy and can be used as companion diagnostics (CDx). Shortly after surgery, original colorectal cancer tumor pieces were shipped in gentamicin containing RPMI-1640 medium to the mouse facility. After arrival at the mouse facilities they were transplanted onto immunodeficient mice and were further passaged until a stably grown tumor xenografts has developed.

Surgical colorectal tumor samples were cut into pieces of 3 to 4 mm and transplanted within 30 min s.c. to 3 to 6 immunodeficient NOD/SCID mice (Taconic); the gender of the mice was chosen according to the donor patient. Additional tissue samples were immediately snap-frozen and stored at -80°C for genetic, genomic, and protein analyses. All animal experiments were done in accordance with the United Kingdom Co-ordinating Committee on Cancer Research regulations for the Welfare of Animals and of the German Animal Protection Law and approved by the local responsible authorities. Mice were observed daily for tumor growth. At a size of about 1 cm3, tumors were removed and passaged to naive NMRI: nu/nu mice (Charles River) for chemosensitivity testing. Tumors were passaged no more than 10 times. Numerous samples from early passages were stored in the tissue bank in liquid nitrogen and used for further experiments. Several rethawings led to successful engraftment in nude mice. All xenografts as well as the corresponding primary tumors were subjected to histological evaluation using snap-frozen, haematoxylin-eosin-stained tissue sections.

### Testing of colorectal cancer drugs

75 xenograft models were used in therapy experiments testing responsiveness towards drugs approved in the treatment of patients with colorectal cancer including cetuximab as an anti-EGRF antibody, bevacizumab, and oxaliplatin. Each of the 75 tumors was transplanted onto 20 mice (5 controls and 5 for each drug). Models with treated-to-control ratios of relative median tumor volumes of 20 % or lower were defined as responders.

The chemotherapeutic response of the passagable tumors was determined in male NMRI: *nu*/*nu* mice. For that purpose, one tumor fragment each was transplanted s.c. to a number of mice. At palpable tumor size (50-100 mm³), 6 to 8 mice each were randomized to treatment and control groups and treatment was initiated. If not otherwise mentioned, the following drugs and treatment modalities were used: Bevacizumab (Avastin®; Genentech Inc., South San Francisco, CA, USA) 50 mg/kg/d, qd 7x2, I.p., Cetuximab (Erbitux; Merck) 50 mg/kg/d, qd 7x2, i.p.; Oxaliplatin (Eloxatin, Sanofi-Avensis), 50 mg/kg/d, qd1-5, I.p. Doses and schedules were chosen according to previous experience in animal experiments and represent the maximum tolerated or efficient doses. The injection volume was 0.2 ml/20 g body weight.

Tumor size was measured in two dimensions twice weekly with a caliper-like instrument. Individual tumor volumes (*V*) were calculated by the formula: *V* = (length + [width]2) / 2 and related to the values at the first day of treatment (relative tumor volume). Median treated to control (T/C) values of relative tumor volume were used for the evaluation of each treatment modality and categorized according to scores (- to ++++;). The mean tumor doubling time of each xenograft model was calculated by comparing the size between 2- and 4-fold relative tumor volumes. Statistical analyses were done with the *U* test (Mann and Whitney) with *P* < 0.05. The body weight of mice was determined every 3 to 4 days and the change in body weight was taken as variable for tolerability.

**Table 1: Mutation Counts by Gene within Breast Cancer Tumor Samples**

| **Gene Symbol** | **Number of Mutations** | **Analyzed Samples** | **Frequency %** |
|---|---|---|---|
| TP53 | 2447 | 10721 | 22.8% |
| PIK3CA | 2068 | 8153 | 25.4% |
| CDH1 | 155 | 1161 | 13.4% |
| AKT1 | 97 | 2415 | 4.0% |
| PTEN | 76 | 1514 | 5.0% |
| CDKN2A | 36 | 1441 | 2.5% |
| GATA3 | 42 | 570 | 7.4% |
| KRAS | 27 | 1523 | 1.8% |
| APC | 26 | 1027 | 2.5% |
| BRCA1 | 28 | 1304 | 2.1% |
| RB1 | 27 | 697 | 3.9% |
| ATM | 19 | 832 | 2.3% |
| BRAF | 16 | 855 | 1.9% |
| EGFR | 16 | 1502 | 1.1% |
| NOTCH1 | 15 | 435 | 3.4% |
| ERBB2 | 14 | 828 | 1.7% |
| BRCA2 | 11 | 634 | 1.7% |
| NRAS | 9 | 674 | 1.3% |
| CTNNB1 | 7 | 679 | 1.0% |
| ALK | 6 | 315 | 1.9% |
| HRAS | 6 | 881 | 0.7% |
| SMAD4 | 6 | 327 | 1.8% |

| | | | |
|---|---|---|---|
| Legend Table 1: Each row presents mutations in breast cancer samples by genes found in the COSMIC (Catalogue Of Somatic Mutations In Cancer) database ordered by decreasing mutation count | | | |

**Table 2: Mutation Counts by Gene within Lung Cancer Tumor Samples**

| **Gene Symbol** | **Number of Mutations** | **Analyzed Samples** | **Frequency %** |
|---|---|---|---|
| EGFR | 11490 | 42070 | 27.3% |
| KRAS | 3228 | 20176 | 16.0% |
| TP53 | 1984 | 5640 | 35.2% |
| CDKN2A | 305 | 2421 | 12.6% |
| STK11 | 189 | 2205 | 8.6% |
| BRAF | 143 | 7271 | 2.0% |
| ERBB2 | 107 | 6068 | 1.8% |
| PIK3CA | 102 | 3862 | 2.6% |
| RB1 | 88 | 882 | 10.0% |
| PTEN | 65 | 1888 | 3.4% |
| MET | 47 | 1921 | 2.4% |
| NFE2L2 | 44 | 669 | 6.6% |
| CTNNB1 | 40 | 1404 | 2.8% |
| NRAS | 34 | 3732 | 0.9% |
| SMARCA4 | 27 | 308 | 8.8% |
| ATM | 23 | 434 | 5.3% |
| APC | 18 | 1294 | 1.4% |
| ERBB4 | 18 | 409 | 4.4% |
| KDR | 16 | 500 | 3.2% |
| NOTCH1 | 16 | 1135 | 1.4% |
| PDGFRA | 15 | 734 | 2.0% |
| ALK | 14 | 557 | 2.5% |
| FBXW7 | 14 | 663 | 2.1% |

| | | | |
|---|---|---|---|
| Legend Table 2: Each row presents mutations in lung cancer samples by genes found in the COSMIC (Catalogue Of Somatic Mutations In Cancer) database ordered by decreasing mutation count | | | |

**Table 3: Mutation Counts by Gene within Melanoma Samples**

| **Gene Symbol** | **Number of Mutations** | **Analyzed Samples** | **Frequency %** |
|---|---|---|---|
| BRAF | 5084 | 11291 | 45% |
| NRAS | 976 | 5414 | 18% |
| CDKN2A | 382 | 1413 | 27% |
| KIT | 218 | 2413 | 9% |
| PTEN | 107 | 690 | 16% |
| TP53 | 60 | 368 | 16% |
| GRIN2A | 36 | 145 | 25% |
| PREX2 | 34 | 144 | 24% |
| CTNNB1 | 34 | 745 | 5% |
| FGFR2 | 25 | 285 | 9% |
| KRAS | 22 | 1106 | 2% |
| ERBB4 | 22 | 97 | 23% |
| HRAS | 16 | 1000 | 2% |
| STK11 | 15 | 180 | 8% |

| | | | |
|---|---|---|---|
| Legend Table 3: Each row presents mutations in melanoma samples by genes found in the COSMIC (Catalogue Of Somatic Mutations In Cancer) database ordered by decreasing mutation count | | | |

**Table 4: Mutation Counts by Gene within Ovarian Cancer Tumor Samples**

| **Gene Symbol** | **Number of Mutations** | **Analyzed Samples** | **Frequency %** |
|---|---|---|---|
| TP53 | 1627 | 3687 | 44.1% |
| KRAS | 599 | 4830 | 12.4% |
| FOXL2 | 331 | 1842 | 18.0% |
| BRAF | 275 | 3578 | 7.7% |
| PIK3CA | 224 | 2574 | 8.7% |
| CTNNB1 | 106 | 1517 | 7.0% |
| ARID1A | 101 | 934 | 10.8% |
| CDKN2A | 80 | 1475 | 5.4% |
| PTEN | 65 | 1596 | 4.1% |
| BRCA1 | 36 | 1549 | 2.3% |
| EGFR | 33 | 1354 | 2.4% |
| PPP2R1A | 31 | 1065 | 2.9% |
| KIT | 23 | 979 | 2.3% |
| BRCA2 | 22 | 1302 | 1.7% |
| ERBB2 | 17 | 604 | 2.8% |
| GNAS | 16 | 741 | 2.2% |

| | | | |
|---|---|---|---|
| Legend Table 4: Each row presents mutations in ovarian cancer samples by genes found in the COSMIC (Catalogue Of Somatic Mutations In Cancer) database ordered by decreasing mutation count | | | |

**Table 5: Mutation Counts by Gene within Pancreatic Cancer Tumor Samples**

| **Gene Symbol** | **Number of Mutations** | **Analyzed Samples** | **Frequency %** |
|---|---|---|---|
| **KRAS** | 3414 | 5945 | 57.4% |
| **TP53** | 380 | 950 | 40.0% |
| **CDKN2A** | 192 | 768 | 25.0% |
| **SMAD4** | 164 | 750 | 21.9% |
| **CTNNB1** | 125 | 476 | 26.3% |
| **MEN1** | 62 | 244 | 25.4% |
| **GNAS** | 56 | 292 | 19.2% |
| **APC** | 26 | 184 | 14.1% |
| **VHL** | 18 | 186 | 9.7% |
| **PIK3CA** | 17 | 521 | 3.3% |
| **BRAF** | 15 | 728 | 2.1% |
| **PTEN** | 6 | 259 | 2.3% |
| **STK11** | 6 | 240 | 2.5% |
| **NRAS** | 5 | 316 | 1.6% |
| **RB1** | 5 | 74 | 6.8% |

| | | | |
|---|---|---|---|
| Legend Table 5: Each row presents mutations in pancreatic cancer samples by genes found in the COSMIC (Catalogue Of Somatic Mutations In Cancer) database ordered by decreasing mutation count | | | |

**Table 6: Mutation Counts by Gene within Prostate Cancer Tumor Samples**

| **Gene Symbol** | **Number of Mutations** | **Analyzed Samples** | **Frequency %** |
|---|---|---|---|
| TP53 | 214 | 969 | 22.1% |
| PTEN | 104 | 670 | 15.5% |
| KRAS | 83 | 1106 | 7.5% |
| EGFR | 31 | 440 | 7.0% |
| HRAS | 31 | 560 | 5.5% |
| SPOP | 29 | 118 | 24.6% |
| CTNNB1 | 28 | 415 | 6.7% |
| BRAF | 24 | 1082 | 2.2% |
| APC | 15 | 166 | 9.0% |
| RB1 | 11 | 135 | 8.1% |
| FGFR3 | 9 | 344 | 2.6% |
| ATM | 8 | 67 | 11.9% |
| CDKN2A | 8 | 324 | 2.5% |
| NRAS | 8 | 588 | 1.4% |
| PIK3CA | 8 | 353 | 2.3% |

| | | | |
|---|---|---|---|
| Legend Table 6: Each row presents mutations in prostate cancer samples by genes found in the COSMIC (Catalogue Of Somatic Mutations In Cancer) database ordered by decreasing mutation count | | | |

**Table 7: Mutation Counts by Gene within Stomach Cancer Tumor Samples**

| **Gene Symbol** | **Number of Mutations** | **Analyzed Samples** | **Frequency %** |
|---|---|---|---|
| TP53 | 1115 | 3505 | 31.8% |
| KRAS | 197 | 3059 | 6.4% |
| CTNNB1 | 157 | 1891 | 8.3% |
| APC | 130 | 927 | 14.0% |
| PIK3CA | 116 | 1174 | 9.9% |
| CDH1 | 68 | 348 | 19.5% |
| CDKN2A | 44 | 839 | 5.2% |
| EGFR | 36 | 855 | 4.2% |
| PTEN | 30 | 781 | 3.8% |
| MSH6 | 21 | 275 | 7.6% |
| FBXW7 | 16 | 249 | 6.4% |
| PDGFRA | 15 | 340 | 4.4% |
| HRAS | 14 | 621 | 2.3% |
| ERBB2 | 13 | 700 | 1.9% |
| BRAF | 11 | 1367 | 0.8% |
| STK11 | 9 | 435 | 2.1% |
| ACVR2A | 8 | 74 | 10.8% |
| NRAS | 5 | 453 | 1.1% |

| | | | |
|---|---|---|---|
| Legend Table 7: Each row presents mutations in stomach cancer samples by genes found in the COSMIC (Catalogue Of Somatic Mutations In Cancer) database ordered by decreasing mutation count | | | |

**Table 8: Mutation Counts by Gene within Colorectal Cancer Tumor Samples**

| **Gene Symbol** | **Mutations Number** | **Analyzed Samples** | **Frequency %** |
|---|---|---|---|
| KRAS | 14422 | 41383 | 34.9% |
| BRAF | 6608 | 53752 | 12.3% |
| TP53 | 4907 | 11341 | 43.3% |
| APC | 2332 | 5808 | 40.2% |
| PIK3CA | 1120 | 8589 | 13.0% |
| CTNNB1 | 247 | 4594 | 5.4% |
| FBXW7 | 139 | 1089 | 12.8% |
| SMAD4 | 131 | 981 | 13.4% |
| NRAS | 97 | 2229 | 4.4% |
| EGFR | 77 | 1803 | 4.3% |
| PTEN | 75 | 1145 | 6.6% |
| MSH6 | 64 | 290 | 22.1% |
| MLL3 | 43 | 350 | 12.3% |
| MLH1 | 42 | 405 | 10.4% |
| ARID1A | 36 | 155 | 23.2% |
| ATM | 36 | 198 | 18.2% |
| MSH2 | 36 | 416 | 8.7% |
| GNAS | 34 | 568 | 6.0% |
| FAM123B | 32 | 164 | 19.5% |
| NF1 | 29 | 180 | 16.1% |
| EP300 | 26 | 131 | 19.8% |
| MAP2K4 | 26 | 439 | 5.9% |
| PIK3R1 | 25 | 361 | 6.9% |
| TRRAP | 25 | 152 | 16.4% |
| ALK | 21 | 211 | 10.0% |
| MTOR | 20 | 151 | 13.2% |
| AXIN1 | 19 | 208 | 9.1% |
| HNF1A | 19 | 131 | 14.5% |
| NTRK3 | 19 | 314 | 6.1% |
| PTCH1 | 18 | 147 | 12.2% |
| ROS1 | 17 | 149 | 11.4% |
| BRCA2 | 16 | 130 | 12.3% |
| KDR | 15 | 118 | 12.7% |
| KIT | 15 | 369 | 4.1% |
| SRC | 15 | 1109 | 1.4% |
| TRIO | 15 | 146 | 10.3% |
| ERBB2 | 14 | 365 | 3.8% |
| PDGFRA | 14 | 254 | 5.5% |
| RET | 14 | 254 | 5.5% |
| SMARCA4 | 14 | 115 | 12.2% |
| STK11 | 14 | 487 | 2.9% |
| ROR1 | 13 | 169 | 7.7% |
| TGFBR2 | 13 | 167 | 7.8% |
| LRRK1 | 12 | 144 | 8.3% |
| CDKN2A | 11 | 327 | 3.4% |
| DCLK3 | 11 | 131 | 8.4% |
| ROR2 | 11 | 142 | 7.7% |
| VHL | 11 | 288 | 3.8% |
| CDK12 | 10 | 142 | 7.0% |
| JAK3 | 10 | 139 | 7.2% |
| PTK7 | 10 | 142 | 7.0% |
| CDH1 | 9 | 136 | 6.6% |
| SMO | 9 | 107 | 8.4% |
| CYLD | 8 | 141 | 5.7% |
| IDH2 | 8 | 162 | 4.9% |
| JAK1 | 8 | 288 | 2.8% |
| NEK11 | 8 | 137 | 5.8% |
| NF2 | 8 | 335 | 2.4% |
| ABL1 | 7 | 189 | 3.7% |
| AKT1 | 7 | 917 | 0.8% |
| ARAF | 7 | 161 | 4.3% |
| CHUK | 7 | 139 | 5.0% |
| IDH1 | 7 | 482 | 1.5% |
| MET | 7 | 310 | 2.3% |
| PAK3 | 7 | 139 | 5.0% |
| RB1 | 7 | 133 | 5.3% |
| SgK495 | 7 | 126 | 5.6% |
| BRCA1 | 6 | 123 | 4.9% |
| FLT3 | 6 | 225 | 2.7% |
| JAK2 | 6 | 505 | 1.2% |
| PRKCH | 6 | 138 | 4.3% |
| PTPN11 | 6 | 294 | 2.0% |
| RIPK1 | 6 | 136 | 4.4% |
| BMPR1A | 5 | 137 | 3.6% |
| FGFR1 | 5 | 257 | 1.9% |
| FGFR3 | 5 | 280 | 1.8% |
| AURKA | 4 | 136 | 2.9% |
| PIM1 | 4 | 136 | 2.9% |
| FGFR2 | 3 | 111 | 2.7% |
| GNAQ | 3 | 234 | 1.3% |
| CAMKK2 | 2 | 134 | 1.5% |
| CAMKV | 2 | 133 | 1.5% |
| DAPK3 | 2 | 134 | 1.5% |
| EEF2K | 2 | 134 | 1.5% |
| EML4 | 2 | 169 | 1.2% |
| GNA11 | 2 | 134 | 1.5% |
| HRAS | 2 | 756 | 0.3% |
| NFE2L2 | 2 | 108 | 1.9% |
| FOXL2 | 1 | 328 | 0.3% |
| NOTCH1 | 1 | 161 | 0.6% |
| NPM1 | 1 | 193 | 0.5% |
| PHKG1 | 1 | 133 | 0.8% |
| VTI1A | 1 | 110 | 0.9% |

| | | | |
|---|---|---|---|
| Legend Table 8: Each row presents mutations in colorectal cancer samples by genes found in the COSMIC (Catalogue Of Somatic Mutations In Cancer) database ordered by decreasing mutation count | | | |

**Table 9: Performance of Presence of Missense Sequence Variations (Detected on 1 gene) On Prediction of Metastasis vs. No progression of Disease Event in Colorectal Cancer UICC Stage II**

| **By Mutation Number** | | **Sequence Variation Count** | **Sensitivity** | **Specificity** | **AROC** | **Combined Jaccard Ratio** | **Ranked By Decreasing AROC** | **Ranked By Decreasing CJR** |
|---|---|---|---|---|---|---|---|---|
| | **Prediction Function** | | | | | | | |
| 1. | !TP53 | 68 | 0.675 | 0.585 | 0.630 | 0.428 | 1. | 1. |
| | TP53 | | 0.325 | 0.415 | 0.370 | 0.230 | | |
| 2. | KRAS | 47 | 0.425 | 0.681 | 0.553 | 0.395 | 2. | 2. |
| | !KRAS | | 0.575 | 0.319 | 0.447 | 0.246 | | |
| 3. | KDR | 45 | 0.300 | 0.649 | 0.474 | 0.332 | | 17. |
| | !KDR | | 0.700 | 0.351 | 0.526 | 0.294 | 5. | |
| 4. | KIT | 26 | 0.223 | 0.819 | 0.522 | 0.387 | 7. | 4. |
| | !KIT | | 0.775 | 0.181 | 0.488 | 0.215 | | |
| 5. | PIK3CA | 25 | 0.225 | 0.830 | 0.527 | 0.392 | 4. | 3. |
| | !PIK3CA | | 0.775 | 0.170 | 0.473 | 0.209 | | |
| 6. | BRAF | 13 | 0.125 | 0.915 | 0.520 | 0.385 | 8. | 5. |
| | !BRAF | | 0.875 | 0.085 | 0.480 | 0.179 | | |
| 7. | FLT3 | 13 | 0.075 | 0.894 | 0.484 | 0.351 | | 12. |
| | !FLT3 | | 0.925 | 0.106 | 0.516 | 0.201 | 9. | |
| 8. | MET | 11 | 0.100 | 0.926 | 0.513 | 0.377 | 11. | 7. |
| | !MET | | 0.900 | 0.074 | 0.487 | 0.177 | | |
| 9. | FBXW7 | 11 | 0.100 | 0.926 | 0.513 | 0.377 | 12. | 8. |
| | !FBXW7 | | 0.900 | 0.074 | 0.487 | 0.177 | | |
| 10. | ATM | 8 | 0.075 | 0.947 | 0.511 | 0.373 | 13. | 9. |
| | !ATM | | 0.925 | 0.053 | 0.489 | 0.169 | | |
| 11. | APC | 6 | 0.000 | 0.934 | 0.468 | 0.328 | | 18. |
| | !APC | | 1.000 | 0.064 | 0.532 | 0.188 | 3. | |
| 12. | SMAD4 | 5 | 0.050 | 0.968 | 0.509 | 0.368 | 17. | 10. |
| | !SMAD4 | | 0.950 | 0.032 | 0.491 | 0.161 | | |
| 13. | PTEN | 3 | 0.000 | 0.964 | 0.484 | 0.340 | | 16. |
| | !PTEN | | 1.000 | 0.032 | 0.516 | 0.169 | 10. | |
| 14. | AKT1 | 2 | 0.000 | 0.973 | 0.489 | 0.343 | | 13. |
| | !AKT1 | | 1.000 | 0.021 | 0.510 | 0.162 | 15. | |
| 15. | RET | | 0.000 | 0.979 | 0.489 | 0.343 | | 14. |
| | !RET | | 1.000 | 0.021 | 0.510 | 0.016 | 16. | |
| 16. | SMO | | 0.050 | 1.000 | 0.525 | 0.381 | 6. | 6. |
| | !SMO | | 0.950 | 0.000 | 0.475 | 0.142 | | |
| 17. | ERBB4 | | 0.025 | 0.989 | 0.507 | 0.362 | 18. | 11. |
| | !ERBB4 | | 0.975 | 0.110 | 0.493 | 0.152 | | |
| 18. | GNAS | | 0.000 | 0.979 | 0.489 | 0.343 | | 15. |

| | | | | | | | | |
|---|---|---|---|---|---|---|---|---|
| Legend Table 9: AROC = Area under the receiver operating characteristic curve; CJR = combined Jaccard Ratio. | | | | | | | | |

**Table 10: Prediction of Metastasis vs. No Progression of Disease Event in Colorectal Cancer UICC Stage II based on Missense Sequence Variations**

| | Prediction Function | **S+** | **S-** | **PPV** | **NPV** | **AROC** | **CJR** | **TP** | **FP** | **TN** | **FP** |
|---|---|---|---|---|---|---|---|---|---|---|---|
| **1** | !TP53 | 0.675 | 0.585 | 0.409 | 0.809 | 0.630 | 0.428 | 27 | 13 | 55 | 39 |
| | | | | | | | | | | | |
| **2** | !TP53 XOR BRAF | 0.700 | 0.606 | 0.431 | 0.826 | 0.653 | 0.451 | 28 | 12 | 57 | 37 |
| | BRAF XOR !TP53 | 0.700 | 0.606 | 0.431 | 0.826 | 0.653 | 0.451 | 28 | 12 | 57 | 37 |
| | TP53 XOR !BRAF | 0.700 | 0.606 | 0.431 | 0.826 | 0.653 | 0.451 | 28 | 12 | 57 | 37 |
| | !BRAF XOR TP53 | 0.700 | 0.606 | 0.431 | 0.826 | 0.653 | 0.451 | 28 | 12 | 57 | 37 |
| | | | | | | | | | | | |
| **3** | !TP53 XOR BRAF OR SMO | 0.750 | 0.606 | 0.431 | 0.826 | 0.653 | 0.451 | 30 | 10 | 57 | 37 |
| | !TP53 OR SMO XOR BRAF | 0.725 | 0.606 | 0.439 | 0.838 | 0.666 | 0.46 | 29 | 11 | 57 | 37 |
| | BRAF XOR !TP53 OR SMO | 0.750 | 0.606 | 0.431 | 0.826 | 0.653 | 0.451 | 30 | 10 | 57 | 37 |
| | BRAF OR SMO XOR !TP53 | 0.725 | 0.606 | 0.439 | 0.838 | 0.666 | 0.46 | 29 | 11 | 57 | 37 |
| | SMO XOR !TP53 XOR BRAF | 0.750 | 0.606 | 0.431 | 0.826 | 0.653 | 0.451 | 30 | 10 | 57 | 37 |
| | SMO XOR BRAF XOR !TP53 | 0.750 | 0.606 | 0.431 | 0.826 | 0.653 | 0.451 | 30 | 10 | 57 | 37 |
| | | | | | | | | | | | |
| **4** | !TP53 XOR BRAF OR SMO AND !APC | 0.750 | 0.628 | 0.462 | 0.855 | 0.689 | 0.484 | 30 | 10 | 59 | 35 |
| | !TP53 XOR BRAF AND !APC OR SMO | 0.750 | 0.628 | 0.462 | 0.855 | 0.689 | 0.484 | 30 | 10 | 59 | 35 |
| | !TP53 OR SMO XOR BRAF AND !APC | 0.725 | 0.628 | 0.453 | 0.843 | 0.676 | 0.474 | 29 | 11 | 59 | 35 |
| | !TP53 AND !APC XOR BRAF OR SMO | 0.750 | 0.628 | 0.462 | 0.855 | 0.689 | 0.484 | 30 | 10 | 59 | 35 |
| | !TP53 AND !APC OR SMO XOR BRAF | 0.725 | 0.628 | 0.453 | 0.843 | 0.676 | 0.474 | 29 | 11 | 59 | 35 |
| | !TP53 OR SMO AND !APC XOR BRAF | 0.725 | 0.628 | 0.453 | 0.843 | 0.676 | 0.474 | 29 | 11 | 59 | 35 |
| | | | | | | | | | | | |
| | BRAF XOR !TP53 OR SMO AND !APC | 0.750 | 0.628 | 0.462 | 0.855 | 0.689 | 0.484 | 30 | 10 | 59 | 35 |
| | BRAF XOR !TP53 AND !APC OR SMO | 0.750 | 0.628 | 0.462 | 0.855 | 0.689 | 0.484 | 30 | 10 | 59 | 35 |
| | BRAF OR SMO XOR !TP53 AND !APC | 0.725 | 0.628 | 0.453 | 0.843 | 0.676 | 0.474 | 29 | 11 | 59 | 35 |
| | BRAF AND !APC XOR !TP53 OR SMO | 0.750 | 0.606 | 0.448 | 0.851 | 0.678 | 0.469 | 30 | 10 | 57 | 37 |
| | BRAF OR SMO AND !APC XOR !TP53 | 0.725 | 0.606 | 0.439 | 0.838 | 0.666 | 0.46 | 29 | 11 | 59 | 35 |
| | BRAF AND !APC OR SMO XOR !TP53 | 0.725 | 0.606 | 0.439 | 0.838 | 0.666 | 0.46 | 29 | 11 | 59 | 35 |
| | | | | | | | | | | | |
| | SMO XOR !TP53 XOR BRAF AND !APC | 0.750 | 0.628 | 0.462 | 0.855 | 0.689 | 0.484 | 30 | 10 | 59 | 35 |
| | SMO XOR !TP53 AND !APC XOR BRAF | 0.750 | 0.628 | 0.462 | 0.855 | 0.689 | 0.484 | 30 | 10 | 59 | 35 |
| | SMO XOR BRAF XOR !TP53 AND !APC | 0.750 | 0.628 | 0.462 | 0.855 | 0.689 | 0.484 | 30 | 10 | 59 | 35 |
| | SMO AND !APC XOR !TP53 XOR BRAF | 0.750 | 0.606 | 0.448 | 0.851 | 0.678 | 0.469 | 30 | 10 | 57 | 37 |
| | SMO XOR BRAF AND !APC XOR !TP53 | 0.750 | 0.606 | 0.448 | 0.851 | 0.678 | 0.469 | 30 | 10 | 57 | 37 |
| | SMO AND !APC XOR BRAF XOR !TP53 | 0.750 | 0.606 | 0.448 | 0.851 | 0.678 | 0.469 | 30 | 10 | 57 | 37 |
| | | | | | | | | | | | |
| | !APC XOR !TP53 XOR BRAF OR SMO | 0.750 | 0.585 | 0.435 | 0.846 | 0.668 | 0.454 | 30 | 10 | 55 | 39 |
| | !APC XOR !TP53 OR SMO XOR BRAF | 0.725 | 0.585 | 0.426 | 0.833 | 0.655 | 0.445 | 29 | 11 | 55 | 39 |
| | !APC XOR BRAF XOR !TP53 OR SMO | 0.750 | 0.585 | 0.435 | 0.846 | 0.668 | 0.454 | 30 | 10 | 55 | 39 |
| | !APC OR SMO XOR !TP53 XOR BRAF | 0.750 | 0.585 | 0.435 | 0.846 | 0.668 | 0.454 | 30 | 10 | 55 | 39 |
| | !APC XOR BRAF OR SMO XOR !TP53 | 0.725 | 0.585 | 0.426 | 0.833 | 0.655 | 0.445 | 29 | 11 | 55 | 39 |
| | !APC OR SMO XOR BRAF XOR !TP53 | 0.750 | 0.585 | 0.435 | 0.846 | 0.668 | 0.454 | 30 | 10 | 55 | 39 |
| | | | | | | | | | | | |
| **6** | !TP53 XOR BRAF OR SMO AND !APC AND !PTEN AND !RET | 0.750 | 0.666 | 0.484 | 0.861 | 0.705 | 0.506 | 30 | 10 | 62 | 32 |
| | BRAF XOR !TP53 OR SMO AND !APC AND !PTEN AND !RET | 0.750 | 0.666 | 0.484 | 0.861 | 0.705 | 0.506 | 30 | 10 | 62 | 32 |
| | BRAF OR SMO XOR !TP53 AND !APC AND !PTEN AND !RET | 0.725 | 0.660 | 0.475 | 0.849 | 0.692 | 0.497 | 29 | 11 | 62 | 32 |
| | BRAF OR SMO AND !APC XOR !TP53 AND !PTEN AND !RET | 0.725 | 0.638 | 0.460 | 0.845 | 0.682 | 0.482 | 29 | 11 | 60 | 34 |
| | BRAF OR SMO AND !APC AND !PTEN XOR !TP53 AND !RET | 0.725 | 0.617 | 0.446 | 0.841 | 0.671 | 0.467 | 29 | 11 | 58 | 36 |
| | BRAF OR SMO AND !APC AND !PTEN AND !RET XOR !TP53 | 0.725 | 0.606 | 0.439 | 0.838 | 0.666 | 0.460 | 29 | 11 | 57 | 37 |
| | !TP53 XOR BRAF OR SMO AND !APC AND !PTEN AND !RET | 0.75 | 0.666 | 0.484 | 0.861 | 0.705 | 0.506 | 30 | 10 | 62 | 32 |
| | !TP53 OR SMO XOR BRAF AND !APC AND !PTEN AND !RET | 0.725 | 0.666 | 0.475 | 0.85 | 0.692 | 0.497 | 29 | 11 | 62 | 32 |
| | !TP53 OR SMO AND !APC XOR BRAF AND !PTEN AND !RET | 0.725 | 0.666 | 0.475 | 0.85 | 0.692 | 0.497 | 29 | 11 | 62 | 32 |
| | !TP53 OR SMO AND !APC AND !PTEN XOR BRAF AND !RET | 0.725 | 0.638 | 0.46 | 0.845 | 0.682 | 0.482 | 29 | 11 | 60 | 34 |
| | !TP53 XOR BRAF OR SMO AND !APC AND !PTEN AND !RET | 0.75 | 0.666 | 0.484 | 0.861 | 0.705 | 0.506 | 30 | 10 | 62 | 32 |
| | SMO XOR !TP53 XOR BRAF AND !APC AND !PTEN AND !RET | 0.75 | 0.666 | 0.484 | 0.861 | 0.705 | 0.506 | 30 | 10 | 62 | 32 |
| | SMO AND !APC XOR !TP53 XOR BRAF AND !PTEN AND !RET | 0.75 | 0.638 | 0.469 | 0.857 | 0.694 | 0.491 | 30 | 10 | 60 | 34 |
| | SMO AND !APC AND !PTEN XOR !TP53 XOR BRAF AND !RET | 0.75 | 0.617 | 0.455 | 0.853 | 0.684 | 0.476 | 30 | 10 | 58 | 36 |
| | SMO AND !APC AND !PTEN AND !RET XOR !TP53 XOR BRAF | 0.75 | 0.606 | 0.448 | 0.851 | 0.678 | 0.469 | 30 | 10 | 57 | 37 |

| | | | | | | | | | | | |
|---|---|---|---|---|---|---|---|---|---|---|---|
| Legend Table 10: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | | |

**Table 11: Further Preferred Functions Predicting of Metastasis vs. No Progression of Disease Event in Colorectal Cancer UICC Stage II based on Missense Sequence Variations**

| | | | |
|---|---|---|---|
| a. | Adding KRAS | Best function of 3 | KRAS OR !TP53 XOR BRAF |
| b. | Adding KDR | Best function of 3 | KDR OR !TP53 OR BRAF |
| | | Best function of 6 | KDR OR !TP53 OR BRAF AND !APC AND !PTEN OR SMO |
| c. | Adding PIK3CA | Best function of 7 | PIK3CA OR !TP53 XOR BRAF OR SMO AND !APC AND !PTEN AND !RET |
| d. | Adding MET | Best function of 7 | MET OR !TP53 XOR BRAF OR SMO AND !APC AND !PTEN AND !RET |
| e. | Adding KIT | Best function of 8 | !KIT And !TP53 XOR BRAF OR SMO OR MET AND !APC AND !AKTAND !RET |
| f. | FLT3 | best function of 8 | FLT3 OR !TP53 AND !PTEN XOR SMAD4 OR SMO AND !APC AND !RET AND !AKT1 |

**Table 12: Preferred Functions Predicting of Metastasis vs. No Progression of Disease Event in Colorectal Cancer UICC Stage II based on Missense and Nonsense Sequence Variations**

| Operands | Prediction Function | **S+** | **S-** | **PPV** | **NPV** | **AROC** | **CJR** | **TP** | **FP** | **TN** | **FP** |
|---|---|---|---|---|---|---|---|---|---|---|---|
| 1 | !TP53 | 0.600 | 0.628 | 0.407 | 0.787 | 0.63 | 0.428 | | | | |
| 2 | !TP53 XOR BRAF | 0.725 | 0.649 | 0.468 | 0.847 | 0.687 | 0.489 | | | | |
| 3 | !TP53 XOR BRAF OR SMO | 0.750 | 0.649 | 0.478 | 0.859 | 0.699 | 0.499 | | | | |
| 4 | !TP53 XOR BRAF OR SMO AND !APC | 0.750 | 0.670 | 0.492 | 0.863 | 0.71 | 0.514 | 30 | 10 | 63 | 31 |
| 5 | !TP53 XOR BRAF OR SMO AND !PTEN AND !RET | 0.750 | 0.681 | 0.5 | 0.865 | 0.715 | 0.522 | 30 | 10 | 64 | 30 |

| | | | | | | | | | | | |
|---|---|---|---|---|---|---|---|---|---|---|---|
| Legend Table 12: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | | |

**Table 13: Further Preferred Functions Predicting of Metastasis vs. No Progression of Disease Event in Colorectal Cancer UICC Stage II based on Missense and Nonsense Sequence Variations**

| | | Prediction Function | **S+** | **S-** | **PPV** | **NPV** | **AROC** | **CJR** | **TP** | **FP** | **TN** | **FP** |
|---|---|---|---|---|---|---|---|---|---|---|---|---|
| Adding KRAS | 6 | KRAS OR Rectum AND !TP53 XOR BRAF And !PTEN OR SMO | 0.55 | 0.83 | 0.579 | 0.813 | 0.69 | 0.545 | 22 | 18 | 78 | 16 |
| Adding !KRAS | 6 | !KRAS XOR Rectum AND FLT3 OR BRAF OT !TP53 AND !PTEN | 0.875 | 0.468 | 0.412 | 0.898 | 0.672 | 0.417 | 35 | 5 | 44 | 50 |
| Adding KDR | 5 | KDR XOR KRAS XOR BRAF AND !TP53 OR SMO | 0.45 | 0.872 | 0.6 | 0.788 | 0.661 | 0.527 | 18 | 22 | 82 | 12 |
| | 6 | KDR XOR KRAS XOR BRAF AND !TP53 OR SMO AND !PTEN | 0.45 | 0.883 | 0.621 | 0.79 | 0.666 | 0.534 | 18 | 22 | 83 | 11 |
| Adding PIK3CA | 6 | PIK3CA OR !TP53 XOR BRAF OR SMO AND !PTEN AND !RET | 0.8 | 0.596 | 0.457 | 0.875 | 0.698 | 0.48 | 32 | 8 | 56 | 38 |
| Adding !KIT | 7 | !KIT AND !TP53 XOR BRAF OR SMAD4 OR SMO AND !AKT1 AND !RET | 0.65 | 0.713 | 0.491 | 0.827 | 0.681 | 0.504 | 26 | 14 | 67 | 27 |
| Adding FLT3 | 6 | FLT3 Or !TP53 XOR BRAF OR SMO AND !RET AND !PTEN | 0.725 | 0.628 | 0.453 | 0.843 | 0.676 | 0.474 | 29 | 11 | 59 | 35 |
| Legend Table 13: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | | | |

**Table 14: Further Preferred Functions Predicting of Metastasis vs. No Progression of Disease Event in Colorectal Cancer UICC Stage II based on Missense, Nonsense, Silent and Synonymous Sequence Variations**

| Optimization | Operands | | **S+** | **S-** | **PPV** | **NPV** | **AROC** | **CJR** | **TP** | **FP** | **TN** | **FP** |
|---|---|---|---|---|---|---|---|---|---|---|---|---|
| | 1 | !RET | 0.825 | 0.33 | 0.344 | 0.816 | 0.577 | 0.314 | | | | |
| | 2 | !RET XOR KIT | 0.725 | 0.532 | 0.397 | 0.82 | 0.628 | 0.41 | | | | |
| | 3 | !RET XOR KIT OR Rectum | 0.8 | 0.521 | 0.416 | 0.86 | 0.66 | 0.428 | 32 | 8 | 49 | 45 |
| AROC | 1 | !RET | | | | | | | | | | |
| | 2 | !RET AND !KIT | 0.625 | 0.596 | 0.397 | 0.789 | 0.61 | 0.417 | | | | |
| | 3 | !RET AND !KIT XOR FLT3 | 0.675 | 0.638 | 0.44 | 0.822 | 0.654 | 0.463 | 27 | 13 | 60 | 34 |
| | 6 | !RET AND !KIT XOR FLT3 OR GNA11 AND !AKT1 AND CSF1R | 0.7 | 0.681 | 0.48 | 0.84 | 0.69 | 0.502 | 28 | 12 | 64 | 30 |
| | 7 | !RET AND !KIT XOR FLT3 OR GNA11 AND !AKT1 AND CSF1R AND ABL1 | | | | | | | 28 | 12 | 65 | 29 |
| CJR | 6 | !RET AND !TP53 AND !EGFR XOR BRAF AND !AKT1 OR GNA11 | 0.5 | 0.894 | 0.667 | 0.808 | 0.697 | 0.568 | 20 | 20 | 84 | 10 |

| | | | | | | | | | | | | |
|---|---|---|---|---|---|---|---|---|---|---|---|---|
| Legend Table 14: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | | | |

**Table 15: Further Preferred Functions Predicting of Metastasis vs. No Progression of Disease Event in Colorectal Cancer UICC Stage II based on Missense, Nonsense, Silent and Synonymous Sequence Variations**

| Action | Operands | Prediction Function | **S+** | **S-** | **PPV** | **NPV** | **AROC** | **CJR** | **TP** | **FP** | **TN** | **FP** | Comment |
|---|---|---|---|---|---|---|---|---|---|---|---|---|---|
| Adding PTEN | 6 | PTEN OR RET XOR KIT XOR FLT3 AND !CSF1R AND !ABL1 | 0.75 | 0.628 | 0.462 | 0.855 | 0.689 | 0.48 | 30 | 10 | 59 | 35 | |
| | 7 | PTEN OR RET XOR KIT XOR FLT3 AND !CSF1R AND !ABL1 AND !AKT1 | 0.75 | 0.638 | 0.468 | 0.857 | 0.694 | 0.491 | 30 | 10 | 60 | 34 | |
| Adding SMAD4 | 6 | SMAD4 AND !TP53 OR IDH1 OR pT4 OR GNA11 XOR ATM | 0.52 | 0.798 | 0.525 | 0.798 | 0.66 | 0.51 | 21 | 19 | 75 | 19 | |
| Adding EGFR | 6 | EGFR XOR !TP53 XOR Therapy AND !RET OR GNA11 AND !V+ | 0.675 | 0.755 | 0.54 | 0.845 | 0.71 | 0.546 | 27 | 13 | 71 | 23 | |
| Adding HNF1A | 7 | HNF1A OR !RET AND !TP53 XOR BRAF XOR SMARCB1 AND !AKT1 And !FGFR3 | 0.625 | 0.723 | 0.49 | 0.819 | 0.674 | 0.501 | 25 | 15 | 68 | 26 | |
| Adding KIT | 6 | KIT XOR !RET OR Rectum XOR FGFR3 XOR MET XOR GNAQ | 0.875 | 0.564 | 0.46 | 0.914 | 0.719 | 0.481 | 35 | 5 | 53 | 41 | |
| !KIT | 6 | !KIT AND !RET XOR FLT3 OR GNA11 AND !AKT1 AND CSF1R | 0.7 | 0.681 | 0.48 | 0.84 | 0.69 | 0.502 | 28 | 12 | 64 | 30 | |
| PDGFRA | | bad performance | | | | | | | | | | | |
| PIK3CA | | bad performance | | | | | | | | | | | |
| Adding SMO | 5 | SMO XOR !APC OR !TP53 XOR BRAF AND !RET | 0.75 | 0.666 | 0.484 | 0.861 | 0.705 | 0.506 | 30 | 10 | 62 | 32 | equal to best signature with only missense mutations |
| | 6 | SMO XOR !APC OR !TP53 XOR BRAF AND !RET AND !Therapy | 0.65 | 0.787 | 0.565 | 0.841 | 0.719 | 0.559 | 26 | 14 | 74 | 20 | |
| Adding APC | 6 | APC XOR !FLT3 OR Rectum AND !RET XOR PTEN AND !V+ | 0.75 | 0.638 | 0.469 | 0.857 | 0.694 | 0.491 | | | | | |
| Adding FLT3 | 9 | FLT3 XOR KRAS AND !RET AND !SMAD4 XOR BRAF AND !FGFR1 And !AKT1 OR GNA11 AND !GNAS | 0.5 | 0.851 | 0.588 | 0.8 | 0.67 | 0.53 | 20 | 20 | 80 | 14 | |
| Adding TP53 | 4 | !TP53 AND !EGFR XOR BRAF And !RET | 0.45 | 0.894 | 0.64 | 0.79 | 0.672 | 0.542 | 18 | 22 | 84 | 10 | best shorted signature with few false positives |
| | 5 | !TP53 AND !EGFR XOR BRAF And !RET ORpT4 | 0.6 | 0.787 | 0.54 | 0.82 | 0.69 | 0.536 | | | | | |

| | | | | | | | | | | | | | |
|---|---|---|---|---|---|---|---|---|---|---|---|---|---|
| Legend Table 15: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | | | | |

**Table 16: Further Preferred Functions Predicting of Metastasis vs. No Progression of Disease Event in Colorectal Cancer UICC Stage II based on Missense and Nonsense Sequence Variations With Sensitivity > 70%**

| Operands | Prediction Function | **S+** | **S-** | **PPV** | **NPV** | **AROC** | **CJR** | **TP** | **FP** | **TN** | **FP** |
|---|---|---|---|---|---|---|---|---|---|---|---|
| 5 | !TP53 XOR BRAF OR SMO AND !PTEN and !RET | 0.75 | 0.681 | 0.5 | 0.865 | 0.715 | 0.522 | 30 | 10 | 64 | 30 |
| 4 | !TP53 XOR BRAF OR SMO AND !PTEN | 0.75 | 0.67 | 0.492 | 0.863 | 0.71 | 0.514 | 30 | 10 | 63 | 31 |

| | | | | | | | | | | | |
|---|---|---|---|---|---|---|---|---|---|---|---|
| Legend Table 16: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | | |

**Table 17: Further Preferred Functions Predicting of Metastasis vs. No Progression of Disease Event in Colorectal Cancer UICC Stage II based on Missense Sequence Variations With Sensitivity > 70%**

| Operands | Prediction Function | **S+** | **S-** | **PPV** | **NPV** | **AROC** | **CJR** | **TP** | **FP** | **TN** | **FP** |
|---|---|---|---|---|---|---|---|---|---|---|---|
| 6 | !TP53 XOR BRAF OR SMO AND !APC AND !PTEN AND !RET | 0.75 | 0.66 | 0.484 | 0.861 | 0.705 | 0.506 | 30 | 10 | 62 | 32 |

| | | | | | | | | | | | |
|---|---|---|---|---|---|---|---|---|---|---|---|
| Legend Table 17: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | | |

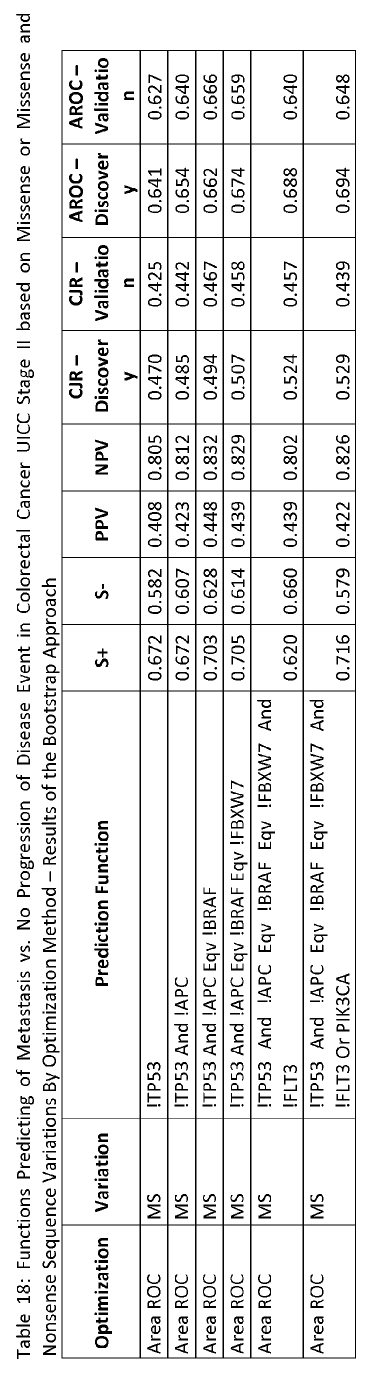

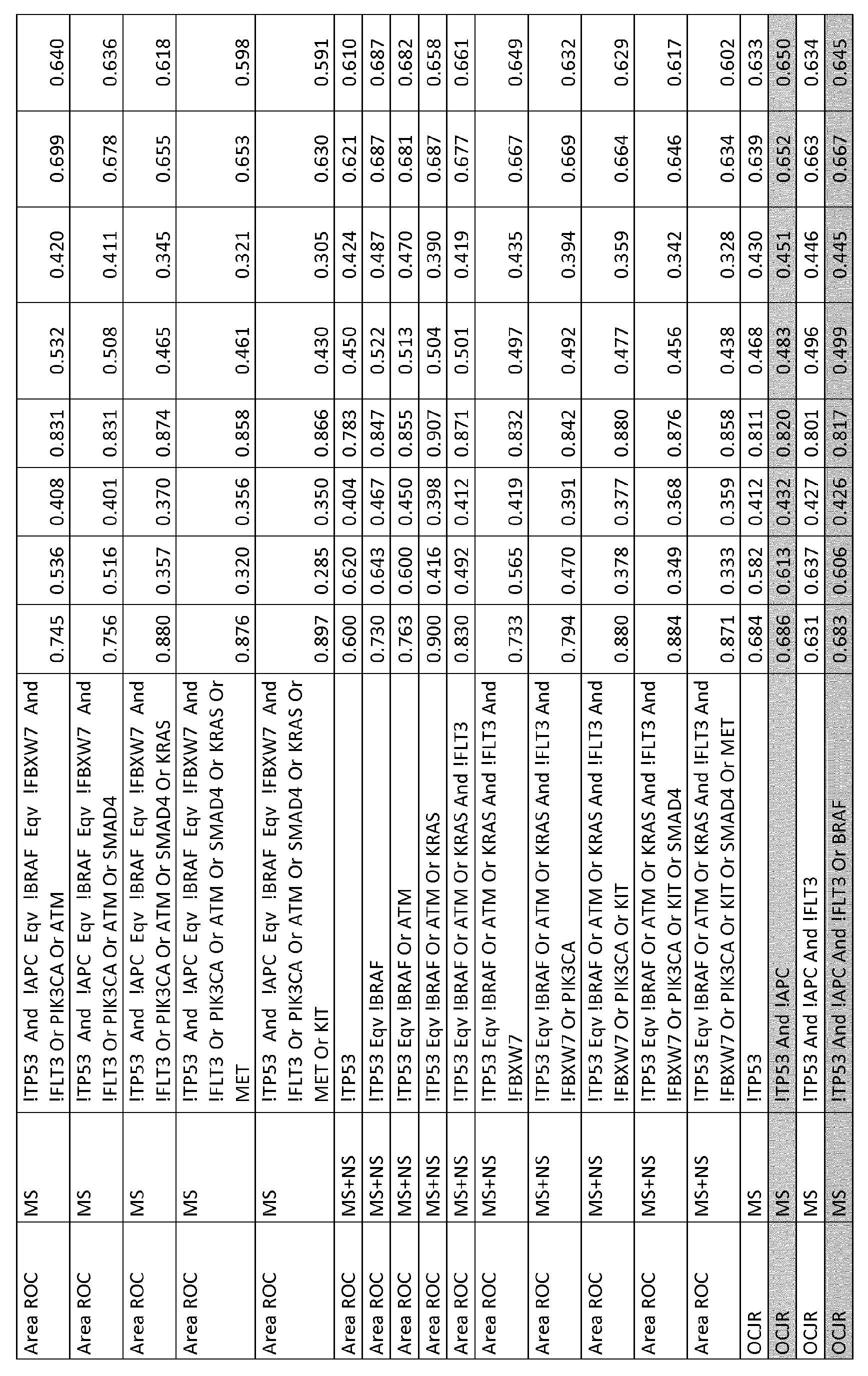

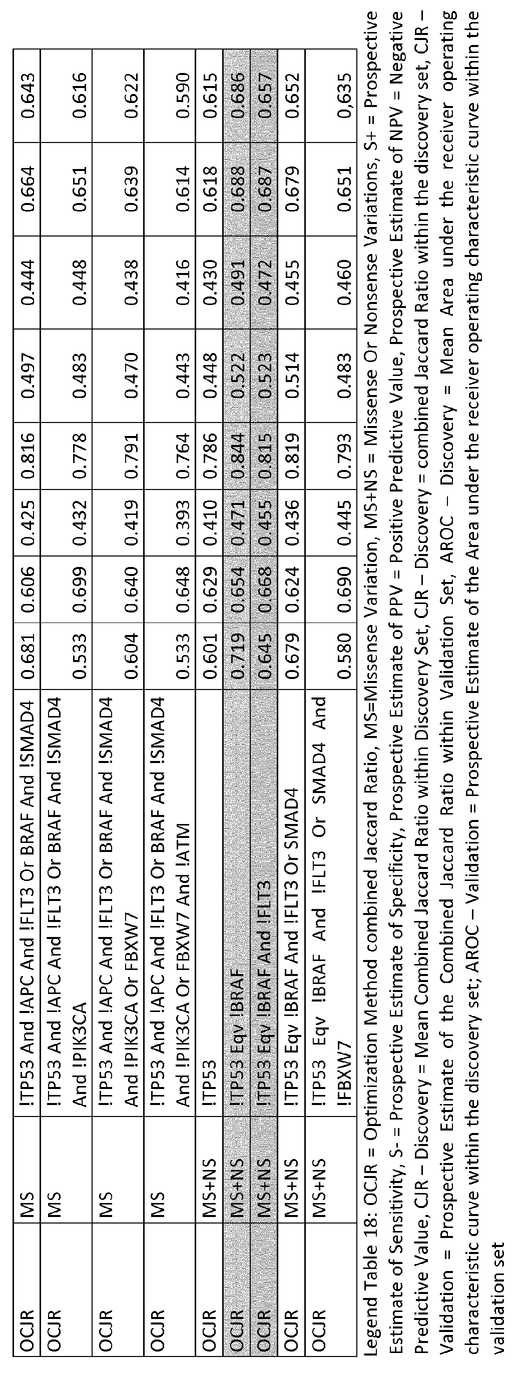

**Table 19: Functions Predicting Response to Bevacizumab + Chemotherapy in Colorectal Cancer UICC Stage IV based on Missense and Nonsense Sequence Variations**

| **Rank By Variation Count** | **Prediction Function** | **Sequence Variation Count** | **S+** | **S-** | **PPV** | **NPV** | **TP** | **TN** | **Rank By AROC** | **Rank By CJR** |
|---|---|---|---|---|---|---|---|---|---|---|
| 1. | !TP53 | | 0.545 | 0.682 | **0.614** | 0.444 | 6 | 15 | **1.** | |
| | TP53 | 20 | 0.455 | 0.318 | 0.250 | 0.538 | 5 | 7 | | 1. |
| 2. | !PIK3CA | | 0.727 | 0.045 | 0.273 | 0.386 | 8 | 1 | | 3. |
| | PIK3CA | 4 | 0.273 | 0.955 | **0.614** | 0.475 | 3 | 21 | **2.** | |
| 3. | !SMAD4 | | 0.727 | 0.045 | 0.386 | 0.145 | 8 | 1 | | |
| | SMAD4 | 4 | 0.273 | 0.955 | **0.614** | 0.475 | 3 | 21 | **2.** | |
| 4. | !CTNNB! | | 1.000 | 0.136 | **0.568** | 0.252 | 11 | 3 | **3.** | |
| | CTNNB1 | 3 | 0.000 | 0.864 | 0.432 | 0.288 | 0 | 19 | | |
| 5. | !KIT | | 0.727 | 0.182 | 0.455 | 0.218 | 8 | 4 | | 4. |
| | KIT | 7 | 0.273 | 0.818 | **0.545** | 0.400 | 3 | 18 | **4.** | |
| 6. | !KRAS | | 0.545 | 0.364 | 0.455 | 0.268 | 6 | 8 | | |
| | KRAS | 13 | 0.455 | 0.636 | **0.545** | 0.382 | 5 | 14 | **5.** | 3. |
| 7. | !JAK3 | | 0.909 | 0.000 | 0.455 | 0.152 | 10 | 0 | | |
| | JAK3 | 1 | 0.091 | 1.000 | **0.545** | 0.389 | 1 | 22 | **5.** | 2. |
| 8. | !KDR | | 0.636 | 0.455 | **0.545** | 0.344 | 7 | 12 | **6.** | |
| | KDR | 14 | 0.364 | 0.545 | 0.455 | 0.302 | 4 | 12 | | |
| 9. | !MET | | 0.100 | 0.091 | **0.545** | 0.223 | 11 | 2 | **7.** | |
| | MET | 2 | 0.000 | 0.909 | 0.455 | 0.303 | 0 | 20 | | |
| 10. | !FBXW7 | | 1.000 | 0.091 | **0.545** | 0.223 | 11 | 2 | **7.** | |
| | FBXW7 | 2 | 0.000 | 0.909 | 0.455 | 0.303 | 0 | 20 | | |
| 11. | !ERBB4 | | 1.000 | 0.091 | **0.545** | 0.223 | 11 | 2 | **7.** | |
| | ERBB4 | 2 | 0 | 0.909 | 0.455 | 0.303 | 0 | 20 | | |
| 12. | !ERBB2 | | 1.000 | 0.091 | **0.545** | 0.223 | 11 | 2 | **7.** | |
| | ERBB2 | 2 | 0.000 | 0.909 | 0.455 | 0.303 | 0 | 20 | | |
| 13. | !FLT3 | | 1.000 | 0.091 | **0.545** | 0.223 | 11 | 2 | **7.** | |
| | FLT3 | 2 | 0.000 | 0.909 | 0.455 | 0.303 | 0 | 20 | | |
| 14. | !ATM | | 0.909 | 0.045 | 0.477 | 0.178 | 10 | 1 | | |
| | ATM | 2 | 0.091 | 0.955 | **0.523** | 0.370 | 1 | 21 | **8.** | 4. |
| 15. | !ABL1 | | 1.000 | 0.455 | **0.523** | 0.195 | 11 | 1 | **9.** | |
| | ABL1 | 1 | 0.000 | 0.955 | 0.477 | 0.318 | 0 | 21 | | |
| 16. | !NRAS | | 1.000 | 0.455 | **0.523** | 0.195 | 11 | 1 | **9.** | |
| | NRAS | 1 | 0.000 | 0.955 | 0.477 | 0.318 | 0 | 21 | | |
| 17. | !CDH1 | | 1.000 | 0.045 | **0.523** | 0.195 | 11 | 1 | **9.** | |
| | CDH1 | 1 | 0.000 | 0.955 | 0.478 | 0.318 | 0 | 21 | | |
| 18. | !APC | | 0.636 | 0.364 | 0.500 | 0.294 | 7 | 8 | | |
| | APC | 12 | 0.364 | 0.636 | **0.500** | 0.347 | 4 | 14 | **10.** | |
| 19. | !BRAF | | 0.909 | 0.091 | 0.500 | 0.205 | 10 | 2 | | |
| | BRAF | 3 | 0.091 | 0.909 | **0.500** | 0.351 | 1 | 20 | **11.** | 5. |

| | | | | | | | | | | |
|---|---|---|---|---|---|---|---|---|---|---|
| Legend Table 19: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, TN = Count of true negatives, | | | | | | | | | | |

**Table 20: Functions Predicting Response to Bevacizumab + Chemotherapy in Colorectal Cancer UICC Stage IV based on Missense and Nonsense Sequence Variations Using All Genes with Variations in at least one patient**

| **Operands** | **Prediction Function** | **Comment** | **S+** | **S-** | **PPV** | **NPV** | **AROC** | **CJR** | **TP** | **FP** | **TN** | **FP** |
|---|---|---|---|---|---|---|---|---|---|---|---|---|
| **1** | PIK3CA | | 0.273 | 0.955 | 0.750 | 0.724 | 0.614 | 0.475 | 3 | 8 | 21 | 1 |
| | !TP53 | | 0.545 | 0.682 | 0.462 | 0.75 | 0.614 | 0.444 | 6 | 5 | 15 | 7 |
| | SMAD4 | | 0.273 | 0.955 | 0.750 | 0.724 | 0.614 | 0.475 | 3 | 8 | 21 | 1 |
| | !CTNNB1 | | 1.000 | 0.136 | 0.367 | 1.000 | 0.568 | 0.252 | 11 | 0 | 3 | 19 |
| | | | | | | | | | | | | |
| **2** | PIK3CA OR JAK3 | | 0.364 | 0.955 | 0.800 | 0.750 | 0.659 | 0.529 | 4 | 7 | 21 | 1 |
| | !TP53 OR KIT | | 0.727 | 0.591 | 0.471 | 0.813 | 0.636 | 0.460 | 8 | 11 | 13 | 9 |
| | SMAD4 OR JAK3 | | 0.364 | 0.955 | 0.800 | 0.750 | 0.659 | 0.529 | 4 | 7 | 21 | 1 |
| | !CTNNB1 AND !TP53 | | 0.545 | 0.773 | 0.545 | 0.697 | 0.659 | 0.502 | 6 | 5 | 17 | 5 |
| | | | | | | | | | | | | |
| **3** | PIK3CA OR JAK3 AND !NRAS | | 0.364 | 1.000 | 1.000 | 0.759 | 0.682 | 0.561 | 4 | 7 | 22 | 0 |
| | !TP53 OR KIT AND CTNNB1 | | 0.727 | 0.682 | 0.533 | 0.833 | 0.705 | 0.522 | 8 | 3 | 15 | 7 |
| | SMAD4 OR JAK3 OR !TP53 | | 0.727 | 0.636 | 0.500 | 0.824 | 0.667 | 0.491 | 8 | 3 | 14 | 8 |
| | !CTNNB1 AND !TP53 OR JAK3 | | 0.636 | 0.773 | 0.583 | 0.810 | 0.705 | 0.546 | 7 | 4 | 17 | 5 |
| | | | | | | | | | | | | |
| **4** | PIK3CA OR JAK3 AND !NRAS OR ATM | | 0.455 | 0.955 | 0.833 | 0.778 | 0.705 | 0.583 | 5 | 6 | 21 | 1 |
| | !TP53 OR KIT AND CTNNB1 AND MET | | 0.727 | 0.773 | 0.615 | 0.850 | 0.750 | 0.590 | 8 | 3 | 17 | 5 |
| | SMAD4 OR JAK3 OR !TP53 AND CTNNB1 | | 0.727 | 0.727 | 0.571 | 0.842 | 0.727 | 0.555 | 8 | 3 | 16 | 6 |
| | !CTNNB1 AND !TP53 OR JAK3 AND !MET | | 0.636 | 0.818 | 0.636 | 0.818 | 0.727 | 0.579 | 7 | 4 | 18 | 4 |
| | | | | | | | | | | | | |
| **5** | **PIK3CA OR JAK3 AND !NRAS OR ATM OR SMAD4** | max. | **0.545** | **0.909** | **0.750** | **0.800** | **0.727** | **0.610** | **6** | **5** | **20** | **2** |
| | !TP53 OR KIT AND CTNNB1 AND MET OR SMAD4 | max. | 0.818 | 0.727 | 0.600 | 0.889 | 0.773 | 0.598 | 9 | 2 | 16 | 6 |
| | SMAD4 OR JAK3 OR !TP53 AND CTNNB1 AND !MET | | 0.727 | 0.773 | 0.615 | 0.850 | 0.750 | 0.590 | 8 | 3 | 17 | 5 |
| | !CTNNB1 AND !TP53 OR JAK3 AND !MET | | 0.727 | 0.773 | 0.615 | 0.850 | 0.750 | 0.590 | 8 | 3 | 17 | 5 |
| **6** | PIK3CA OR JAK3 AND !NRAS OR ATM OR SMAD4 | | | | | | | | | | | |
| | !TP53 OR KIT AND CTNNB1 AND MET OR SMAD4 | | | | | | | | | | | |
| | SMAD4 OR JAK3 OR !TP53 AND CTNNB1 AND !MET | | | | | | | | | | | |
| | !CTNNB1 AND !TP53 OR JAK3 AND !MET AND !KDR | | 0.545 | 0.909 | 0.75 | 0.8 | 0.727 | 0.601 | 6 | 5 | 20 | 2 |

| | | | | | | | | 2014 | **Positives** | **Negatives** | **Negatives** | **Positives** | |
|---|---|---|---|---|---|---|---|---|---|---|---|---|---|
| | **2er** | PIK3CA AND KRAS | 0,273 | 1,000 | 1,000 | 0,733 | 0,636 | 0,503 | 3 | 8 | 22 | 0 | |
| SIG02 | 4EP-3 | **!TP53 OR KIT** | **0,727** | **0,591** | **0,471** | **0,813** | **0,659** | **0,460** | **8** | **3** | **13** | **9** | |
| | | **!TP53 AND PIK3CA** | **0,273** | **1,000** | **1,000** | **0,733** | **0,636** | **0,503** | **3** | **8** | **20** | **0** | |
| | | !ATM XOR PIK3CA | 0,364 | 0,909 | 0,667 | 0,741 | 0,636 | 0,499 | 4 | 7 | 20 | 2 | |
| | | SMAD4 OR ATM | 0,364 | 0,909 | 0,667 | 0,741 | 0,636 | 0,499 | 4 | 7 | 20 | 2 | |
| | | !CTNNB1 AND !TP53 | 1,000 | 0,136 | 0,367 | 1,000 | 0,568 | 0,252 | 11 | 0 | 3 | 19 | |
| | | | | | | | | | | | | | |
| | **3er** | PIK3CA AND KRAS OR ATM | 0,364 | 0,955 | 0,800 | 0,750 | 0,659 | 0,529 | 4 | 7 | 21 | 1 | |
| | | !TP53 OR KIT AND !CTNNB1 | 0,727 | 0,682 | 0,533 | 0,833 | 0,705 | 0,522 | 3 | 8 | 15 | 7 | |
| | | !TP53 AND PIK3CA OR ATM | 0,364 | 0,955 | 0,800 | 0,750 | 0,659 | 0,529 | 4 | 7 | 21 | 1 | |
| | | **!ATM XOR PIK3CA AND !TP53** | **0,364** | **1,000** | **1,000** | **0,759** | **0,682** | **0,561** | **4** | **11** | **22** | **0** | 4th |
| | | SMAD4 OR ATM OR KIT | 0,545 | 0,773 | 0,545 | 0,773 | 0,659 | 0,502 | 6 | 5 | 17 | 5 | |
| | | SMAD4 OR ATM OR PIK3CA | 0,455 | 0,864 | 0,625 | 0,760 | 0,659 | 0,518 | 5 | 6 | 19 | 3 | |
| | | **!CTNNB1 AND !TP53 OR KIT** | **0,727** | **0,682** | **0,533** | **0,833** | **0,705** | **0,522** | **8** | **3** | **15** | **7** | |
| | | !CTNNB1 AND !TP53 AND !KDR | 0,455 | 0,909 | 0,714 | 0,769 | 0,682 | 0,549 | 5 | 6 | 20 | **2** | |
| | | | | | | | | | | | | | |
| | **4er** | PIK3CA AND KRAS OR ATM AND !TP53 | 0,364 | 1,000 | 1,000 | 0,759 | 0,682 | 0,561 | 4 | 7 | 22 | 0 | |
| | | **!TP53 OR KIT AND !CTNNB1 AND !MET** | **0,727** | **0,773** | **0,615** | **0,850** | **0,750** | **0,590** | **8** | **3** | **17** | **5** | 2nd |
| max | | !TP53 AND PIK3CA OR ATM OR SMAD4 | 0,455 | 0,909 | 0,714 | 0,969 | 0,682 | 0,549 | 5 | 6 | 20 | 2 | |
| | | !ATM XOR PIK3CA AND !TP53 OR SMAD4 | 0,455 | 0,955 | 0,833 | 0,778 | 0,705 | 0,585 | 5 | 11 | 21 | 1 | 3rd |
| | | SMAD4 OR ATM OR KIT AND !FBXW7 | 0,545 | 0,818 | 0,600 | 0,783 | 0,682 | 0,533 | 6 | 5 | 18 | 4 | |
| max | | SMAD4 OR ATM OR PIK3CA AND !TP53 | 0,364 | 1,000 | 1,000 | 0,759 | 0,682 | 0,561 | 4 | 7 | 22 | 0 | |
| | | **!CTNNB1 AND !TP53 OR KIT AND MET** | **0,727** | **0,773** | **0,615** | **0,850** | **0,750** | **0,590** | **8** | **3** | **17** | **5** | 2nd |
| | | !CTNNB1 AND !TP53 AND !KDR AND !MET | 0,455 | 0,955 | 0,833 | 0,788 | 0,705 | 0,583 | 5 | 6 | 21 | 1 | |
| | | | | | | | | | | | | | |
| max | **5er** | PIK3CA AND KRAS OR ATM AND !TP53 OR SMAD4 | 0,455 | 0,955 | 0,833 | 0,778 | 0,705 | 0,583 | 5 | 6 | 21 | 1 | |
| max | | **!TP53 OR KIT AND !CTNNB1 AND !MET OR SMAD4** | **0,818** | **0,727** | **0,600** | **0,889** | **0,773** | **0,598** | **9** | **2** | **16** | **6** | 1st |
| | | !TP53 AND PIK3CA OR ATM OR SMAD4 OR KIT | 0,636 | 0,773 | 0,583 | 0,810 | 0,705 | 0,546 | 7 | 4 | 17 | 5 | |
| max | | SMAD4 OR ATM OR KIT AND !FBXW7 OR PIK3CA | 0,636 | 0,773 | 0,583 | 0,810 | 0,705 | 0,546 | 7 | 4 | 17 | 5 | |
| max | | SMAD4 OR ATM OR PIK3CA AND !TP53 AND !BRAF | 0,364 | 1,000 | 1,000 | 0,759 | 0,682 | 0,561 | 4 | 7 | 22 | 0 | |
| max | | !CTNNB1 AND !TP53 OR KIT AND MET OR SMAD4 | 0,818 | 0,727 | 0,600 | 0,889 | 0,773 | 0,598 | 9 | 2 | 16 | 6 | |
| | | **!CTNNB1 AND !TP53 AND !KDR AND !MET OR PIK3CA** | **0,545** | **0,909** | **0,750** | **0,800** | **0,727** | **0,601** | **6** | **5** | **20** | **2** | |
| | | | | | | | | | | | | | |
| max | **6er** | !TP53 AND PIK3CA OR | 0,636 | 0,818 | 0,636 | 0,818 | 0,727 | 0,579 | 7 | 4 | 18 | 4 | |
| max | **6er** | !TP53 AND PIK3CA OR ATM OR SMAD4 OR KIT AND FBXW7 | 0,636 | 0,818 | 0,636 | 0,818 | 0,727 | 0,579 | 7 | 4 | 18 | 4 | |
| | | **!CTNNB1 AND !TP53 AND !KDR AND !MET OR PIK3CA** | | | | | | | | | | | |

| | | | | | | | | | | | | | |
|---|---|---|---|---|---|---|---|---|---|---|---|---|---|
| Legend Table 20: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | | | | |

**Table 21: Functions Predicting Response to Bevacizumab + Chemotherapy in Colorectal Cancer UICC Stage IV based on Missense and Nonsense Sequence Variations Using All Genes with Variations in at least two patients**

| **Com ment** | **Operands** | **Prediction Function** | **S+** | **S-** | **PPV** | **NPV** | **AROC** | **CJR** | **TP** | **FP** | **TN** | **FP** | |
|---|---|---|---|---|---|---|---|---|---|---|---|---|---|
| | **2** | PIK3CA AND KRAS | 0.273 | 1.000 | 1.000 | 0.733 | 0.636 | 0.503 | 3 | 8 | 22 | 0 | |
| | | **!TP53 OR KIT** | **0.727** | **0.591** | **0.471** | **0.813** | **0.659** | **0.460** | **8** | **3** | **13** | **9** | |
| | | **!TP53 AND** PIK3CA | **0.273** | **1.000** | **1.000** | **0.733** | **0.636** | **0.503** | **3** | **8** | **20** | **0** | |
| | | !ATM XOR PIK3CA | 0.364 | 0.909 | 0.667 | 0.741 | 0.636 | 0.499 | 4 | 7 | 20 | **2** | |
| | | SMAD4 OR ATM | 0.364 | 0.909 | 0.667 | 0.741 | 0.636 | 0.499 | 4 | 7 | 20 | **2** | |
| | | !CTNNB1 AND !TP53 | 1.000 | 0.136 | 0.367 | 1.000 | 0.568 | 0.252 | 11 | 0 | 3 | 19 | |
| | | | | | | | | | | | | | |
| | **3** | PIK3CA AND KRAS OR ATM | 0.364 | 0.955 | 0.800 | 0.750 | 0.659 | 0.529 | 4 | 7 | 21 | 1 | |
| | | !TP53 OR KIT AND !CTNNB1 | 0.727 | 0.682 | 0.533 | 0.833 | 0.705 | 0.522 | 3 | 8 | 15 | 7 | |
| | | !TP53 AND PIK3CA OR ATM | 0.364 | 0.955 | 0.800 | 0.750 | 0.659 | 0.529 | 4 | 7 | 21 | 1 | |
| | | **!ATM XOR PIK3CA AND !TP53** | **0.364** | **1.000** | **1.000** | **0.759** | **0.682** | **0.561** | **4** | **11** | **22** | **0** | 4th |
| | | SMAD4 OR ATM OR KIT | 0.545 | 0.773 | 0.545 | 0.773 | 0.659 | 0.502 | 6 | 5 | 17 | 5 | |
| | | SMAD4 OR ATM OR PIK3CA | 0.455 | 0.864 | 0.625 | 0.760 | 0.659 | 0.518 | 5 | 6 | 19 | 3 | |
| | | **!CTNNB1 AND !TP53 OR KIT** | **0.727** | **0.682** | **0.533** | **0.833** | **0.705** | **0.522** | **8** | **3** | **15** | **7** | |
| | | !CTNNB1 AND !TP53 AND !KDR | 0.455 | 0.909 | 0.714 | 0.769 | 0.682 | 0.549 | 5 | 6 | 20 | **2** | |
| | | | | | | | | | | | | | |
| | **4** | PIK3CA AND KRAS OR ATM AND !TP53 | 0.364 | 1.000 | 1.000 | 0.759 | 0.682 | 0.561 | 4 | 7 | 22 | 0 | |
| | | **!TP53 OR KIT AND !CTNNB1 AND !MET** | **0.727** | **0.773** | **0.615** | **0.850** | **0.750** | **0.590** | **8** | **3** | **17** | **5** | 2nd |
| max | | !TP53 AND PIK3CA OR ATM OR SMAD4 | 0.455 | 0.909 | 0.714 | 0.969 | 0.682 | 0.549 | 5 | 6 | 20 | 2 | |
| | | !ATM XOR PIK3CA AND !TP53 OR SMAD4 | 0.455 | 0.955 | 0.833 | 0.778 | 0.705 | 0.585 | 5 | 11 | 21 | 1 | 3rd |
| | | SMAD4 OR ATM OR KIT AND !FBXW7 | 0.545 | 0.818 | 0.600 | 0.783 | 0.682 | 0.533 | 6 | 5 | 18 | 4 | |
| max | | SMAD4 OR ATM OR PIK3CA AND !TP53 | 0.364 | 1.000 | 1.000 | 0.759 | 0.682 | 0.561 | 4 | 7 | 22 | 0 | |
| | | **!CTNNB1 AND !TP53 OR KIT AND MET** | **0.727** | **0.773** | **0.615** | **0.850** | **0.750** | **0.590** | **8** | **3** | **17** | **5** | 2nd |
| | | !CTNNB1 AND !TP53 AND !KDR AND !MET | 0.455 | 0.955 | 0.833 | 0.788 | 0.705 | 0.583 | 5 | 6 | 21 | 1 | |
| | | | | | | | | | | | | | |
| max | **5** | PIK3CA AND KRAS OR ATM AND !TP53 OR SMAD4 | 0.455 | 0.955 | 0.833 | 0.778 | 0.705 | 0.583 | 5 | 6 | 21 | 1 | |
| max | | **!TP53 OR KIT AND !CTNNB1 AND !MET OR SMAD4** | **0.818** | **0.727** | **0.600** | **0.889** | **0.773** | **0.598** | **9** | **2** | **16** | **6** | 1st |
| | | !TP53 AND PIK3CA OR ATM OR SMAD4 OR KIT | 0.636 | 0.773 | 0.583 | 0.810 | 0.705 | 0.546 | 7 | 4 | 17 | 5 | |
| max | | SMAD4 OR ATM OR KIT AND !FBXW7 OR PIK3CA | 0.636 | 0.773 | 0.583 | 0.810 | 0.705 | 0.546 | 7 | 4 | 17 | 5 | |
| max | | SMAD4 OR ATM OR PIK3CA AND !TP53 AND !BRAF | 0.364 | 1.000 | 1.000 | 0.759 | 0.682 | 0.561 | 4 | 7 | 22 | 0 | |
| max | | !CTNNB1 AND !TP53 OR KIT AND MET OR SMAD4 | 0.818 | 0.727 | 0.600 | 0.889 | 0.773 | 0.598 | 9 | **2** | 16 | 6 | |
| | | **!CTNNB1 AND !TP53 AND !KDR AND !MET OR PIK3CA** | **0.545** | **0.909** | **0.750** | **0.800** | **0.727** | **0.601** | **6** | **5** | **20** | **2** | |
| | | | | | | | | | | | | | |
| max | **6er** | !TP53 AND PIK3CA OR ATM OR SMAD4 OR KIT AND FBXW7 | 0.636 | 0.818 | 0.636 | 0.818 | 0.727 | 0.579 | 7 | 4 | 18 | 4 | |
| max | | **!CTNNB1 AND !TP53 AND !KDR AND !MET OR PIK3CA OR SMAD4** | **0.636** | **0.864** | **0.700** | **0.826** | **0.750** | **0.615** | **7** | **4** | **19** | **3** | |

| | | | | | | | | | | | | | |
|---|---|---|---|---|---|---|---|---|---|---|---|---|---|
| Legend Table 21: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | | | | |

**Table 22: Functions Predicting Response to Bevacizumab + Chemotherapy in Colorectal Cancer UICC Stage IV based on Missense and Nonsense Sequence Variations Using All Genes with Variations in at least five patients**

| Operands | Prediction Function | **Comment** | **S+** | **S-** | **PPV** | **NPV** | **AROC** | **CJR** | **TP** | **FP** | **TN** | **FP** | |
|---|---|---|---|---|---|---|---|---|---|---|---|---|---|
| **2er** | !TP53 OR KIT | | 0.727 | 0.591 | 0.471 | 0.813 | 0.659 | 0.46 | 8 | 3 | 13 | 9 | |
| | **!CTNNB1 AND !TP53** | | **0.545** | **0.773** | **0.545** | **0.773** | **0.659** | **0.502** | **6** | **5** | **17** | **5** | |
| | !ATM XOR !KIT | | 0.364 | 0.864 | 0.571 | 0.731 | **0.614** | 0.47 | 4 | 7 | 19 | 3 | |
| | !PIK3CA XOR KRAS | | 0.818 | 0.409 | 0.409 | 0.818 | **0.614** | 0.375 | 9 | 2 | 9 | 13 | |
| | SMAD4 OR !TP53 | | 0.636 | 0.636 | 0.467 | 0.778 | 0.636 | 0.453 | 7 | 4 | 14 | 8 | |
| | | | | | | | | | | | | | |
| **3er** | !TP53 OR KIT OR KRAS | | 0.909 | 0.409 | 0.435 | 0.900 | 0.659 | 0.404 | 10 | 1 | 9 | 13 | |
| | **!CTNNB1 AND !TP53 OR KIT** | | **0.727** | **0.682** | **0.553** | **0.833** | **0.705** | **0.522** | **8** | **3** | **15** | **7** | 4th |
| | !ATM XOR !KIT OR !TP53 | | 0.727 | 0.636 | 0.500 | 0.824 | 0.682 | 0.491 | 8 | 3 | 14 | 8 | |
| | !ATM XOR !PIK3CA OR !TP53 | | 0.364 | 1 | 1.000 | 0.759 | 0.682 | 0.561 | 4 | 7 | 22 | 0 | 4th |
| | !PIK3CA XOR KRAS AND !TP53 | | 0.545 | 0.818 | 0.600 | 0.783 | 0.682 | 0.533 | 6 | 5 | 18 | 4 | 5th |
| | SMAD4 OR !TP53 OR KIT | | 0.818 | 0.545 | 0.474 | 0.857 | 0.682 | 0.464 | 9 | **2** | 12 | 10 | |
| | | | | | | | | | | | | | |
| **4er** | !TP53 OR KIT OR KRAS AND KDR | | 0.636 | 0.727 | 0.538 | 0.800 | 0.682 | 0.517 | 7 | 4 | 16 | 6 | 6th |
| | !CTNNB1 AND !TP53 OR KIT OR KRAS | sensitivity optimized signatu re | 0.909 | 0.455 | 0.455 | 0.909 | 0.682 | 0.435 | 10 | 1 | 10 | 12 | |
| | !ATM XOR KIT OR !TP53 OR KRAS | | 0.909 | 0.455 | 0.455 | 0.909 | 0.682 | 0.435 | 10 | 1 | 10 | 12 | |
| | **!PIK3CA XOR KRAS AND !TP53 OR KIT** | best signature | **0.727** | **0.727** | **0.571** | **0.842** | **0.727** | **0.555** | **8** | **3** | **16** | **6** | 2nd |
| | **!PIK3CA XOR KRAS AND !TP53 XOR KIT** | specificity optimzed signatu re | **0.636** | **0.818** | **0.636** | **0.818** | **0.727** | **0.579** | **7** | **4** | **18** | **4** | 1st |
| | SMAD4 OR !TP53 OR KIT OR KRAS | | 0.909 | 0.409 | 0.435 | 0.9 | 0.659 | 0.404 | 10 | 1 | 9 | 13 | |
| | | | | | | | | | | | | | |
| **5er** | !TP53 containing 5er string does not work | | | | | | | | | | | | |
| | **!CTNNB1 AND !TP53 OR KIT OR KRAS AND !KDR** | | **0.636** | **0.773** | **0.583** | **0.810** | **0.705** | **0.546** | **7** | **4** | **17** | **5** | 3rd |
| | **!ATM XOR KIT OR !TP53 OR KRAS AND !KDR** | | **0.636** | **0.773** | **0.583** | **0.810** | **0.705** | **0.546** | **7** | **4** | **17** | **5** | 3rd |
| | !PIK3CA XOR KRAS AND !TP53 XOR KIT AND !APC | | 0.545 | 0.818 | 0.600 | 0.783 | 0.682 | 0.533 | 6 | 5 | 18 | 4 | 5th |
| | SMAD4 OR !TP53 OR KIT OR KRAS | | 0.636 | 0.727 | 0.538 | 0.800 | 0.682 | 0.514 | 7 | 4 | 16 | 6 | |

| | | | | | | | | | | | | | |
|---|---|---|---|---|---|---|---|---|---|---|---|---|---|
| Legend Table 22: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | | | | |

**Table 23: Functions Predicting Response to Bevacizumab + Chemotherapy in Colorectal Cancer UICC Stage IV based on Missense Sequence Variations**

| Operands | Prediction Function | | **S+** | **S-** | **PPV** | **NPV** | **AROC** | **CJR** | **TP** | **FP** | **TN** | **FP** | **Comment** |
|---|---|---|---|---|---|---|---|---|---|---|---|---|---|
| 3 | !ATM XOR PIK3CA AND !TP53 | max | 0.364 | 1.000 | 1.000 | 0.759 | 0.682 | 0.561 | 4 | 7 | 22 | 0 | 3 Genes reach maximum. with additional nonsense mutations 4er string is slightly better |
| | | | | | | | | | | | | | |
| 4 | PIK3CA AND KRAS OR ATM AND !TP53 | max | 0.364 | 1.000 | 1.000 | 0.759 | 0.682 | 0.561 | 4 | 7 | 22 | 0 | 4 Genes reach maximum. |
| | !TP53 OR KIT AND !CTNNB1 AND !MET | max | 0.727 | 0.682 | 0.533 | 0.833 | 0.705 | 0.522 | 8 | 3 | 15 | 7 | 4 Genes reach maximum. with additional nonsense mutations similar 4er string os slightly better |
| | SMAD4 OR ATM AND !TP53 OR PIK3CA | max | 0.364 | 0.909 | 0.667 | 0.741 | 0.636 | 0.499 | 4 | 7 | 20 | 2 | 4 Genes reach max. performs less good than similar signature with nonsense mutations |
| | | | | | | | | | | | | | |
| 5 | **!TP53 AND PIK3CA OR ATM OR KIT AND !FBXW7** | | **0.545** | **0.864** | **0.667** | **0.792** | **0.705** | **0.566** | **6** | **5** | **19** | **3** | |
| | !CTNNB! AND Plk3CA AND KRAS OR ATM AND !TP53 | max | 0.364 | 1.000 | 1.000 | 0.759 | 0.682 | 0.561 | 4 | 7 | 22 | 0 | 5 Genes reach maximum. performance less good than CTNNB! Containing strings when missense and nonsense mutations are considered |

| | | | | | | | | | | | | | |
|---|---|---|---|---|---|---|---|---|---|---|---|---|---|
| Legend Table 23: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | | | | |

**Table 24: Functions Predicting Response to Bevacizumab + Chemotherapy in Colorectal Cancer UICC Stage IV based on Missense and Synonymous Sequence Variations**

| Operands | Prediction Function | Comment | **S+** | **S-** | **PPV** | **NPV** | **AROC** | **CJR** | **TP** | **FP** | **TN** | **FP** |
|---|---|---|---|---|---|---|---|---|---|---|---|---|
| 5 | **!CTNNB1 AND EGFR OR PIK3CA XOR ERBB4 OR IDH1** | **max** | **0.818** | **0.864** | **0.75** | **0.905** | **0.841** | **0.717** | **9** | **2** | **19** | **3** |
| 6 | PIK3CA OR IDH1 OR ATM AND !TP53 OR ERBB4 AND !ERBB2 | max | 0.636 | 0.955 | 0.875 | 0.84 | 0.795 | 0.696 | 7 | 4 | 21 | 1 |
| | !TP53 AND PIK3CA OR IDH1 OR ATM OR ERBB4 AND !ERBB2 | max | 0.722 | 0.864 | 0.727 | 0.864 | 0.795 | 0.666 | 8 | 11 | 19 | 3 |
| 7 | SMAD4 OR IDH1 OR ATM AND !APC OR PIK3CA OR ERBB4 AND !ERBB2 | max | 0.722 | 0.909 | 0.8 | 0.87 | 0.818 | 0.708 | 8 | 11 | 20 | 2 |

| | | | | | | | | | | | | |
|---|---|---|---|---|---|---|---|---|---|---|---|---|
| Legend Table 24: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | | | |

**Table 25: Functions Predicting Response to Bevacizumab + Chemotherapy in Colorectal Cancer UICC Stage IV based on Missense, Nonsense and Synonymous Sequence \/ariations**

| Operands | Prediction Function / Comment | | **S+** | **S-** | **PPV** | **NPV** | **AROC** | **CJR** | **TP** | **FP** | **TN** | **FP** |
|---|---|---|---|---|---|---|---|---|---|---|---|---|
| | Sequence Variation Count at least 2 | | | | | | | | | | | |
| 4 | **SMAD4 XOR ERBB4 XOR ALK OR IDH1** | **max** | **0.909** | **0.864** | **0.769** | **0.95** | **0.886** | **0.770** | **10** | **1** | **19** | **3** |
| | !CTNNB1 AND SMAD4 OR ERBB4 XOR ALK | | 0.818 | 0.864 | 0.75 | 0.905 | 0.841 | 0.717 | 9 | 2 | 19 | 3 |
| | | | | | | | | | | | | |
| 5 | !CTNNB1 AND SMAD4 OR ERBB4 XOR ALK OR IDH1 | max | 0.909 | 0.811 | 0.714 | 0.947 | 0.864 | 0.725 | 10 | 1 | 18 | 4 |
| | Sequence Variation Count at least 5 | | | | | | | | | | | |
| 3 | SMAD4 OR IDH1 And !TP53 | max | 0.450 | 1.000 | 1.000 | 0.786 | 0.722 | 0.620 | 5 | 6 | 22 | 0 |

| | | | | | | | | | | | | |
|---|---|---|---|---|---|---|---|---|---|---|---|---|
| Legend Table 25: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | | | |

**Table 25B: Functions Predicting Response to Bevacizumab + Chemotherapy in Colorectal Cancer UICC Stage IV based on Missense, Nonsense and Synonymous Sequence Variations**

| **Max** | **Ther** | **Var** | **Signature** | **M1** | **M2** | **M3** | **M4** | **M5** | **M6** | **M7** | **M8** | **M9** | **M10** |
|---|---|---|---|---|---|---|---|---|---|---|---|---|---|
| Area ROC | Bevacizum ab | Missense | PIK3CA | 0.28 | 0.94 | 0.72 | 0.72 | 0.388 | 0.474 | 0.623 | 0.613 | F | T |
| Area ROC | Bevacizum ab | Missense | PIK3CA Xor !KRAS | 0.82 | 0.42 | 0.41 | 0.82 | 0.478 | 0.379 | 0.644 | 0.616 | F | T |
| Area ROC | Bevacizum ab | Missense | PIK3CA Xor !KRAS Xor TP53 | 0.81 | 0.59 | 0.50 | 0.86 | 0.600 | 0.492 | 0.746 | 0.701 | F | T |
| Area ROC | Bevacizum ab | Missense | PIK3CA Xor !KRAS Xor TP53 Nimp CTNNB1 | 0.84 | 0.67 | 0.56 | 0.89 | 0.621 | 0.564 | 0.764 | 0.756 | F | T |
| Area ROC | Bevacizum ab | Missense | PIK3CA Xor !KRAS Xor TP53 Nimp CTNNB1 And !KDR | 0.67 | 0.87 | 0.72 | 0.84 | 0.604 | 0.634 | 0.776 | 0.766 | T | T |
| Area ROC | Bevacizum ab | Missense | PIK3CA Xor !KRAS Xor TP53 Nimp CTNNB1 And !KDR And !BRAF | 0.64 | 0.87 | 0.71 | 0.83 | 0.617 | 0.624 | 0.784 | 0.756 | T | T |
| Area ROC | Bevacizum ab | Missense | PIK3CA Xor !KRAS Xor TP53 Nimp CTNNB1 And !KDR And !BRAF Or KIT | 0.81 | 0.71 | 0.58 | 0.88 | 0.640 | 0.576 | 0.782 | 0.757 | F | T |
| Area ROC | Bevacizum ab | Missense | PIK3CA Xor !KRAS Xor TP53 Nimp CTNNB1 And !KDR And !BRAF Or KIT Or SMAD4 | 0.84 | 0.70 | 0.58 | 0.89 | 0.580 | 0.581 | 0.733 | 0.766 | T | T |
| Area ROC | Bevacizum ab | Missense and Nonsense | PIK3CA | 0.26 | 0.97 | 0.79 | 0.72 | 0.393 | 0.475 | 0.625 | 0.614 | F | T |
| Area ROC | Bevacizum ab | Missense and Nonsense | PIK3CA Eqv KRAS | 0.82 | 0.41 | 0.41 | 0.82 | 0.481 | 0.374 | 0.646 | 0.613 | F | T |
| Area ROC | Bevacizum ab | Missense and Nonsense | PIK3CA Eqv KRAS Nimp CTNNB1 | 0.81 | 0.45 | 0.42 | 0.82 | 0.520 | 0.398 | 0.680 | 0.629 | F | F |
| Area ROC | Bevacizum ab | Missense and Nonsense | PIK3CA Eqv KRAS Nimp CTNNB1 And !TP53 | 0.57 | 0.88 | 0.70 | 0.80 | 0.506 | 0.588 | 0.694 | 0.723 | F | T |
| Area ROC | Bevacizum ab | Missense and Nonsense | PIK3CA Eqv KRAS Nimp CTNNB1 And !TP53 Or KIT | 0.74 | 0.78 | 0.62 | 0.85 | 0.571 | 0.598 | 0.730 | 0.756 | T | T |
| Area ROC | Bevacizum ab | Missense and Nonsense | PIK3CA Eqv KRAS Nimp CTNNB1 And !TP53 Or KIT Or SMAD4 | 0.82 | 0.73 | 0.60 | 0.89 | 0.579 | 0.599 | 0.732 | 0.774 | T | T |
| Area ROC | Bevacizum ab | Missense and Nonsense | PIK3CA Eqv KRAS Nimp CTNNB1 And !TP53 Or KIT Or SMAD4 Nimp BRAF | 0.72 | 0.73 | 0.57 | 0.84 | 0.543 | 0.553 | 0.703 | 0.724 | T | T |
| Area ROC | Bevacizum ab | Missense and Nonsense | PIK3CA Eqv KRAS Nimp CTNNB1 And !TP53 Or KIT Or SMAD4 Nimp BRAF Nimp KDR | 0.55 | 0.85 | 0.65 | 0.79 | 0.494 | 0.559 | 0.686 | 0.701 | F | T |
| Area ROC | Bevacizum ab | Missense and Nonsense | PIK3CA Eqv KRAS Nimp CTNNB1 And !TP53 Or KIT Or SMAD4 Nimp BRAF Nimp KDR And !APC | 0.34 | 0.86 | 0.55 | 0.72 | 0.417 | 0.458 | 0.627 | 0.602 | T | T |
| Combined Jacc ard Ratio | Bevacizum ab | Missense | !TP53 | 0.61 | 0.56 | 0.41 | 0.74 | 0.401 | 0.397 | 0.569 | 0.585 | T | T |
| Combined Jacc ard Ratio | Bevacizum ab | Missense | !TP53 Xor CTNNB1 | 0.64 | 0.60 | 0.44 | 0.77 | 0.456 | 0.432 | 0.627 | 0.621 | T | T |
| Combined Jacc ard Ratio | Bevacizum ab | Missense | !TP53 Xor CTNNB1 Nimp BRAF | 0.62 | 0.60 | 0.44 | 0.76 | 0.462 | 0.423 | 0.632 | 0.608 | T | T |
| Combined Jacc ard Ratio | Bevacizum ab | Missense | !TP53 Xor CTNNB1 Nimp BRAF Or KRAS | 0.80 | 0.44 | 0.42 | 0.82 | 0.468 | 0.391 | 0.636 | 0.622 | F | T |
| Combined Jacc ard Ratio | Bevacizum ab | Missense | !TP53 Xor CTNNB1 Nimp BRAF Or KRAS Xor KDR | 0.83 | 0.67 | 0.56 | 0.88 | 0.575 | 0.557 | 0.729 | 0.748 | T | T |
| Combined Jacc ard Ratio | Bevacizum ab | Missense | !TP53 Xor CTNNB1 Nimp BRAF Or KRAS Xor KDR Or PIK3CA | 0.91 | 0.58 | 0.52 | 0.93 | 0.663 | 0.525 | 0.792 | 0.744 | F | T |
| Combined Jacc ard Ratio | Bevacizum ab | Missense | !TP53 Xor CTNNB1 Nimp BRAF Or KRAS Xor KDR Or PIK3CA Or SMAD4 | 0.89 | 0.58 | 0.51 | 0.91 | 0.670 | 0.514 | 0.797 | 0.734 | F | F |
| Combined Jacc ard Ratio | Bevacizum ab | Missense | !TP53 Xor CTNNB1 Nimp BRAF Or KRAS Xor KDR Or PIK3CA Or SMAD4 Or KIT | 1.00 | 0.47 | 0.48 | 1.00 | 0.596 | 0.476 | 0.743 | 0.734 | F | T |
| Combined Jacc ard Ratio | Bevacizum ab | Missense and Nonsense | !TP53 | 0.54 | 0.70 | 0.47 | 0.75 | 0.400 | 0.451 | 0.575 | 0.618 | F | T |
| Combined Jacc ard Ratio | Bevacizum ab | Missense and Nonsense | !TP53 Eqv !CTNNB1 | 0.54 | 0.75 | 0.51 | 0.76 | 0.463 | 0.481 | 0.644 | 0.642 | T | T |
| Combined Jacc ard Ratio | Bevacizum ab | Missense and Nonsense | !TP53 Eqv !CTNNB1 Eqv !KDR | 0.71 | 0.63 | 0.49 | 0.81 | 0.522 | 0.478 | 0.685 | 0.669 | T | T |
| Combined Jacc ard Ratio | Bevacizum ab | Missense and Nonsense | !TP53 Eqv !CTNNB1 Eqv !KDR Xor KRAS | 0.84 | 0.64 | 0.54 | 0.89 | 0.625 | 0.543 | 0.767 | 0.743 | F | T |
| Combined Jacc ard Ratio | Bevacizum ab | Missense and Nonsense | !TP53 Eqv !CTNNB1 Eqv !KDR Xor KRAS Or SMAD4 | 1.00 | 0.62 | 0.57 | 1.00 | 0.753 | 0.597 | 0.850 | 0.812 | F | T |
| Combined Jacc ard Ratio | Bevacizum ab | Missense and Nonsense | !TP53 Eqv !CTNNB1 Eqv !KDR Xor KRAS Or SMAD4 Or PIK3CA | 1.00 | 0.57 | 0.54 | 1.00 | 0.757 | 0.555 | 0.853 | 0.786 | F | F |
| Combined Jacc ard Ratio | Bevacizum ab | Missense and Nonsense | !TP53 Eqv !CTNNB1 Eqv !KDR Xor KRAS Or SMAD4 Or PIK3CA Nimp BRAF | 0.92 | 0.65 | 0.57 | 0.94 | 0.719 | 0.581 | 0.831 | 0.784 | F | T |
| Combined Jacc ard Ratio | Bevacizum ab | Missense and Nonsense | !TP53 Eqv !CTNNB1 Eqv !KDR Xor KRAS Or SMAD4 Or PIK3CA Nimp BRAF Or KIT | 1.00 | 0.50 | 0.50 | 1.00 | 0.635 | 0.503 | 0.771 | 0.752 | F | T |
| Combined Jacc ard Ratio | Bevacizum ab | Missense and Nonsense | !TP53 Eqv !CTNNB1 Eqv !KDR Xor KRAS Or SMAD4 Or PIK3CA Nimp BRAF Or KIT Nimp APC | 0.63 | 0.63 | 0.46 | 0.77 | 0.495 | 0.449 | 0.665 | 0.632 | T | T |

| | | | | | | | | | | | | | |
|---|---|---|---|---|---|---|---|---|---|---|---|---|---|
| Legend Table 25B: Max: Maximization; Ther: Therapy; Var: Variation; M1: Mean (Sensitivity - Validation); M2: Mean (Specificity - Validation); M3: Mean (Positive Predictive Value - Validation); M4 Mean (Negative Predictive Value - Validation); M5: Mean (Combined Jaccard Rate - Discovery); M6: Mean (Combined Jaccard Rate - Validation); M7: Mean(Area under the ROC-Curve - Discovery); M8: Mean(Area under the ROC-Curve - Validation); M9: Comb. Jaccard Rate - Valid Validation (F: FALSE. T: TRUE); M10: AROC - Valid Validation (F: FALSE. T: TRUE) | | | | | | | | | | | | | |

**Table 26: Functions Predicting Response to Bevacizumab in Patient Derived Xenografts of Colorectal Cancer based on Missense, Nonsense and Synonymous Sequence Variations**

| **Operands** | **Prediction Function** | | **Comment** | **S+** | **S-** | **PPV** | **NPV** | **AROC** | **CJR** | **TP** | **FP** | **TN** | **FP** |
|---|---|---|---|---|---|---|---|---|---|---|---|---|---|
| | | | | | | | | | | | | | |
| **1** | KRAS | | | 0.538 | 0.667 | 0.228 | 0.857 | 0.603 | 0.413 | 7 | 6 | 36 | 18 |
| | MET | | | 0.231 | 0.87 | 0.3 | 0.825 | 0.551 | 0.442 | 3 | 10 | 47 | 7 |
| | KDR | | | 0.308 | 0.778 | 0.25 | 0.824 | 0.543 | 0.413 | 4 | 9 | 42 | 12 |
| | PIK3CA | | | 0.154 | 0.852 | 0.200 | 0.807 | 0.503 | 0.401 | 2 | 11 | 46 | 8 |
| | | | | | | | | | | | | | |
| | !BRAF | | | 1.000 | 0.148 | 0.220 | 1.000 | 0.574 | 0.184 | 13 | 0 | 8 | 46 |
| | !SMAD4 | | | 0.923 | 0.204 | 0.218 | 0.917 | 0.563 | 0.207 | 12 | 1 | 11 | 43 |
| | !TP53 | | | 1.000 | 0.130 | 0.217 | 1.000 | 0.565 | 0.173 | 13 | 0 | 7 | 47 |
| | !APC | | | 0.923 | 0.185 | 0.214 | 0.909 | 0.554 | 0.196 | 12 | 1 | 10 | 44 |
| **2** | KRAS XOR KDR | AROC | | 0.692 | 0.667 | 0.333 | 0.900 | 0.679 | 0.456 | 9 | 4 | 36 | 18 |
| | KRAS AND !SMAD4 | CJR | | 0.538 | 0.778 | 0.368 | 0.875 | 0.658 | 0.490 | 7 | 6 | 42 | 12 |
| | MET OR KRAS | AROC | | 0.692 | 0.593 | 0.290 | 0.889 | 0.642 | 0.404 | 9 | 4 | 32 | 22 |
| | MET AND !APC | CJR | | 0.231 | 0.870 | 0.300 | 0.825 | 0.551 | 0.442 | 3 | 10 | 47 | 7 |
| | KDR XOR KRAS | AROC | | 0.692 | 0.667 | 0.333 | 0.900 | 0.679 | 0.456 | 9 | 4 | 36 | 18 |
| | KDR AND !KIT | | | 0.308 | 0.87 | 0.364 | 0.839 | 0.589 | 0.473 | 4 | 9 | 47 | 7 |
| | PIK3CA XOR KDR | AROC | | 0.462 | 0.778 | 0.333 | 0.857 | 0.620 | 0.464 | 6 | 7 | 42 | 12 |
| | !BRAF AND !APC | AROC | | 0.923 | 0.333 | 0.250 | 0.947 | 0.628 | 0.286 | 12 | 1 | 18 | 36 |
| | !BRAF AND MET | CJR | | 0.231 | 0.889 | 0.333 | 0.828 | 0.560 | 0.454 | 3 | 10 | 48 | 6 |
| | !SMAD4 AND KRAS | AROC | | 0.538 | 0.778 | 0.368 | 0.875 | 0.658 | 0.490 | 7 | 6 | 42 | 12 |
| | !TP53 AND KRAS | AROC | | 0.538 | 0.772 | 0.318 | 0.867 | 0.630 | 0.450 | 7 | 6 | 39 | 15 |
| | !TP53 AND MET | CJR | | 0.231 | 0.907 | 0.375 | 0.831 | 0.569 | 0.466 | 3 | 10 | 49 | 5 |
| | !APC AND KRAS | AROC | | 0.462 | 0.796 | 0.353 | 0.860 | 0.629 | 0.477 | 6 | 7 | 43 | 11 |
| | | | | | | | | | | | | | |
| **3** | KRAS XOR KDR AND !SMAD4 | AROC | | 0.692 | 0.759 | 0.409 | 0.911 | 0.726 | 0.527 | 9 | 4 | 41 | 13 |
| | KRAS AND !SMAD4 AND !APC | CJR | | 0.462 | 0.87 | 0.462 | 0.870 | 0.666 | 0.535 | 6 | 7 | 47 | 7 |
| | MET OR KRAS AND !SMAD4 | AROC | | 0.692 | 0.704 | 0.360 | 0.905 | 0.698 | 0.483 | 9 | 4 | 38 | 16 |
| | MET AND !APC AND !KIT | CJR | | 0.231 | 0.944 | 0.500 | 0.836 | 0.588 | 0.492 | 3 | 10 | 51 | 3 |
| | KDR XOR KRAS AND !SMAD4 | AROC | | 0.692 | 0.759 | 0.409 | 0.911 | 0.726 | 0.527 | 9 | 4 | 41 | 13 |
| | KDR AND !KIT AND !PIK3CA | AROC | | 0.308 | 0.926 | 0.500 | 0.847 | 0.617 | 0.514 | 4 | 9 | 50 | 4 |
| | PIK3CA XOR KDR XOR KIT | AROC | | 0.462 | 0.778 | 0.333 | 0.857 | 0.620 | 0.464 | 6 | 7 | 42 | 12 |
| | !BRAF AND !APC XOR PIK3CA | AROC | | 0.923 | 0.444 | 0.286 | 0.960 | 0.684 | 0.358 | 12 | 1 | 24 | 30 |
| | !BRAF AND MET OR KRAS | CJR 2 x. AROC | | 0.692 | 0.611 | 0.300 | 0.892 | 0.652 | 0.417 | 9 | 4 | 33 | 21 |
| | !SMAD4 AND KRAS XOR KDR | AROC | | 0.692 | 0.741 | 0.391 | 0.909 | 0.717 | 0.511 | 9 | 4 | 40 | 14 |
| | !TP53 AND KRAS XOR KDR | AROC | | 0.692 | 0.722 | 0.375 | 0.907 | 0.707 | 0.497 | 9 | 4 | 39 | 15 |
| | !TP53 AND MET OR KRAS | CJR 2x. AROC | | 0.692 | 0.611 | 0.300 | 0.892 | 0.652 | 0.417 | 9 | 4 | 33 | 21 |
| | !APC AND KRAS AND !SMAD4 | AROC | | 0.462 | 0.870 | 0.462 | 0.870 | 0.666 | 0.535 | 6 | 7 | 47 | 7 |
| | | | | | | | | | | | | | |
| **4** | **KRAS XOR KDR AND !SMAD4 AND !BRAF** | **AROC** | | **0.692** | **0.796** | **0.450** | **0.915** | **0.744** | **0.558** | **9** | **4** | **43** | **11** |
| | KRAS AND !SMAD4 AND !APC AND !TP53 | CJR | Specificity optimized signature | 0.462 | 0.889 | 0.500 | 0.873 | 0.673 | 0.551 | 6 | 7 | 48 | 6 |
| | MET OR KRAS AND !SMAD4 OR KDR | AROC | | 0.846 | 0.593 | 0.333 | 0.941 | 0.719 | 0.443 | 11 | 2 | 32 | 22 |
| | MET AND !APC AND !KIT OR KRAS | CJR 3x. AROC | | 0.692 | 0.630 | 0.310 | 0.895 | 0.661 | 0.429 | 9 | 4 | 34 | 20 |
| | **KDR XOR KRAS AND !SMAD4 AND !BRAF** | AROC | | 0.692 | 0.796 | 0.450 | 0.915 | 0.744 | 0.558 | 9 | 4 | 43 | 11 |
| | KDR AND !KIT AND !PIK3CA AND !APC | CJR | | 0.308 | 0.944 | 0.571 | 0.850 | 0.626 | 0.530 | 4 | 9 | 51 | 3 |
| | PIK3CA XOR KDR XOR KIT AND !BRAF | AROC | | 0.538 | 0.815 | 0.412 | 0.88 | 0.677 | 0.519 | 7 | 6 | 44 | 10 |
| | !BRAF AND !APC XOR PIK3CA AND SMAD4 | AROC | | 0.846 | 0.574 | 0.324 | 0.939 | 0.710 | 0.430 | 11 | 2 | 31 | 23 |
| | !BRAF AND MET OR KRAS AND | CJR 2x | | 0.692 | 0.722 | 0.375 | 0.907 | 0.707 | 0.497 | 9 | 4 | 39 | 15 |
| | !SMAD4 | AROC 2x | | | | | | | | | | | |
| | !SMAD4 AND KRAS XOR KDR AND !BRAF | AROC | | 0.692 | 0.778 | 0.429 | 0.913 | 0.735 | 0.542 | 9 | 4 | 42 | 12 |
| | !TP53 AND KRAS XOR KDR AND SMAD4 | AROC | | 0.692 | 0.778 | 0.429 | 0.913 | 0.735 | 0.542 | 9 | 4 | 42 | 12 |
| | !TP53 AND MET OR KRAS AND !SMAD4 | CJR 2x AROC 2x | | 0.692 | 0.722 | 0.375 | 0.907 | 0.707 | 0.497 | 9 | 4 | 39 | 15 |
| | !APC AND KRAS AND !SMAD4 OR KDR | AROC | | 0.692 | 0.685 | 0.346 | 0.902 | 0.689 | 0.469 | 9 | 4 | 37 | 17 |
| **5** | **KRAS XOR KDR AND !SMAD4 AND !BRAF AND !TP53** | **AROC** | **best signature** | **0.692** | **0.833** | **0.500** | **0.918** | **0.763** | **0.592** | **9** | **4** | **45** | **9** |
| | KRAS AND !SMAD4 AND !APC AND !TP53 AND !BRAF | CJR | | 0.462 | 0.889 | 0.500 | 0.873 | 0.675 | 0.551 | 6 | 7 | 48 | 6 |
| | MET OR KRAS AND !SMAD4 OR KDR AND !BRAF | AROC | | 0.846 | 0.648 | 0.367 | 0.946 | 0.747 | 0.484 | 11 | 2 | 32 | 22 |
| | MET AND !APC AND !KIT OR KRAS AND !SMAD4 | CJR 3x AROC 2x | | 0.692 | 0.741 | 0.391 | 0.909 | 0.717 | 0.511 | 9 | 4 | 40 | 14 |
| | **KDR XOR KRAS AND !SMAD4 AND !BRAF AND !TP53** | AROC | | 0.692 | 0.833 | 0.500 | 0.918 | 0.763 | 0.592 | 9 | 4 | 45 | 9 |
| | KDR AND !KIT AND !PIK3CA AND !APC AND !BRAF | CJR | Specificty optimized signature | 0.308 | 0.963 | 0.667 | 0.852 | 0.635 | 0.546 | 4 | 9 | 52 | 2 |
| | PIK3CA XOR KDR XOR KIT AND !BRAF AND !SMAD4 | AROC | | 0.538 | 0.833 | 0.438 | 0.882 | 0.686 | 0.534 | 7 | 6 | 45 | 9 |
| | !BRAF AND !APC XOR PIK3CA AND SMAD4 XOR ATM | AROC | | 0.923 | 0.537 | 0.324 | 0.967 | 0.730 | 0.422 | 12 | 1 | 29 | 25 |
| | !BRAF AND MET OR KRAS AND !SMAD4 OR KDR | CJR 2x. AROC 3x | | 0.846 | 0.611 | 0.344 | 0.943 | 0.729 | 0.456 | 11 | 2 | 33 | 21 |
| | **!SMAD4 AND KRAS XOR KDR AND !BRAF AND !TP53** | **AROC** | second best signature | **0.692** | **0.815** | **0.474** | **0.917** | **0.754** | **0.575** | **9** | **4** | **44** | **10** |
| | **!TP53 AND KRAS XOR KDR AND !SMAD4 AND !BRAF** | **AROC** | | **0.692** | **0.815** | **0.474** | **0.917** | **0.754** | **0.575** | **9** | **4** | **44** | **10** |
| | !TP53 AND MET OR KRAS AND !SMAD4 OR KDR | CJR 2x. AROC 3x | | 0.846 | 0.593 | 0.333 | 0.941 | 0.719 | 0.443 | 11 | 2 | 32 | 22 |
| | !APC AND KRAS AND !SMAD4 OR KDR AND !BRAF | AROC | | 0.692 | 0.722 | 0.375 | 0.907 | 0.707 | 0.497 | 9 | 4 | 39 | 15 |
| **6** | KRAS XOR KDR AND !SMAD4 AND !BRAF AND !TP53 OR MET | AROC | | 0.769 | 0.741 | 0.417 | 0.930 | 0.755 | 0.536 | 10 | 3 | 40 | 14 |
| | KRAS AND !SMAD4 AND !APC AND !TP53 AND !BRAF AND !KDR | CJR | | 0.385 | 0.926 | 0.556 | 0.862 | 0.655 | 0.550 | 5 | 8 | 50 | 4 |
| | **MET OR KRAS AND !SMAD4 OR KDR AND !BRAF AND !TP53** | **AROC** | Sensitivity optimized signature | **0.846** | **0.685** | **0.393** | **0.949** | **0.766** | **0.514** | **11** | **2** | **37** | **17** |
| | MET AND !APC AND !KIT OR KRAS AND !SMAD4 OR KDR | CJR3x. AROC 2x | | 0.846 | 0.630 | 0.355 | 0.944 | 0.738 | 0.470 | 11 | 2 | 34 | 20 |
| | KDR XOR KRAS AND !SMAD4 AND !BRAF AND !TP53 OR MET | AROC | | 0.769 | 0.741 | 0.417 | 0.930 | 0.755 | 0.536 | 10 | 3 | 40 | 14 |
| | KDR AND !KIT AND !PIK3CA AND !APC AND !BRAF AND !ATM | CJR | | 0.308 | 0.963 | 0.667 | 0.852 | 0.635 | 0.546 | 4 | 9 | 52 | 2 |
| | PIK3CA XOR KDR XOR KIT AND !BRAF AND !SMAD4 AND !TP53 | AROC | | 0.538 | 0.833 | 0.438 | 0.882 | 0.686 | 0.534 | 7 | 6 | 45 | 9 |
| | !BRAF AND !APC XOR PIK3CA AND SMAD4 XOR ATM AND KIT | AROC | | 0.846 | 0.63 | 0.355 | 0.944 | 0.738 | 0.470 | 11 | 2 | 34 | 20 |
| | !BRAF AND MET OR KRAS AND !SMAD4 OR KDR AND !TP53 | CJR 2x. AROC 4x | | 0.846 | 0.648 | 0.367 | 0.946 | 0.747 | 0.484 | 11 | 2 | 35 | 19 |
| | !SMAD4 AND KRAS XOR KDR AND !BRAF AND !TP53 OR MET | AROC | | 0.769 | 0.722 | 0.400 | 0.929 | 0.746 | 0.521 | 10 | 3 | 39 | 15 |
| | !TP53 AND KRAS XOR KDR AND !SMAD4 AND !BRAF OR MET | AROC | | 0.769 | 0.741 | 0.417 | 0.93 | 0.755 | 0.536 | 10 | 3 | 40 | 14 |
| | !TP53 AND MET OR KRAS AND !SMAD4 OR KDR AND !BRAF | CJR 2x. AROC 4x | | 0.846 | 0.648 | 0.367 | 0.946 | 0.747 | 0.484 | 11 | 2 | 35 | 19 |
| | !APC AND KRAS AND !SMAD4 OR KDR AND !BRAF AND !TP53 | AROC | | 0.692 | 0.759 | 0.409 | 0.911 | 0.726 | 0.527 | 9 | 4 | 41 | 13 |
| **7** | **KRAS XOR KDR AND !SMAD4 AND !BRAF AND !TP53 OR MET AND !APC** | **AROC** | | **0.692** | **0.815** | **0.474** | **0.917** | **0.754** | **0.575** | **9** | **4** | **44** | **10** |
| **7** | KRAS AND !SMAD4 AND !APC AND !TP53 AND !BRAF AND !KDR AND !MET | CJR | | 0.308 | 0.944 | 0.571 | 0.85 | 0.626 | 0.53 | 4 | 9 | 51 | 3 |
| | MET OR KRAS AND !SMAD4 OR KDR AND !BRAF AND !TP53 | | | | | | | | | | | | |
| **8** | MET AND !APC AND !KIT OR KRAS AND !SMAD4 OR KDR AND !BRAF AND !TP53 | CJR 3x. AROC 4x | | 0.846 | 0.704 | 0.407 | 0.95 | 0.775 | 0.529 | 11 | 2 | 38 | 16 |

| | | | | | | | | | | | | | |
|---|---|---|---|---|---|---|---|---|---|---|---|---|---|
| Legend Table 26: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | | | | |

**Table 27: Functions Predicting Response (T/C <25) to Bevacizumab in Patient Derived Xenografts of Colorectal Cancer based on Missense, Nonsense and Synonymous Sequence Variations**

| **Prediction Function** | **S+** | **S-** | **PPV** | **NPV** | **AROC** | **CJR** | **TP** | **FP** | **TN** | **FP** |
|---|---|---|---|---|---|---|---|---|---|---|
| KDR XOR Plk3CA XOR KIT AND !BRAF AND !SMAD4 | 0.636 | 0.839 | 0.438 | 0.922 | 0.738 | 0.567 | 7 | **4** | 47 | 9 |

| | | | | | | | | | | |
|---|---|---|---|---|---|---|---|---|---|---|
| Legend Table 27: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | |

**Table 28: Functions Predicting Response (T/C <35) to Bevacizumab in Patient Derived Xenografts of Colorectal Cancer based on Missense, Nonsense and Synonymous Sequence Variations**

| **Prediction Function** | **S+** | **S-** | **PPV** | **NPV** | **AROC** | **CJR** | **TP** | **FP** | **TN** | **FP** |
|---|---|---|---|---|---|---|---|---|---|---|
| !TP53 AND !BRAF AND !APC XOR Plk3CA AND !KIT | 0.789 | 0.563 | 0.417 | 0.871 | 0.676 | 0.447 | 15 | 4 | 27 | 21 |
| PIK3CA XOR !APC XOR KIT AND !TP53 AND !BRAF | 0.842 | 0.563 | 0.432 | 0.9 | 0.702 | 0.465 | 16 | 3 | 27 | 21 |

| | | | | | | | | | | |
|---|---|---|---|---|---|---|---|---|---|---|
| Legend Table 28: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | |

**Table 29: Functions Predicting Response to Bevacizumab + Chemotherapy in Colorectal Cancer UICC Stage IV based on Missense and Nonsense Sequence Variations With Sensitivity > 70%**

| Operands | | **S+** | **S-** | **PPV** | **NPV** | **AROC** | **CJR** | **TP** | **FP** | **TN** | **FP** |
|---|---|---|---|---|---|---|---|---|---|---|---|
| 4 | !TP53 OR KIT AND !CTNNB1 AND !MET | 0.727 | 0.773 | 0.615 | 0.850 | 0.750 | 0.590 | 8 | 3 | 17 | 5 |
| 5 | !TP53 OR KIT AND !CTNNB1 AND !MET OR SMAD4 | 0.818 | 0.727 | 0.600 | 0.889 | 0.773 | 0.598 | 9 | 2 | 16 | 6 |
| 6 | !CTNNB1 AND !TP53 AND !KDR AND !MET OR PIK3CA OR SMAD4 | 0.636 | 0.864 | 0.700 | 0.826 | 0.750 | 0.615 | 7 | 4 | 19 | 3 |

| | | | | | | | | | | | |
|---|---|---|---|---|---|---|---|---|---|---|---|
| Legend Table 29: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | | |

**Table 30: Functions Predicting Response to Bevacizumab + Chemotherapy in Colorectal Cancer UICC Stage IV based on Missense and Nonsense Sequence Variation With Sensitivity > 70%**

| Operands | **Prediction Funcion** | Comment | **S+** | **S-** | **PPV** | **NPV** | **AROC** | **CJR** | **TP** | **FP** | **TN** | **FP** |
|---|---|---|---|---|---|---|---|---|---|---|---|---|
| 3 | **!CTNNB1 AND !TP53 OR KIT** | | **0.727** | **0.682** | **0.553** | **0.833** | **0.705** | **0.522** | **8** | **3** | **15** | **7** |
| 4 | !CTNNB1 AND !TP53 OR KIT OR KRAS | with max sensitivity | 0.909 | 0.455 | 0.455 | 0.909 | 0.682 | 0.435 | 10 | 1 | 10 | 12 |
| | **!PIK3CA XOR KRAS AND !TP53 OR KIT** | balanced sensitivity and specificity | **0.727** | **0.727** | **0.571** | **0.842** | **0.727** | **0.555** | **8** | **3** | **16** | **6** |
| | **!PIK3CA XOR KRAS AND !TP53 XOR KIT** | with more specificity | **0.636** | **0.818** | **0.636** | **0.818** | **0.727** | **0.579** | **7** | **4** | **18** | **4** |

| | | | | | | | | | | | | |
|---|---|---|---|---|---|---|---|---|---|---|---|---|
| Legend Table 30: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | | | |

**Table 31: Functions Predicting Response (T/C <30) to Bevacizumab in Patient Derived Xenografts of Colorectal Cancer based on Missense, Nonsense and Synonymous Sequence Variations**

| | **Prediction Funcion** | Comment | **S+** | **S-** | **PPV** | **NPV** | **AROC** | **CJR** | **TP** | **FP** | **TN** | **FP** |
|---|---|---|---|---|---|---|---|---|---|---|---|---|
| 5 | **KRAS XOR KDR AND !SMAD4 AND !BRAF AND !TP53** | | **0.692** | **0.833** | **0.500** | **0.918** | **0.763** | **0.592** | **9** | **4** | **45** | **9** |
| | **KDR AND !KIT AND !PIK3CA AND !APC AND !BRAF** | with max specif icity | 0.308 | 0.963 | 0.667 | 0.852 | 0.635 | 0.546 | 4 | 9 | 52 | 2 |
| 6 | **MET OR KRAS AND !SMAD4 OR KDR AND !BRAF AND !TP53** | with max sensit ivity | **0.846** | **0.685** | **0.393** | **0.949** | **0.766** | **0.514** | **11** | **2** | **37** | **17** |

| | | | | | | | | | | | | |
|---|---|---|---|---|---|---|---|---|---|---|---|---|
| Legend Table 31: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | | | |

**Table 32: Functions Predicting Response (T/C <35) to Bevacizumab in Patient Derived Xenografts of Colorectal Cancer based on Missense, Nonsense and Synonymous Sequence Variations**

| **Prediction Funcion** | **S+** | **S-** | **PPV** | **NPV** | **AROC** | **CJR** | **TP** | **FP** | **TN** | **FP** |
|---|---|---|---|---|---|---|---|---|---|---|
| **PIK3CA XOR !APC XOR KIT AND !TP53 AND !BRAF** | **0.842** | **0.563** | **0.432** | **0.900** | **0.702** | **0.465** | **16** | **3** | **27** | **21** |

| | | | | | | | | | | |
|---|---|---|---|---|---|---|---|---|---|---|
| Legend Table 32: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | |

**Table 33: Functions Predicting Response (T/C <25) to Bevacizumab in Patient Derived Xenografts of Colorectal Cancer based on Missense, Nonsense and Svnonvmous Sequence Variations**

| **Prediction Function** | **S+** | **S-** | **PPV** | **NPV** | **AROC** | **CJR** | **TP** | **FP** | **TN** | **FP** |
|---|---|---|---|---|---|---|---|---|---|---|
| **KDR XOR PIK3CA XOR KIT AND !BRAF AND !SMAD4** | **0.636** | **0.839** | **0.438** | **0.922** | **0.738** | **0.567** | **7** | **4** | **47** | **9** |

| | | | | | | | | | | |
|---|---|---|---|---|---|---|---|---|---|---|
| Legend Table 33: S+ = Sensitivity, S- = Specificity, PPV = Positive Predictive Value, NPV = Negative Predictive Value, AROC = Area under the receiver operating characteristic curve; CJR = combined Jaccard Ratio, TP = Count of true positives, FP = Count of false positives, TN = Count of true negatives, FP = Count of false positives. | | | | | | | | | | |

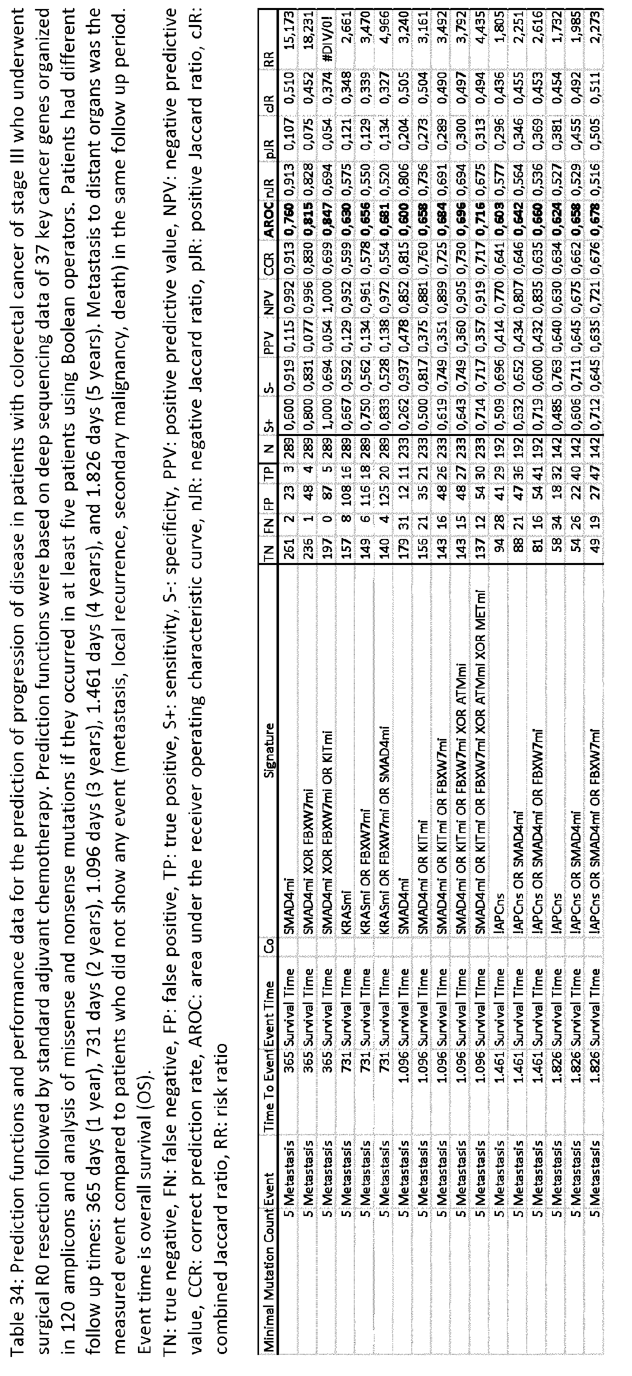

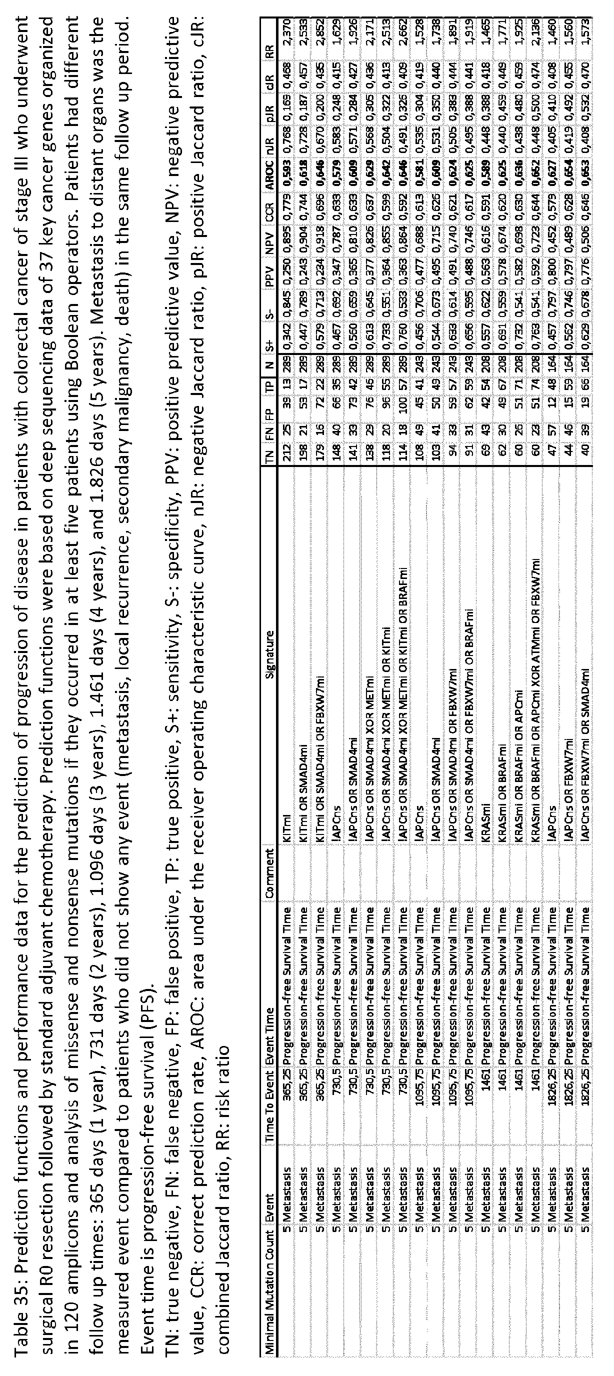

## Claims

1. A method for predicting a manifestation of an outcome measure of a cancer patient based on a tumor DNA containing tissue sample from the cancer patient, comprising:
- Determining an existence of a sequence variation within at least two gene segments of the tumor DNA as:
- Present, if at least one significant sequence variation can be determined, or as
- Absent, if no significant sequence variation can be determined;
wherein the at least two gene segments of the tumor DNA are associated with the outcome measure of the patient; and
wherein the sequence variation comprises a silent or synonymous sequence variation;
- Combining the existence of sequence variations of the at least two gene segments using a logical operation (prediction function), thereby aggregating the biological information contained in each sequence variation status, and
- Predicting based on the results of the logical operation the manifestation of an outcome measure of the patient,
wherein the prediction function comprises sequence variations or its negation as variables and a logical operator combining the variables, wherein the logical operator is chosen from the group consisting of: negation of conjunction (Nand), negation of disjunction (Nor), equivalence (Eqv), negation of equivalence (exclusive disjunction, Xor), material implication (Imp), and negation of material implication (Nimp)
wherein the sequence variation comprises analyzing sequence variations that do not alter the protein sequence of the encoded protein.

2. The method of claim 1, wherein predicting the manifestation of an outcome measure of the cancer patient comprises
- The prediction of progression of disease of a cancer of the patient, such as the local recurrence of the cancer, the occurrence of secondary malignancy, or the occurrence of metastasis, or
- The prediction of the outcome measure response to therapy with the manifestation response and nonresponse of the patient to a cancer treatment with a drug, such as adjuvant chemotherapy, neo-adjuvant chemotherapy, palliative chemotherapy or the use of targeted drugs in combination with a chemotherapy or radio-chemotherapy.

3. The method of claim 1 or 2, wherein the tumor DNA containing tissue sample is tumor tissue, sputum, stool, urine, bronchial lavage, cerebro-spinal fluid, blood, plasma, or serum.

4. The method of claim 1 to 3, wherein the prediction of the sequence variation comprises analyzing
- Sequence variations that alter the protein sequence.

5. The method of claim 4, wherein the sequence variation that alter the protein sequence comprise missense, nonsense, splicing, deletion, insertion, or frame shift variation.

6. The method of claims 1 to 5, wherein standard logic rules of Boolean algebra apply, in particular the law of the excluded middle, double negative elimination, law of noncontradiction, principle of explosion, monotonicity of entailment, idempotency of entailment, commutativity of conjunction, and De Morgan duality.

7. The method of claims 1 to 6, wherein the prediction function is optimized for at least one of the following: sensitivity, specificity, positive predictive value, negative predictive value, correct classification rate, miss-classification rate, area under the receiver operating characteristic curve (AROC), odds-ratio, pappa, negative Jaccard Ratio, positive Jaccard ratio, combined Jaccard ratio or cost, wherein area under the receiver operating characteristic curve (AROC) and the combined Jaccard Ratio are preferred.

8. A method for determining a function that allows for the prediction of the manifestation of an outcome measure of a cancer patient based on a tumor DNA containing tissue sample from the patient, comprising:
- Determining the DNA sequence of segments of at least two gene segments in a group of cancer patients which is comprised of patients with at least two disjunctive manifestations of the outcome measure;
- Determining the sequence variation of the at least two gene segments of the tumor DNA as:
- Present, if at least one significant sequence variation can be determined, or as
- Absent, if no significant sequence variation can be determined;
wherein the sequence variation comprises a silent or synonymous sequence variation;
- Combining the sequence variation statuses of the at least two gene segments using a logical operator, thereby aggregating the biological information contained in each sequence variation status and generating a prediction function, such that patients with one specific manifestation of the outcome measure are distinguishable from patients with another disjunctive manifestation of the outcome measure,
wherein the prediction function comprises sequence variations or its negation as variables and a logical operator combining the variables, wherein the logical operator is chosen from the group consisting of: negation of conjunction (Nand), negation of disjunction (Nor), equivalence (Eqv), negation of equivalence (exclusive disjunction, Xor), material implication (Imp), and negation of material implication (Nimp).

9. The method of claim 8, wherein predicting the outcome measure of the cancer patient comprises
- The prediction of progression of disease of a cancer of the patient, such as the local recurrence of the cancer, the occurrence of secondary malignancy, or the occurrence of metastasis, or
- The prediction of the response vs. nonresponse of the patient to a cancer treatment with a drug, such as adjuvant chemotherapy, neo-adjuvant chemotherapy, palliative chemotherapy or the use of targeted drugs in combination with a chemotherapy or radio-chemotherapy.

10. The method of claim 8 or 9, wherein the tumor DNA containing tissue sample is tumor tissue, sputum, stool, urine, bronchial lavage, cerebro-spinal fluid, blood, plasma, or serum.

11. The method of claims 8 to 10, wherein the prediction of the sequence variation comprises analyzing
- Sequence variations that alter the protein sequence.

12. The method of claims 8 to 11, wherein the sequence variation that alter the protein sequence comprise missense, nonsense, splicing, deletion, insertion, or frame shift.

13. The method of claims 8 to 12, wherein the relative frequency of sequence variations within a gene of the at least two gene segments is at least 1 %, preferably at least 3 % in a given patient population.

14. A computer program, adapted to perform the method of claims 8 to 13, in particular the steps of
- Determining an existence of a sequence variation within segments of at least two gene segments of the tumor DNA as:
- Present, if at least one sequence variation can be determined, or as
- Absent, if no sequence variation can be determined;
wherein the at least two gene segments of the tumor DNA are associated with the outcome measure of the patient; and
wherein the sequence variation comprises a silent or synonymous sequence variation,
- Combining the existence of significant sequence variations of the at least two gene segments using a logical operation (prediction function), thereby aggregating the biological information contained in each sequence variation status, and
- Predicting based on the results of the logical operation the manifestation of the outcome measure of the patient,
wherein the prediction function comprises sequence variations or its negation as variables and a logical operator combining the variables, wherein the logical operator is chosen from the group consisting of: negation of conjunction (Nand), negation of disjunction (Nor), equivalence (Eqv), negation of equivalence (exclusive disjunction, Xor), material implication (Imp), and negation of material implication (Nimp).

## Patentansprüche

1. Verfahren zum Vorhersagen einer Manifestation eines Zielkriteriums eines Krebspatienten basierend auf einer Tumor-DNA beinhaltenden Gewebeprobe von dem Krebspatienten, aufweisend:
- Bestimmen eines Vorliegens einer Sequenzvariation innerhalb von mindestens zwei Gensegmenten der Tumor-DNA als:
- Vorhanden, wenn mindestens eine signifikante Sequenzvariation bestimmt werden kann oder als
- Fehlend, wenn keine signifikante Sequenzvariation bestimmt werden kann;
wobei die mindestens zwei Gensegmente der Tumor-DNA mit dem Zielkriterium des Patienten assoziiert sind; und
wobei die Sequenzvariation eine stille oder synonyme Sequenzvariation aufweist;
- Kombinieren des Vorliegens von Sequenzvariationen der mindestens zwei Gensegmente mittels einer logischen Operation (Vorhersagefunktion), wobei die in jedem Sequenzvariationsstatus enthaltende biologische Informationen aggregiert wird und
- Vorhersagen der Manifestation eines Zielkriteriums des Patienten basierend auf den Ergebnissen der logischen Operation;
wobei die Vorhersagefunktion Sequenzvariationen oder deren Negation als Variable und einen logischen Operator aufweist, der die Variablen kombiniert, wobei der logische Operator ausgewählt ist aus der Gruppe bestehend aus: Negation der Konjunktion (Nand), Negation der Disjunktion (Nor), Äquivalenz (Eqv), Negation der Äquivalenz (ausschließende Disjunktion, Xor), materiale Implikation (Imp) und Negation der materialen Implikation (Nimp);
wobei die Sequenzvariation ein Analysieren der Sequenzvariationen umfasst, die die Proteinsequenz des kodierten Proteins nicht ändert.

2. Verfahren nach Anspruch 1, wobei die Vorhersagen der Manifestation eines Zielkriteriums des Krebspatienten umfasst:
- die Vorhersage der Progression der Krankheit des Krebspatienten, so wie das lokale Wiederauftreten des Krebses, das Auftreten von sekundärer Malignität oder das Auftreten von Metastasen oder
- die Vorhersage des Ansprechens des Zielkriteriums auf Therapie durch das Ansprechen oder Nicht-Ansprechen der Manifestation des Patienten auf eine Krebsbehandlung mit einem Medikament, etwa adjuvante Chemotherapie, neo-adjuvante Chemotherapie, palliative Chemotherapie oder der Nutzung von zielgerichteten Medikamenten in Kombination mit einer Chemotherapie oder Radio-Chemotherapie.

3. Verfahren nach Anspruch 1 oder 2, wobei die Tumor-DNA beinhaltende Gewebeprobe Tumorgewebe, Sputum, Stuhl, Urin, Bronchiallavage, Zerebrospinalflüssigkeit, Blut, Plasma oder Serum ist.

4. Verfahren nach Anspruch 1 bis 3, wobei die Vorhersage der Sequenzvariation das Analysieren der Sequenzvariationen umfasst, die die Proteinsequenz ändern.

5. Verfahren nach Anspruch 4, wobei die Sequenzvariation, die die Proteinsequenz ändert Missens, Nonsens, Spleißen, Deletion, Insertion oder Frameshift Variationen umfasst.

6. Verfahren nach Anspruch 1 bis 5, wobei Standard-Logikregeln der Booleschen Algebra angewendet werden, insbesondere der Satz vom ausgeschlossenen Dritten, doppelte Negations-Beseitigung, Satz vom ausgeschlossenen Widerspruch, ex falso quodlibet, die Monotonie einer notwendigen Bedingung, die Idempotenz einer Notwendigen Bedingung, die Kommutativität der Konjunktion und die De Morgan Dualität.

7. Verfahren nach Anspruch 1 bis 6, wobei die Vorhersagefunktion optimiert ist für mindestens eine der Folgenden: Sensitivität, Spezifizität, positiver Vorhersagewert, negativer Vorhersagewert, korrekte Klassifizierungsrate, Falsch-Klassifizierungsrate, Fläche unter der Receiver-Operating-Characteristic-Kurve (AROC), Odds-Ratio, Pappa, negativer Jaccard Koeffizient, positiver Jaccard Koeffizient, kombinierter Jaccard Koeffizient oder Kosten, wobei die Fläche unter der Receiver-Operating-Characteristic-Kurve (AROC) und der kombinierte Jaccard Koeffizient bevorzugt sind.

8. Verfahren zur Bestimmung einer Funktion, die die Vorhersage der Manifestation eines Zielkriteriums eines Krebspatienten basierend auf einer Tumor-DNA enthaltenden Gewebeprobe des Patienten erlaubt, aufweisend:
- Bestimmen der DNA-Sequenz von Segmenten mindestens zweier Gensegmenten in einer Gruppe von Krebspatienten, welche Patienten mit mindestens zwei disjunktiven Manifestationen des Zielkriteriums umfassen;
- Bestimmen der Sequenzvariation der mindestens zwei Gensegmente der Tumor-DNA als:
- Vorhanden, wenn mindestens eine signifikante Sequenzvariation bestimmt werden kann oder als
- Fehlend, wenn keine signifikante Sequenzvariation bestimmt werden kann;
wobei die Sequenzvariation eine stille oder synonyme Sequenzvariation aufweist;
- Kombination der Sequenzvariationsstatus der mindestens zwei Gensegmente mittels eines logischen Operators, wobei die in jedem Sequenzvariationsstatus enthaltende biologische Informationen aggregiert wird und Generieren eine Vorhersagefunktion, so dass Patienten mit einer spezifischen Manifestation der Zielkriterien unterscheidbar sind von Patienten mit einer anderen disjunktiven Manifestation des Zielkriteriums,
wobei die Vorhersagefunktion Sequenzvariationen oder deren Negation als Variable und einen logischen Operator aufweist, der die Variablen kombiniert, wobei der logische Operator ausgewählt ist aus der Gruppe bestehend aus: Negation der Konjunktion (Nand), Negation der Disjunktion (Nor), Äquivalenz (Eqv), Negation der Äquivalenz (ausschließende Disjunktion, Xor), materiale Implikation (Imp) und Negation der materialen Implikation (Nimp).

9. Verfahren nach Anspruch 8, wobei die Vorhersage des Zielkriteriums des Krebspatienten umfasst:
- die Vorhersage der Progression der Krankheit des Krebspatienten, so wie das lokale Wiederauftreten des Krebses, das Auftreten von sekundärer Malignität oder das Auftreten von Metastasen oder
- die Vorhersage des Ansprechens des Zielkriteriums auf Therapie durch das Ansprechen oder Nicht-Ansprechen der Manifestation des Patienten auf eine Krebsbehandlung mit einem Medikament, etwa adjuvante Chemotherapie, neo-adjuvante Chemotherapie, palliative Chemotherapie oder der Nutzung von zielgerichteten Medikamenten in Kombination mit einer Chemotherapie oder Radio-Chemotherapie.

10. Verfahren nach Anspruch 8 oder 9, wobei die Tumor-DNA enthaltene Gewebeprobe Tumorgewebe, Sputum, Stuhl, Urin, Bronchiallavage, Zerebrospinalflüssigkeit, Blut, Plasma oder Serum ist.

11. Verfahren nach Anspruch 8 bis 10, wobei die Vorhersage der Sequenzvariation das Analysieren der Sequenzvariationen umfasst, die die Proteinsequenz ändern.

12. Verfahren nach Anspruch 8 bis 11, wobei die Sequenzvariation, die die Proteinsequenz ändert Missens, Nonsens, Spleißen, Deletion, Insertion oder Frameshift Variationen umfasst.

13. Verfahren nach Anspruch 8 bis 12, wobei die relative Frequenz der Sequenzvariationen in einem Gen der mindestens zwei Gensegmente mindestens 1 %, bevorzugt mindestens 3 % in einer gegebenen Patientenpopulation beträgt.

14. Computerprogramm, adaptiert das Verfahren nach Anspruch 8 bis 13 auszuführen, insbesondere die Schritte:
- Bestimmen eines Vorliegens einer Sequenzvariation innerhalb von Segmenten mindestens zweier Gensegmenten der Tumor-DNA als:
- Vorhanden, wenn mindestens eine signifikante Sequenzvariation bestimmt werden kann oder als
- Fehlend, wenn keine signifikante Sequenzvariation bestimmt werden kann;
wobei die mindestens zwei Gensegmente der Tumor-DNA mit dem Zielkriterium des Patienten assoziiert sind und
wobei die Sequenzvariation eine stille oder synonyme Sequenzvariation aufweist;
- Kombinieren des Vorliegens von Sequenzvariationen der mindestens zwei Gensegmenten mittels einer logischen Operation (Vorhersagefunktion), wobei die in jedem Sequenzvariationsstatus enthaltende biologische Informationen aggregiert wird und
- Vorhersagen der Manifestation eines Zielkriteriums des Patienten basierend auf den Ergebnissen der logischen Operation,
wobei die Vorhersagefunktion Sequenzvariationen oder deren Negation als Variable und einen logischen Operator aufweist, der die Variablen kombiniert, wobei der logische Operator ausgewählt ist aus der Gruppe bestehend aus: Negation der Konjunktion (Nand), Negation der Disjunktion (Nor), Äquivalenz (Eqv), Negation der Äquivalenz (ausschließende Disjunktion, Xor), materiale Implikation (Imp) und Negation der materialen Implikation (Nimp).

## Revendications

1. Procédé de prédiction d'une manifestation d'une mesure de résultat d'un patient cancéreux sur la base d'un échantillon de tissu contenant de l'ADN tumoral provenant du patient cancéreux, comprenant :
- la détermination de l'existence d'une variation de séquence dans au moins deux segments de gène de l'ADN tumoral comme étant :
- présente, si au moins une variation de séquence significative peut être déterminée, ou comme étant
- absente, si aucune variation de séquence significative ne peut être déterminée ;
dans lequel les au moins deux segments de gène de l'ADN tumoral sont associés à la mesure de résultat du patient ; et
dans lequel la variation de séquence comprend une variation de séquence silencieuse ou synonyme ;
- la combinaison de l'existence de variations de séquence des au moins deux segments de gène en utilisant une opération logique (fonction de prédiction), de façon à agréger les informations biologiques contenues dans chaque état de variation de séquence, et
- la prédiction, sur la base des résultats de l'opération logique, de la manifestation d'une mesure de résultat du patient,
dans lequel la fonction de prédiction comprend des variations de séquence ou leur négation en tant que variables et un opérateur logique combinant les variables, dans lequel l'opérateur logique est choisi dans le groupe constitué de : négation de conjonction (Nand), négation de disjonction (Nor), équivalence (Eqv), négation d'équivalence (disjonction exclusive, Xor), implication matérielle (Imp), et négation d'implication matérielle (Nimp),
dans lequel la variation de séquence comprend l'analyse de variations de séquence qui ne modifient pas la séquence de protéine de la protéine codée.

2. Procédé selon la revendication 1, dans lequel la prédiction de la manifestation d'une mesure de résultat du patient cancéreux comprend
- la prédiction de la progression de maladie d'un cancer du patient, telle que la récidive locale du cancer, l'apparition d'une tumeur maligne secondaire, ou l'apparition de métastases, ou
- la prédiction de la mesure de résultat de réponse au traitement avec la réponse et non-réponse de manifestation du patient à un traitement anticancéreux avec un médicament, tel qu'une chimiothérapie adjuvante, une chimiothérapie néo-adjuvante, une chimiothérapie palliative ou l'utilisation de médicaments ciblés en combinaison avec une chimiothérapie ou une radio-chimiothérapie.

3. Procédé selon la revendication 1 ou 2, dans lequel l'échantillon de tissu contenant de l'ADN tumoral est du tissu tumoral, de l'expectoration, des selles, de l'urine, un lavage bronchique, du liquide céphalo-rachidien, du sang, du plasma ou du sérum.

4. Procédé selon la revendication 1 à 3, dans lequel la prédiction de la variation de séquence comprend l'analyse de
- variations de séquence qui modifient la séquence protéique.

5. Procédé selon la revendication 4, dans lequel les variations de séquence qui modifient la séquence protéique comprennent une variation faux-sens, non-sens, d'épissage, de délétion, d'insertion, ou de déphasage.

6. Procédé des revendications 1 à 5, dans lequel les règles logiques standard de l'algèbre booléenne s'appliquent, en particulier la loi du tiers exclu, l'élimination de double négation, la loi de non-contradiction, le principe d'explosion, la monotonie d'implication, l'idempotence d'implication, la commutativité de conjonction et la loi de dualité de De Morgan.

7. Procédé des revendications 1 à 6, dans lequel la fonction de prédiction est optimisée pour au moins l'un des suivants : sensibilité, spécificité, valeur prédictive positive, valeur prédictive négative, taux de classification correcte, taux d'erreur de classification, aire sous la courbe de fonction d'efficacité du récepteur (AROC), rapport de cotes, pappa, rapport de Jaccard négatif, rapport de Jaccard positif, rapport ou indice de Jaccard combiné, dans lequel l'aire sous la courbe de fonction d'efficacité du récepteur (AROC) et le rapport de Jaccard négatif sont préférés.

8. Procédé de détermination d'une fonction qui permet la prédiction de la manifestation d'une mesure de résultat d'un patient cancéreux sur la base d'un échantillon de tissu contenant de l'ADN tumoral provenant du patient, comprenant :
- la détermination de la séquence d'ADN de segments d'au moins deux segments de gène dans un groupe de patients cancéreux qui est constitué de patients avec au moins deux manifestations disjonctives de la mesure de résultat ;
- la détermination de la variation de séquence des au moins deux segments de gène de l'ADN tumoral comme étant :
- présente, si au moins une variation de séquence significative peut être déterminée, ou comme étant
- absente, si aucune variation de séquence significative ne peut être déterminée ;
dans lequel la variation de séquence comprend une variation de séquence silencieuse ou synonyme ;
- la combinaison des états de variation de séquence des au moins deux segments de gène en utilisant un opérateur logique, de façon à agréger les informations biologiques contenues dans chaque état de variation de séquence et générer une fonction de prédiction, de sorte que des patients avec une manifestation spécifique de la mesure de résultat puissent être distingués de patients avec une autre manifestation disjonctive de la mesure de résultat,
dans lequel la fonction de prédiction comprend des variations de séquence ou leur négation en tant que variables et un opérateur logique combinant les variables, dans lequel l'opérateur logique est choisi dans le groupe constitué de : négation de conjonction (Nand), négation de disjonction (Nor), équivalence (Eqv), négation d'équivalence (disjonction exclusive, Xor), implication matérielle (Imp), et négation d'implication matérielle (Nimp).

9. Procédé selon la revendication 8, dans lequel la prédiction de la mesure de résultat du patient cancéreux comprend
- la prédiction de la progression de maladie d'un cancer du patient, telle que la récidive locale du cancer, l'apparition d'une tumeur maligne secondaire, ou l'apparition de métastases, ou
- la prédiction de la réponse vs. non-réponse du patient à un traitement anticancéreux avec un médicament, tel qu'une chimiothérapie adjuvante, une chimiothérapie néo-adjuvante, une chimiothérapie palliative ou l'utilisation de médicaments ciblés en combinaison avec une chimiothérapie ou une radio-chimiothérapie.

10. Procédé selon la revendication 8 ou 9, dans lequel l'échantillon de tissu contenant de l'ADN tumoral est du tissu tumoral, de l'expectoration, des selles, de l'urine, un lavage bronchique, du liquide céphalo-rachidien, du sang, du plasma ou du sérum.

11. Procédé des revendications 8 à 10, dans lequel la prédiction de la variation de séquence comprend l'analyse de
- variations de séquence qui modifient la séquence protéique.

12. Procédé des revendications 8 à 11, dans lequel les variations de séquence qui modifient la séquence protéique comprennent une variation faux-sens, non-sens, d'épissage, de délétion, d'insertion, ou de déphasage.

13. Procédé des revendications 8 à 12, dans lequel la fréquence relative de variations de séquence dans un gène des au moins deux segments de gène est au moins 1 %, de préférence au moins 3 % dans une population de patients donnée.

14. Programme informatique, adapté pour effectuer le procédé des revendications 8 à 13, en particulier les étapes de
- détermination de l'existence d'une variation de séquence dans des segments d'au moins deux segments de gène de l'ADN tumoral comme étant :
- présente, si au moins une variation de séquence peut être déterminée, ou comme étant
- absente, si aucune variation de séquence ne peut être déterminée ;
dans lequel les au moins deux segments de gène de l'ADN tumoral sont associés à la mesure de résultat du patient ; et
dans lequel la variation de séquence comprend une variation de séquence silencieuse ou synonyme,
- la combinaison de l'existence de variations de séquence des au moins deux segments de gène en utilisant une opération logique (fonction de prédiction), de façon à agréger les informations biologiques contenues dans chaque état de variation de séquence, et
- la prédiction, sur la base des résultats de l'opération logique, de la manifestation de la mesure de résultat du patient,
dans lequel la fonction de prédiction comprend des variations de séquence ou leur négation en tant que variables et un opérateur logique combinant les variables, dans lequel l'opérateur logique est choisi dans le groupe constitué de : négation de conjonction (Nand), négation de disjonction (Nor), équivalence (Eqv), négation d'équivalence (disjonction exclusive, Xor), implication matérielle (Imp), et négation d'implication matérielle (Nimp).
